# EUROPEAN PATENT SPECIFICATION

(11) **EP 3 039 159 B1**
(45) Date of publication and mention of the grant of the patent: **04.10.2017**
(21) Application number: 14766598.8
(22) Date of filing: 29.08.2014
(51) Int. Cl.: C12Q 1/68, G06F 19/20, G06F 19/28

(54) **METHODS OF IDENTIFYING COSMETIC AGENTS FOR TREATING PERIORBITAL DYSCHROMIA AND SYSTEMS THEREFOR**
VERFAHREN ZUR IDENTIFIZIERUNG VON KOSMETISCHEN WIRKSTOFFEN ZUR BEHANDLUNG VON PERIORBITALER DYSCHROMIE UND SYSTEME DAFÜR
PROCÉDÉS D'IDENTIFICATION D'AGENTS COSMÉTIQUES POUR LE TRAITEMENT D'UNE DYSCHROMIE PÉRIORBITAIRE ET SYSTÈMES ASSOCIÉS

(30) Priority: 30.08.2013 US 201361872262 P
(43) Date of publication of application: 06.07.2016
(73) Proprietor: The Procter & Gamble Company, Cincinnati, OH 45202 (US)
(72) Inventor: OSORIO, Karen, Marie, Cincinnati Ohio 45202 (US); ZHAO, Wenzhu, Cincinnati Ohio 45202 (US)
(74) Representative: Engisch, Gautier
(86) International application number: PCT/US2014/053331
(87) International publication number: WO 2015/031708

(56) References cited:
- WO-A1-2012/135651
- WO-A1-2012/172220
- WO-A1-2013/093329
- WO-A2-2004/059001
- WO-A2-2012/116081
- FERNANDA MAGAGNIN FREITAG ET AL: "What causes dark circles under the eyes?", JOURNAL OF COSMETIC DERMATOLOGY, vol. 6, no. 3, 1 September 2007 (2007-09-01), pages 211-215, XP055029891, ISSN: 1473-2130, DOI: 10.1111/j.1473-2165.2007.00324.x
- GOODMAN R M ET AL: "Periorbital hyperpigmentation. An overlooked genetic disorder of pigmentation", ARCHIVES OF DERMATOLOGY, AMERICAN MEDICAL ASSOCIATION, US, vol. 100, no. 2, 1 August 1969 (1969-08-01), pages 169-174, XP008173092, ISSN: 0003-987X, DOI: 10.1001/ARCHDERM.1969.01610260045007

## Description

### TECHNICAL FIELD

Methods of identifying and/or evaluating potential cosmetic agents useful for treating periorbital dyschromia are generally provided. More specifically, the present invention relates to using gene expression signatures, data architechtures and connectivity mapping to identify and/or evaluate cosmetic agents useful for treating different types of periorbital dyschromia are provided.

### BACKGROUND

A person's eyes are a prominent and noticeable facial feature. Thus, aesthetic features associated with the eyes may influence an individual's perception of herself or the impression she makes on others. Not surprisingly, a variety of ways to accentuate and/or beautify the eyes have been devised throughout history. For example, some people may use cosmetic compositions to hide undesirable aesthetic features around theif eyes. Undesirable aesthetic features typically include fine lines, wrinkles and discoloration of the skin around the eye. A particularly undesirable aesthectic feature is periorbital dyschromia, sometimes referred to as "dark circles" or "under-eye dark circles." Periorbital dychromia can be particularly undesirable because it is commonly associated with fatigue and/or old age, which are the antithesis of a desired perception or youthful beauty.

While using cosmetic compositions such as make up (e.g., foundation, concealer or eyeshadow) to hide a perceived flaw (e.g., skin discoloration, lines, and wrinkles) or accentuate a particular feature of the eye may provide a temporary cosmetic benefit, most conventional make up products requires daily application, and in some instances may even require reapplication throughout the day. Thus, a longer lasting or more permanent solution is needed to reduce and/or eliminate some of the undesirable aesthetic features commonly associated with the eye.

In an effort to find a longer lasting cosmetic solution to the problem of periorbital dyschromia, researchers have tried to identify its underlying causes. A variety of theories ranging from undereye skin thickness to hyperpigmentation related to the overproduction of melanin have been suggested as causing periorbital dyschromia. Up to now, periorbital dyschromia is a multifactorial pathogenesis that is not well elucidated. The belief that a variety of factors are responsible for causing periorbital dyschromia has led to attempts to classify periorbital dyschromia into discrete types according to the different underlying factor(s) believed to be responsible for the condition. But these attempts have failed to provide a commercially viable method of classifying periorbital dyschromia or a system that is suitable for developing and marketing cosmetic products that target periorbital dyschromia. However, it has recently been discovered that different types of periorbital dychromia can be distinguished from one another based on certain factors. Nevertheless, periorbital dychromia is still a highly complex condition with multiple and overlapping etiologies, which manifest, in part, as a function of individual predisposition, and which therefore pose a significant treatment challenge. Thus, there remains a need in the cosmetic arts both for generating potential cosmetic agents suitable for use in treating periorbital dyschromia and for effective and efficient screening methods for identifying agents with efficacy and safety in the cosmetic treatment of periorbital dyschromia. In particular, there remains a need for methods of identifying potential cosmetic agents and for evaluating the efficacy of cosmetic agents using screening methods that are substantially independent of the mechanism of action or etiology of the periorbital dyschromia condition.

Recently, a more detailed genomic understanding of periorbital dyschromia has revealed potentially hundreds of genes and other effectors involved in the appearance of different types of periorbital dyschromia, which may provide genetic targets suitable for use in identifying agents and/or treatments. An investigation into the application of a relatively new technology known as "connectivity mapping" ("C-mapping") was undertaken to the search for new cosmetic agents that may be suitable for treating one or more types of periborbital dyschromia. Connectivity mapping is a hypothesis generating and testing tool having successful application in the fields of operations research, telecommunications, and more recently in pharmaceutical drug discovery.

The general notion that functionality could be accurately determined for previously uncharacterized genes and potential targets of drug agents could be identified by mapping connections in a database of gene expression profiles for drug-treated cells is described by T.R. Hughes et al. in "Functional Discovery Via a Compendium of Expression Profiles" Cell 102, 109-126 (2000)]. The launch of The Connectivity Map ("C-map") Project by Justin Lamb and researchers at MIT ("Connectivity Map: Gene Expression Signatures to Connect Small Molecules, Genes, and Disease", Science, Vol 313, 2006) helped provide support for the connectivity theory. In 2006, Lamb's group began publishing a detailed synopsis of the mechanics of C-map construction and installments of the reference collection of gene expression profiles used to create the first generation C-map and the initiation of an on-going large scale community C-map project, which is available under the "supporting materials" hyperlink at http://www.sciencemag.org/content/313/5795/1929/suppl/DC1.

Connectivity mapping has achieved in confirmed medical successes with identification of new agents for the treatment of various diseases, including cancer. Nonetheless, certain limiting presumptions challenge application of C-map with respect to diseases of polygenic origin or conditions that are characterized by diverse, and often apparently unrelated, cellular phenotypic manifestations (such as periorbital dyschromia). According to Lamb, the challenge to constructing a useful C-map is in the selection of input reference data which permit generation of clinically salient and useful output upon query. For the drug-related C-map of Lamb, strong associations comprise the reference associations, and strong associations are the desired output identified as "hits."

However, agents suitable for use as pharmaceutical agents and agents suitable for use as cosmetic agents are categorically distinct. Pharmaceutical agents are selected for specificity and intended to have measurable effects on structure and function of the body, while cosmetic agents are selected for effect on appearance and may not affect structure and function of the body to a measurable degree. Cosmetic agents also tend to be non-specific with respect to effect on cellular phenotype, and administration to the body is generally limited to application on or close to the body surface.

In constructing C-maps relating to pharmaceutical agents, it has been stressed that particular difficulty may be encountered if reference connections are extremely sensitive and at the same time difficult to detect (weak), and thus compromises have been adopted which are aimed at minimizing numerous, diffuse associations. Since the regulatory scheme for drug products requires high degrees of specificity between a purported drug agent and disease state, and modulation of disease by impacting a single protein with a minimum of tangential associations is desired in development of pharmaceutical actives, the Lamb C-map is well-suited for screening for potential pharmaceutical agents despite such compromises.

The connectivity mapping protocols of Lamb would not be predicted, therefore, to have utility for hypothesis testing/generating in the field of cosmetics and periorbital dyschromia, particularly given the compromises described above, or for a primarily cosmetic disorder where symptoms may be diffuse, systemic and relatively mild. Cosmetics formulators seek agents or compositions of agents capable of modulating multiple targets and having effects across complex phenotypes and conditions. Further, the phenotypic impact of a cosmetic agent (e.g., suitable for treatment of periorbital dyschromia) must be relatively low by definition, so that the agent avoids being subject to the regulatory scheme for pharmaceutical actives. Nonetheless, the impact must be perceptible to the consumer and preferably empirically confirmable by scientific methods. Gene transcription/expression profiles for cosmetic conditions are generally diffuse, comprising many genes with low to moderate fold differentials. Cosmetic agents, therefore, provide more diverse and less acute effects on cellular phenotype and generate the sort of associations expressly taught by Lamb as unsuitable for generating connectivity maps useful for confident hypothesis testing.

Nonetheless, contrary to the teachings of Lamb and the prior art in general, it has been surprisingly discovered that useful connectivity maps could be developed to evaluate and/or identify cosmetic actives for treating periorbital dyschromia, despite the highly diffuse, systemic and low-level effects these sorts of actives generally engender. Additionally, the value of a connectivity map approach to discover functional connections shared by periorbital dychromia phenotypes is counter-indicated by the progenitors of the drug-based C-map; the relevant phenotypes are very complex, the etiology is not well understood, the genetic perturbations are numerous and weak, and cosmetic agent action is likewise diffuse and, by definition, relatively weak. The successful application of connectivity mapping to target periorbital dyschromia, which is multi-factored and poorly elucidated, is a breakthrough in periorbital dyschromia research.

FERNANDA MAGAGNIN FREITAG ET AL: "What causes dark circles under the eyes?", JOURNAL OF COSMETIC DERMATOLOGY, vol. 6, no. 3, 1 September 2007 (2007-09-01), pages 211-215, ISSN: 1473-2130, DOI: 10.1111/j.1473-2165.2007.00324.x, discusses dark circles under the eyes and some of the anatomic features that could explain dark circles and the proposed treatments for the condition.

GOODMAN R M ET AL: "Periorbital hyperpigmentation. An overlooked genetic disorder of pigmentation", ARCHIVES OF DERMATOLOGY, AMERICAN MEDICAL ASSOCIATION, US, vol. 100, no. 2, 1 August 1969 (1969-08-01), pages 169-174, XP008173092, ISSN: 0003-987X, DOI: 10.1001/ARCHDERM.1969.01610260045007, discusses a human family with a genetically determined form of hyperpigmentation involving the periorbital area. Various clinical and genetic aspects of their autosomal dominant trait are discussed

WO 2012/116081 discusses methods and systems for determining functional relationships between a cosmetic agent and a skin tissue condition of interest.

WO 2012/135651discusses methods and systems for determining functional relationships between a skin-active agent and a skin condition of interest, and methods and systems for identifying cosmetic agents effective for treatment of dandruff.

WO2012172220 discusses a molecular signature of cutaneous pigmentary spots, comprising the genes TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV, ITGB1 and ACTN1, and various applications of this signature.

WO2013/093329 discusses a microRNA selected from among hsa-miR-330, hsa-miR-7 and hsa-miR-137, the mature forms thereof, and the precursors thereof, for depigmenting the skin, and an in vitro method for identifying depigmenting compounds.

WO2004/059001 discusses a method for determining the homeostasis of human facial skin in vitro, to test kits and biochips for determining markers of human facial skin, in addition to the use of proteins, mRNA molecules or fragments of proteins or mRNA molecules as markers of human facial skin.

### SUMMARY

In order to provide a solution to the problems above, provided herein is a method of constructing a data architecture for use in identifying connections between perturbagens and genes associated with a type of periorbital dyschromia. The method comprises providing a gene expression profile for a control human cell. The control cell is from a human cell line selected from the group consisting of keratinocyte, fibroblast, melanocyte and melanoma cell lines. The method also comprises generating a gene expression profile for a human cell exposed to at least one perturbagen. The cell is used to generate the gene expression profile for the human cell exposed to the perturbagen is from the same cell line as the control cell. The method further comprises identifying genes differentially expressed in response to the perturbagen by comparing the gene expression profiles of the control cell and the test cell, and then creating an ordered list of identifiers representing the differential 4a expressed genes. The identifiers are ordered according to the differential expression of the genes. The ordered list is stored as an instance on a computer readable medium. The steps are then repeated to construct a data architecture of stored instances. The perturbagen used to generate the test profile is different qualitatively or quantitatively for each instance.

Also disclosed herein is a system for identifying connections between perturbagens and genes associated with periorbital dyschromia. The system comprises a computer readable medium having instances and a periorbital dychromia-relevant gene expression signature. The instances and the gene expression signature are derived from a human epidermal skin cell or a human dermal skin cell. Each instance comprises an instance list of rank-ordered identifiers of differentially expressed genes. The periorbital dychromia-relevant gene expression signature comprises gene expression signature lists of identifiers representing differentially expressed genes associated with a type of periorbital dyschromia. The system also comprises a programmable computer comprising computer-readable instructions that cause the programmable computer to execute one or more of the following: (i) accessing the plurality of instances and a periorbital dychromia-relevant gene expression signature stored on the computer readable medium; (ii) comparing the periorbital dychromia-relevant gene expression signature to the plurality of the instances, wherein the comparison comprises comparing each identifier in the gene expression signature list with the position of the same identifier in the instance list for each of the plurality of instances; and (iii) assigning a connectivity score to each of the plurality of instances.

Further disclosed herein is a method of formulating a cosmetic composition. The method comprises accessing with a computer a plurality of skin instances stored on at least one computer readable medium. Each instance is associated with a perturbagen, and each instance comprises an ordered list comprising a plurality of identifiers representing up-regulated genes and down-regulated genes. The method also comprises accessing with a computer at least one gene expression signature stored on the at least one computer readable medium. The gene expression signature corresponds to a type of periorbital dyschromia and comprises one or more lists of identifiers representing a plurality of up-regulated genes and down-regulated genes. The method further comprises assigning with a computer a connectivity score to each of the plurality of instances. The method is used to formulate a cosmetic composition comprising a dermatologically acceptable carrier and a perturbagen(s), and the connectivity score of the instance associated with the perturbagen is negative.

### BRIEF DESCRIPTION OF THE FIGURES

Figure 1 is an illustration of the appearance of Type I periorbital dyschromia.
Figure 2 is an illustration of the appearance of Type II periorbital dyschromia
Figure 3 is an illustratin of the appearance of Type III periorbital dyschromia.
Figure 4 is a schematic illustration of an exemplary embodiment of the method herein.
Figure 5 is a schematic illustration of an exemplary system for generating an instance.
Figure 6 is a schematic illustration of a computing device suitable for use with the present invention;
Figure 7 is a schematic illustration of an instance associated with a computer readable medium.
Figure 8 is a schematic illustration of a comparison between a gene expression signature and an instance, wherein there is a positive correlation between the lists;
Figure 9 is a schematic illustration of a comparison between a gene expression signature and an instance, wherein there is a negative correlation between the lists; and
Figure 10 is a schematic illustration of a comparison between a gene expression signature and an instance, wherein there is a neutral correlation between the lists.

### DETAILED DESCRIPTION

The terminology used in the description herein is for describing particular embodiments only and is not intended to be limiting. As used in the description and the appended claims, the singular forms "a," "an," and "the" are intended to include the plural forms as well, unless the context clearly indicates otherwise. All percentages are by weight of the relevant composition, unless otherwise specified. All ratios are weight ratios, unless specifically stated otherwise. All numeric ranges are inclusive of narrower ranges; delineated upper and lower range limits are interchangeable to create further ranges not explicitly delineated. The number of significant digits conveys neither limitation on the indicated amounts nor on the accuracy of the measurements. All measurements are understood to be made at about 25° C and at ambient conditions, where "ambient conditions" means conditions under about one atmosphere of pressure and at about 50% relative humidity.

### Definitions.

"Benchmark agent" refers to any chemical, compound, environmental factor, small or large molecule, extract, formulation, or combinations thereof that is(are) known to induce or cause a superior effect (positive or negative) on the gene expression of a periorbital dychromia condition.

"Computer readable medium" refers to any electronic storage medium and includes, but is not limited to, any volatile, nonvolatile, removable, and non-removable media implemented in any method or technology for storage of information such as computer readable instructions, data and data structures, digital files, software programs and applications, or other digital information. Computer readable media includes, but are not limited to, application-specific integrated circuit (ASIC), a compact disk (CD), a digital versatile disk (DVD), a random access memory (RAM), a synchronous RAM (SRAM), a dynamic RAM (DRAM), a synchronous DRAM (SDRAM), double data rate SDRAM (DDR SDRAM), a direct RAM bus RAM (DRRAM), a read only memory (ROM), a programmable read only memory (PROM), an electronically erasable programmable read only memory (EEPROM), a disk, a carrier wave, and a memory stick. Examples of volatile memory include, but are not limited to, random access memory (RAM), synchronous RAM (SRAM), dynamic RAM (DRAM), synchronous DRAM (SDRAM), double data rate SDRAM (DDR SDRAM), and direct RAM bus RAM (DRRAM). Examples of non-volatile memory include, but are not limited to, read only memory (ROM), programmable read only memory (PROM), erasable programmable read only memory (EPROM), and electrically erasable programmable read only memory (EEPROM). A memory can store processes and/or data. Still other computer readable media include any suitable disk media, including but not limited to, magnetic disk drives, floppy disk drives, tape drives, Zip drives, flash memory cards, memory sticks, compact disk ROM (CD-ROM), CD recordable drive (CD-R drive), CD rewriteable drive (CD-RW drive), and digital versatile ROM drive (DVD ROM).

"Connectivity map" and "C-map" refer broadly to devices, systems, articles of manufacture, and methodologies for identifying relationships between cellular phenotypes or cosmetic conditions, gene expression, and perturbagens, such as cosmetic actives. A description of connectivity mapping and methods of using connectivity mapping to identify genes and/or compositions of interest can be found in U.S. Publication No. 2012/0283112 titled "Systems and Methods For Identifying Cosmetic Agents For Skin Care Compositions" filed by Binder, et al., on February 22, 2012 and co-pending U.S. App. Ser. No. 13/851,886, titled "Systems, Models and Methods for Identifying and Evaluating Skin-Active Agents Effective for Treating Conditions and Disorders of Skin Pigmentation," filed by Hakozaki, et al., on March 30, 2012.

"Connectivity score" refers to a derived value representing the degree to which an instance correlates to a query.

"Control sample" means a matched sample (e.g., the same cell type used to generate the gene expression measurements for the plurality of biological conditions) that is identified as not including a particular type of periorbital dyschromia or is identified as having no dyschromia. For example, the gene expression measurements from a control sample may be generated from a biological sample taken earlier in time, prior to exhibiting periorbital dyschromia; a control subject or population whose gene expression measurements are known; or an index value or baseline value. A control gene expression profile can also be derived from prediction algorithms or computed indices from population studies. In various embodiments, the control sample is matched for race, gender, age, geographic location, and/or ethnic origin with respect to origin of the gene expression measurements of the plurality of biological disorders.

"Cosmetic" means providing a desired visual effect on an area of the human body. The visual cosmetic effect may be temporary, semi-permanent, or permanent. Some non-limiting examples of "cosmetic products" include products that leave color on the face, such as foundation, mascara, concealers, eye liners, brow colors, eye shadows, blushers, lip sticks, lip balms, face powders, solid emulsion compact, and the like.

"Cosmetic agent" means any substance, as well any component thereof, intended to be rubbed, poured, sprinkled, sprayed, introduced into, or otherwise applied to a mammalian body or any part thereof to provide a cosmetic effect. Cosmetic agents may include substances that are Generally Recognized as Safe (GRAS) by the US Food and Drug Administration, food additives, and materials used in non-cosmetic consumer products including over-the-counter medications. In some embodiments, cosmetic agents may be incorporated in a cosmetic composition comprising a dermatologically acceptable carrier suitable for topical application to skin. Cosmetic agents include, but are not limited to, (i) chemicals, compounds, small or large molecules, extracts, formulations, or combinations thereof that are known to induce or cause at least one effect (positive or negative) on skin tissue; (ii) chemicals, compounds, small molecules, extracts, formulations, or combinations thereof that are known to induce or cause at least one effect (positive or negative) on skin tissue and are discovered, using the provided methods and systems, to induce or cause at least one previously unknown effect (positive or negative) on the skin tissue; and (iii) chemicals, compounds, small molecules, extracts, formulations, or combinations thereof that are not known have an effect on skin tissue and are discovered, using the provided methods and systems, to induce or cause an effect on skin tissue.

Some examples of cosmetic agents or cosmetically actionable materials can be found in: the PubChem database associated with the National Institutes of Health, USA (http://pubchem.ncbi.nlm.nih. gov); the Ingredient Database of the Personal Care Products Council (http://online. personalcarecouncil.org/jsp/Home.jsp); and the 2010 International Cosmetic Ingredient Dictionary and Handbook, 13th Edition, published by The Personal Care Products Council; the EU Cosmetic Ingredients and Substances list; the Japan Cosmetic Ingredients List; the Personal Care Products Council, the SkinDeep database (URL: http://www.cosmeticsdatabase.com); the FDA Approved Excipients List; the FDA OTC List; the Japan Quasi Drug List; the US FDA Everything Added to Food database; EU Food Additive list; Japan Existing Food Additives, Flavor GRAS list; US FDA Select Committee on GRAS Substances; US Household Products Database; the Global New Products Database (GNPD) Personal Care, Health Care, Food/Drink/Pet and Household database (URL: http://www.gnpd.com); and from suppliers of cosmetic ingredients and botanicals.

Other non-limiting examples of cosmetic agents include botanicals (which may be derived from one or more of a root, stem bark, leaf, seed or fruit of a plant). Some botanicals may be extracted from a plant biomass (e.g., root, stem, bark, leaf, etc.) using one more solvents. Botanicals may comprise a complex mixture of compounds and lack a distinct active ingredient. Another category of cosmetic agents are vitamin compounds and derivatives and combinations thereof, such as a vitamin B3 compound, a vitamin B5 compound, a vitamin B6 compound, a vitamin B9 compound, a vitamin A compound, a vitamin C compound, a vitamin E compound, and derivatives and combinations thereof (e.g., retinol, retinyl esters, niacinamide, folic acid, panthenol, ascorbic acid, tocopherol, and tocopherol acetate). Other non-limiting examples of cosmetic agents include sugar amines, phytosterols, hexamidine, hydroxy acids, ceramides, amino acids, peptides, and polyols.

"Data architecture" refers generally to one or more digital data structures comprising an organized collection of data. In some embodiments, the digital data structures can be stored as a digital file (e.g., a spreadsheet file, a text file, a word processing file, a database file, etc.) on a computer readable medium. In some embodiments, the data architecture is provided in the form of a database that may be managed by a database management system (DBMS) that is be used to access, organize, and select data (e.g., instances and gene expression signatures) stored in a database.

"Dermatologically acceptable" means that the compositions or components described are suitable for use in contact with human skin tissue.

"Effective amount" means an amount of a compound or composition sufficient to significantly induce a positive or desired benefit, (e.g., a positive skin or feel benefit, reverse the expression of a gene, group of genes and/or gene expression signature), including independently or in combinations the benefits disclosed herein, but low enough to avoid serious side effects, i.e., to provide a reasonable benefit to risk ratio, within the scope of sound judgment of the skilled artisan.

"Gene expression signature" refers to a rationally derived list, or plurality of lists, of genes representative of a periorbital dyschromia condition or a cosmetic agent. In some instances, the cosmetic agent may be a benchmark skin agent or a potential agent for treating periorbital dychromia ("periorbital dychromia agent"). Thus, the gene expression signature may serve as a proxy for a phenotype of interest for periorbital dyschromia. A gene expression signature may comprise genes whose expression, relative to a normal or control state, is increased (up-regulated), whose expression is decreased (down-regulated), and combinations thereof. Generally, a gene expression signature for a modified cellular phenotype may be described as a set of genes differentially expressed in the modified cellular phenotype over the cellular phenotype. A gene expression signature can be derived from various sources of data, including but not limited to, from *in vitro* testing, *in vivo* testing and combinations thereof. In some embodiments, a gene expression signature may comprise a first list representative of a plurality of up-regulated genes of the condition of interest and a second list representative of a plurality of down-regulated genes of the condition of interest. For example, a periorbital dyschromia gene expression, which is a gene expression signature associated with one or more types of periorbital dyschromia, can be found in Tables 1 though 12 below.

"Gene expression profiling" refers to the measurement of the expression of multiple genes in a biological sample using any suitable profiling technology. For example, the mRNA expression of thousands of genes may be determined using microarray techniques. Other emerging technologies that may be used include RNA-Seq or whole transcriptome sequencing using NextGen sequencing techniques. Gene expression profiling may be used to generate a gene expression signature.

"Instance" refers to data from a gene expression profiling experiment in which skin cells are dosed with a perturbagen. In some embodiments, the data comprises a list of identifiers representing the genes that are part of the gene expression profiling experiment. The identifiers may include gene names, gene symbols; microarray probe set IDs, or any other identifier. In some embodiments, an instance may comprise data from a microarray experiment and comprises a list of probe set IDs of the microarray ordered by their extent of differential expression relative to a control. The data may also comprise metadata, including but not limited to data relating to one or more of the perturbagen, the gene expression profiling test conditions, the skin cells, and the microarray.

"Keratinous tissue," means keratin-containing tissue layers disposed as the outermost protective covering of mammals which includes, but is not limited to, skin, hair, and nails.

"Microarray" refers broadly to any ordered array of nucleic acids, oligonucleotides, proteins, small molecules, large molecules, and/or combinations thereof on a substrate that enables gene expression profiling of a biological sample. Non-limiting examples of microarrays are available from Affymetrix, Inc.; Agilent Technologies, Inc.; Illumina, Inc.; GE Healthcare, Inc.; Applied Biosystems, Inc.; Beckman Coulter, Inc.; etc.

"Periorbital" means around the orbit of the eye. The "periorbital region" of a person is the area of the face generally around the eye. The periorbital region of a person is typically disposed longitudinally between the bottom of the brow and the top of the cheek and leterally between the bridge of the nose and the temple.

"Periorbital dyschromia" is a condition that occurs when the tone of skin in the periorbital region of person is noticeably different from the tone of skin in a nearby portion of the face, such as the cheek, nose, forehead, temple and/or another portion of the periorbital region. Periorbital dyschromia is bilateral, (i.e., it occurs in the periorbital region of both sides of the face). Periorbital dyschromia may appear as a result of hyperpigmented and/or hypopigmented skin disposed in the periorbital region. Periorbital dyschromia may be identified and/or classified according to one or more of the indicators described in more detail below. Periorbital dychromia herein is classified into one of three types (i.e., Type I, Type II or Type III). The three types of periorbital dyschromia are described and defined in more detail below, and can be readily determined in accordance with the methods herein.

"Personal care composition" means a cosmetic composition or a skin care composition suitable. Is it to be appreciated that a personal care composition may provide both a cosmetic benefit and a skin health benefit.

"Perturbagen" means anything used as a challenge in a gene expression profiling experiment to generate gene expression data for use herein. In some embodiments, the perturbagen is applied to epidermal and/or dermal skin cells and the gene expression data derived from the gene expression profiling experiment may be stored as an instance in a data architecture. Any substance, chemical, compound, active, natural product, extract, drug [e.g. Sigma-Aldrich LOPAC (Library of Pharmacologically Active Compounds) collection], small molecule, and combinations thereof used as to generate gene expression data can be a perturbagen. A perturbagen can also be any other stimulus used to generate differential gene expression data. For example, a perturbagen may also be UV radiation, heat, osmotic stress, pH, a microbe, a virus, and small interfering RNA. A perturbagen may be, but is not required to be, a cosmetic agent.

"Putative cosmetic agent" means a cosmetic agent that has shown promise through preliminary screens as effecting a specific change in skin biology related to periorbital dyschromia but that has not yet been tested for effectiveness through the methods described herein.

"Query" refers to data that is used as an input to a C-map and against which a plurality of instances are compared. A query may include a gene expression signature associated with a skin condition such as age spots, or may include a gene expression signature derived from a physiological process associated with a skin condition. A C-map may be queried with perturbagens, gene expression signatures, periorbital dyschromia types, thematic signatures, or any data feature or combination of data features or associations that comprise the data architecture.

"Reverse" when referring to the gene expression of a gene means that the expression of the gene is changed such that it is opposite of the expression indicated in a gene signature in a significant way (e.g., p-value < 0.1, p-value < 0.05, p-value < 0.01, p-value < 0.001, or p-value < .0001 as determined by a statistical test like ANOVA or to a t-test). For example, if a gene expression signature indicates that a particular gene is up-regulated, then reversing the expression of the gene can mean that the gene is down-regulated relative to the indicated gene expression signature with a p-value of less than 0.05 as determined by a statistical test like ANOVA or t-test. When referring to gene expression signatures, the term "reversing" depends on the method used to determine the change in gene expression signature. For example, when using connectivity mapping, a connectivity score is generated to represent an amount of differential expression relative to a known gene expression signature, e.g., stored in a data architecture, and the connectivity score can be used as a measure of the amount of reversal in a gene expression signature. in a significant way (e.g., p-value < 0.1, p-value < 0.05, p-value < 0.01, p-value < 0.001, or p-value < .0001.

"Skin" means the outermost protective covering of mammals that is composed of cells such as keratinocytes, fibroblasts and melanocytes. Skin includes an outer epidermal layer and an underlying dermal layer. Skin may also include hair and nails as well as other types of cells commonly associated with skin, such as, for example, myocytes, Merkel cells, Langerhans cells, macrophages, stem cells, sebocytes, nerve cells and adipocytes. "Skin care" means regulating and/or improving skin condition. "Skin-care composition" means a composition that regulates and/or improves skin condition.

"Skin tone" refers to the perceived color or pigmentation of skin pigmentation, especially with regard to the evenness of coloration or pigmentation. "Skin tone" may also include other characteristics of skin that contribute to a consumer perception of overall tone. For example, pore size and distribution, and skin texture are also generally considered attributes of overall skin tone.

"Software" and "software application" mean one or more computer readable and/or executable instructions that cause a computing device or other electronic device to perform functions, actions, and/or behave in a desired manner. The instructions may be embodied in one or more various forms like routines, algorithms, modules, libraries, methods, and/or programs. Software may be implemented in a variety of executable and/or loadable forms and can be located in one computer component and/or distributed between two or more communicating, cooperating, and/or parallel processing computer components and thus can be loaded and/or executed in serial, parallel, and other manners. Software can be stored on one or more computer readable medium and may implement, in whole or part, the methods and functionalities of the present invention.

"Topical application" means to apply or spread the compositions of the present invention onto the surface of the keratinous tissue.

Before now, the underlying causes of periorbital dyschromia were not particularly well elucidated. It has unexpectedly been discovered that there are common themes associated with periorbital dyschromia, which lend themselves to differentiation based on a variety of relatively straightforward evaluation techniques. Previous attempts to classify periorbital dyschromia did not appreciate that periorbital dyschromia can be grouped into distinct categories based on, for example, visual evaluation and/or imaging techniques, biomarkers, histology, and/or genetic analysis. Based on these newly discovered distinctions, it is believed that improved cosmetic products and/or treatment regimens particularly suited for treating different types of periorbital dyschromia can be provided. In order to provide improved products and regimens for treating different types of periorbital dyschromia, devices, systems and methods for identifying potential active agents for use in topical cosmetic products are needed.

As described herein, there are three types of periorbital dyschromia conditions and a "No Dyschromia" condition, which may serve as a control condition in certain embodiments. The three types of periorbital dyschromia can be distinguished from one another and from the No Dyschromia condition using, for example, visual classification techniques, imaging techniques, biomarkers, histology, gene expression signatures and combinations of these. For example, a first type of periorbital dyschromia, referred to herein as "Type I," may be visually characterized by the appearance of darker, comtinuous and more chromatic skin tones in particular portions of periorbital skin, which may resemble tanned skin; a second type of periorbital dyschromia, referred to herein as "Type II," may be visually characterized by the presence of darker, continuous and less chormatic tones in periorbital skin, which may resemble bruised skin; and a third type of periorbital dyschromia, referred to herein as "Type III," may be characterized by the presence of darker, discontinuous tones in particular portions of the periorbital skin that resemble the appearance of Type I and II Periorbital dyschromia (e.g., appears as a combination of Type I and II). A No Dyschromia condition may be visually characterized by the lack of an uneven or discontinous skin tone in the majority of the periorbital region.

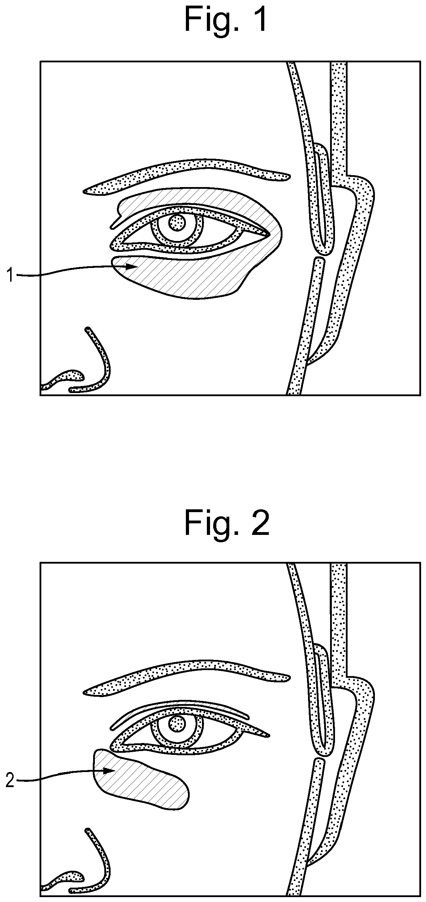

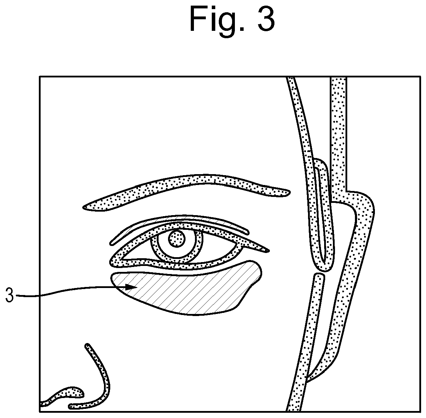

FIG. 1 illustrates an example of how Type I periorbital dyschromia, which is represented by the shaded portion 1 of the periorbital region, may be visually classified based upon its location in the periorbital region; FIG. 2 illustrates an example of how Type II periorbital dyschromia (i.e., the shaded portion 2 of the periorbital region), may be visually classified based upon its location in the periorbital region; and FIG. 3 illustrates an example of how Type III periorbital dyschromia (i.e., the shaded portion 3 of the periorbital region), may be visually classified based upon its location in the periorbital region.

The different types of periorbital dyschromia may also be distinguished from one another and from a No Dyschromia condition using a conventional imaging method such as an RGB color scale measurement method. For example, Type I periorbital dyschromia generally exhibits lower RGB values as compared to Types II and III, when measured according to the RGB method. In certain embodiments, Type I periorbital dyschromia may be characterized by having an R-value of from 143 to 178, a G-value of from 97 to 131, and/or a B-value of from 83 to 113, according to the RGB imaging method described in more detail below. In addition, Type I periorbital dyschromia may be characterized by having a ratio of B-value to G-value ("B/G ratio") of from 0.58 to 0.840. In certain embodiments, Type II periorbital dyschromia may be characterized by having an R-value of from 152 to 178, a G-value of from 106 to 139, and/or a B-value of from 97 to 126, according to the RGB Method. Type II periorbital dyschromia may also be characterized by having a B/G ratio of from 0.800 to 0.91. In certain embodiments, Type III periorbital dyschromia may be characterized by having an R-value of from 150 to 172, a G-value of from 105 to 131, and/or a B-value of from 96 to 116, according to the RGB imaging method described in more detail below. Type III periorbital dyschromia may be characterized by having a B/G ratio of from 0.848 to 0.909.

Examples of suitable methods and systems for classifying periorbital dyschromia are described in U.S. Provisional App. No. 14/215,323 filed by Osorio, et al., on March 15, 2013. Methods of treating different types of periorbital dyschromia and compositions therefor are described in U.S. Provisional App. Nos. 14/215,345 and 14/215,378 filed by Osorio, et al., on March 15, 2013.

Features of the invention are further described below. Section headings are for convenience of reading and not intended to be limiting *per se.* The entire document is intended to be related as a unified disclosure, and it should be understood that all combinations of features described herein are contemplated, even if the combination of features are not found together in the same sentence, or paragraph, or section of this document.

### MEASURING GENE EXPRESSION

In certain embodiments, the methods and systems herein comprise obtaining one or more gene expression measurements from biological samples and analyzing the measurements to identify differential expression in genes of interest. Gene expression measurements can comprise quantitative or qualitative expression data for a number of genes. In the context of periorbital dyschromia, gene expression measurements may be obtained from the analysis of the epidermal and dermal region of skin biopsy samples of periorbital skin. The gene expression measurements may be compared to, e.g., a control sample to provide insights into the biological processes associated with periorbital dyschromia. Alternatively or in addition, gene expression measurements may be obtained from cells challenged *in vitro* to mimic a type of periorbital dyschromia.

Gene expression may be detected and/or measured in a variety of ways. In certain embodiments, the method comprises measuring messenger ribonucleic acid ("mRNA") encoded by one or more genes of interest in a gene expression signature. Optionally, the method may include reverse transcribing mRNA encoded by one or more of the genes and measuring the corresponding complementary DNA ("cDNA"). Any suitable quantitative nucleic acid assay may be used herein. For example, conventional quantitative hybridization, Northern blot, and polymerase chain reaction procedures may be used for quantitatively measuring the amount of an mRNA transcript or cDNA in a biological sample. Optionally, the mRNA or cDNA may be amplified by polymerase chain reaction (PCR) prior to hybridization. The mRNA or cDNA sample is then examined by, e.g., hybridization with oligonucleotides specific for mRNAs or cDNAs encoded by one or more of the genes of the panel, optionally immobilized on a substrate (e.g., an array or microarray). Selection of one or more suitable probes specific for an mRNA or cDNA and selection of hybridization or PCR conditions are within the ordinary skill of those who work with nucleic acids. Binding of the biomarker nucleic acid to oligonucleotide probes specific for the biomarker(s) allows identification and quantification of the biomarker. Suitable examples of methods of quantifying gene expression are disclosed in U.S. Publication No. 2012/0283112; U.S. App. Serial Nos. 13/851858, 13/851864, 13/851873, and 13/851886; and U.S. Serial No. 13/966418, filed by Mills, et al., on August 15, 2012.

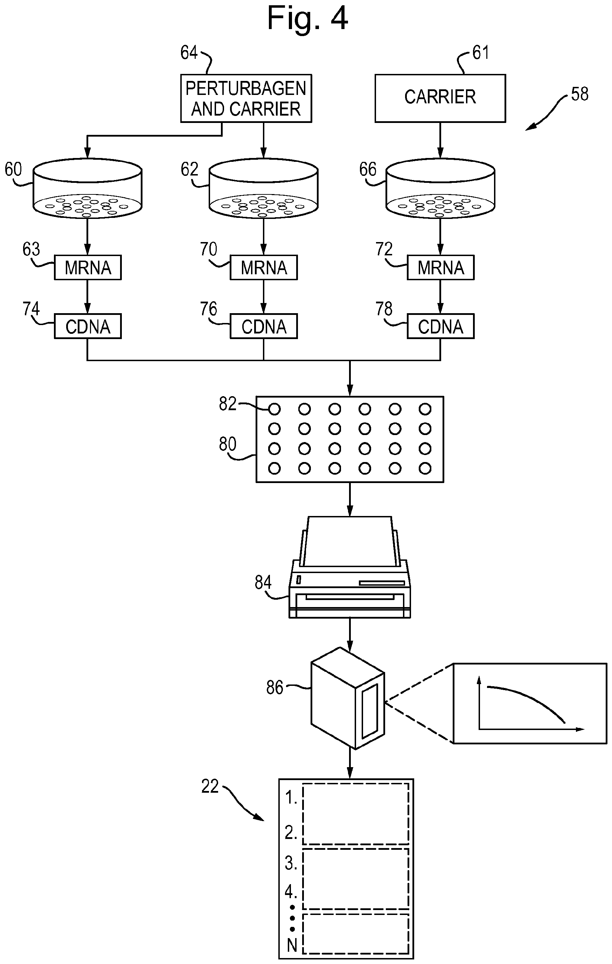

FIG. 4 illustrates an example of a method 58 of measuring gene expression and comparing the gene expression measurements to reference gene expression measurements (e.g., taken from a control sample). The method 58 comprises exposing test cells 60, 62 (e.g., keratinocytes and/or other skin cell associated with the periorbital region) to a perturbagen 64. The perturbagen 64 may be dissolved in a suitable carrier 61 such as dimethyl sulfoxide (DMSO). Optionally, reference cells 66, which are typically the same type of cell as the test cells 60, 62 but which are only exposed to the carrier 61 (i.e., no perturben), may be used as a control. After exposure to the perturben 64 and/or control 61, mRNA is extracted from the test cells 60, 62 and reference cells 66. The mRNA 63, 70, 72 extracted from the cells 60, 62 and 66 may, optionally, be reverse transcribed to cDNA 74, 76, 78 and marked with fluorescent dye(s) (e.g., red and green if a two color microarray analysis is to be performed). Alternatively, the cDNA samples 74, 76 and 78 may be prepped for a one color microarray analysis, and a plurality of replicates may be processed if desired. The cDNA samples 74, 76, 78 may be co-hybridized to the microarray 80 comprising a plurality of probes 82 (e.g., tens, hundreds, or thousands of probes). In some embodiments, each probe 82 on the microarray 80 has a unique probe set identifier. The microarray 80 is scanned by a scanner 84, which excites the dyes and measures the amount fluorescence. A computing device 86 analyzes the raw images to determine the amount of cDNA present, which is representative of the expression levels of a gene. The scanner 84 may incorporate the functionality of the computing device 86. Gene expression data collected by the system may include: i) up-regulation of gene expression (e.g., greater binding of the test material (e.g., cDNA 74, 76) to probes compared to reference material (e.g., cDNA 78)), ii) down-regulation of gene expression (e.g., reduced binding of the test material (e.g., cDNA 74, 76) to probes than the test material (e.g., cDNA 78)), iii) non-fluctuating gene expression (e.g., similar binding of the test material (e.g., cDNA 74, 76) to the probes compared to the reference material (e.g., cDNA 78)), and iv) no detectable signal or noise. The up- and down-regulated genes may be referred to as "differentially expressed," and the gene expression data may be used to generate one or more instances 22, which is described in more detail below.

Microarrays and microarray analysis techniques are well known in the art, and it is contemplated that other microarray techniques may be used with the methods, devices, and systems of the invention. For example, any suitable commercial or non-commercial microarray technology and associated techniques may used, such as, but not limited to Affymetrix GeneChip™ technology and Illumina BeadChip™ technology. One of skill in the art will appreciate that the invention is not limited to the methodology described above, and that other methods and techniques are also contemplated to be within its scope of the invention.

### SYSTEMS AND DEVICES

Devices, systems and methods are provided herein for constructing a database of stored gene expression signatures representative of different types of periorbital dyschromia, which is a valuable tool for, e.g., identifying active agents effective against one or more types of periorbital dyschromia, which is otherwise challenging given the widely variable clinical manifestations and elusive biology underlying different conditions associated with the skin in the undereye area. Devices, systems and methods also are provided for evaluating the influence of perturbagens on periorbital dyschromia, thereby potentially identifying connections (i.e., relationships) between the perturbagens and periorbital skin health and/or the appearance of periorbital dyschromia.

In certain embodiments, the system includes at least one computing device, a computer readable medium associated with at least one of the computing devices (e.g., hard disk or other suitable digital storage medium for storing, accessing and manipulating digital information such as database files), and a communication network. When the system includes more than one computing device, the computing devices may be in electronic communication with one another, for example, via a wired and/or wireless communication network. In certain embodiments, the computer readable medium may comprise a digital file with a plurality of instances in a data structure stored thereon. Additionally or alternatively, the computer readable medium may include a digital file with one or more lists of microarray probe set IDs associated with one or more periorbital dyschromia-relevant gene expression signatures. In certain embodiments, the instances may be stored on a first computer readable medium and the list(s) of probe set IDs on a second computer readable medium. The plurality of instances or lists of probe set IDs may be stored in relational tables and indexes or in other types of computer readable media. The digital file can be provided in wide variety of formats, including but not limited to a word processing file format, a spreadsheet file format, and a database file format. The instances may also be distributed across a plurality of digital files.

Data stored in the digital files may be stored in a wide variety of data structures and/or formats. In certain embodiments, the data is stored in one or more searchable databases, such as free databases, commercial databases, or a company's internal proprietary database. The database may be provided or structured according to any suitable model known in the art. In some embodiments, at least one searchable database is a company's internal proprietary database. A user of the system may use a graphical user interface associated with a database management system to access and retrieve data from the one or more databases or other data sources to which the system is operably connected. In some embodiments, a first digital file may be provided in the form of a first database and a second digital file may be provided in the form of a second database. In other embodiments, the first and second digital files may be combined and provided in the form of a single file.

In certain embodiments, one or more first digital files may include data that is transmitted across a communication network from a second digital file stored, for example, on a remotely located computer readable medium. For example, the first digital file(s) may comprise gene expression data (e.g., instances or periorbital discoloration gene signatures) obtained from a cell line (e.g., a fibroblast cell line and/or a keratinocyte cell line) as well as data from the remotely located second digital file, such as gene expression data from other cell lines or cell types, gene expression signatures, perturbagen information, clinical trial data, scientific literature, chemical databases, pharmaceutical databases, and other such data and metadata.

The computer readable medium may also have stored thereon one or more digital files comprising computer readable instructions or software for reading, writing to, or otherwise managing and/or accessing the digital files. The computer readable medium may also comprise software or computer readable and/or executable instructions that cause the computing device to perform one or more steps of the methods of the present invention, including for example and without limitation, the step(s) associated with comparing a gene expression signature stored in a first digital file to one or more instances stored in a second digital file. In certain embodiments, the one or more digital files may form part of a database management system for managing the digital files. Non-limiting examples of database management systems are described in United States Patent Serial Nos. 4,967,341 and 5,297,279. The computer readable medium may form part of or otherwise be connected to the computing device. The computing device can be provided in a wide variety of forms, including but not limited to any suitable general or special purpose computer known in the art (e.g., server, desktop computer, laptop computer, mainframe computer).

In certain embodiments, an instance may be configured as an ordered listing of microarray probe set identifications ("IDs") (e.g., from 2 to 22,000 IDs or more). The ordered listing may be stored in a data structure of a digital file and the data arranged such that, when the digital file is read by computer software, a plurality of character strings are reproduced representing the ordered listing of probe set IDs. It may be desirable for each instance to include a full list of the probe set IDs, but it is contemplated that one or more of the instances may comprise less than all the probe set IDs of a microarray. It is also contemplated that the instances may include other data in addition to or in place of the ordered listing of probe set IDs. For example, an ordered listing of equivalent gene names and/or gene symbols may be substituted for the ordered listing of probe set IDs. Additional data may be stored with an instance and/or the digital file. In some embodiments, the additional data is referred to as metadata and can include one or more of cell line identification, batch number, exposure duration, and other empirical data, as well as any other descriptive material associated with an instance ID. The ordered list may also comprise a numeric value associated with each identifier that represents the ranked position of that identifier in the ordered list.

List(s) of microarray probe set IDs, if provided, are typically smaller lists of probe set IDs as compared to the instances described above. In certain embodiments, the list(s) may include between 2 and 1000 probe set IDs, for example, greater than 10, 50, 100, 200, or 300 probe set IDs and/or less than 800, 600, or 400 probe set IDs. The list(s) of probe set IDs of the may represent up and/or down-regulated genes selected to represent a type of periorbital dyschromia of interest. In certain embodiments, a first list may represent the up-regulated genes and a second list may represent the down-regulated genes of the gene expression signature.

Conventional microarrays suitable for use herein include Affymetrix GeneChips and Illumina BeadChips, both of which comprise probe sets and custom probe sets. To generate reference gene profiles suitable for use herein, chips designed for profiling the human genome are utilized. Suitable examples of Affymetrix chips include Human Genome brand HG-219 Plus 2.0 and HG-U129 model, HGU133GeneChips. A particularly suitable Affymetrix^{®} brand microarray employed by the instant investigators is model HG-219. Of course, it is to be appreciated that any chip or microarray, regardless of proprietary origin, may be suitable as long as the probe sets of the chips used to construct the data architecture are substantially similar to those according to the present invention.

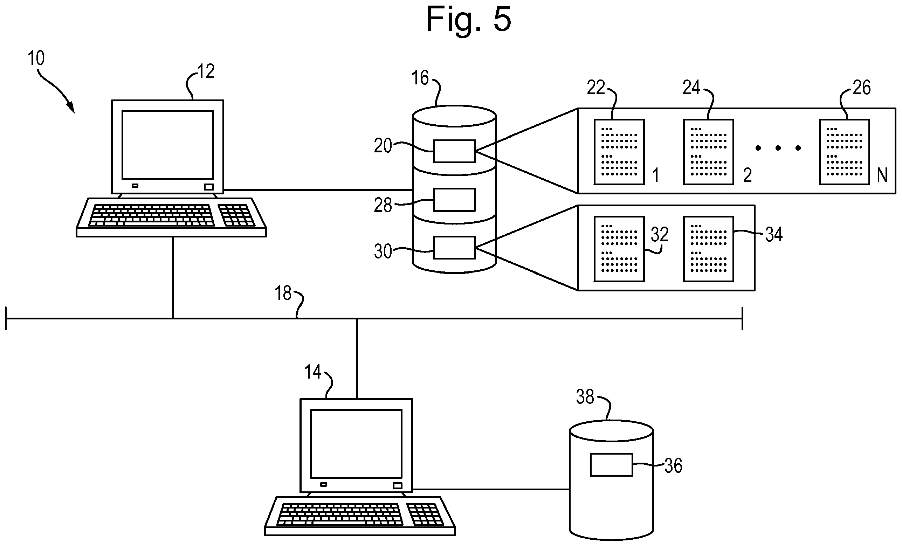

FIG. 5 illustrates an example of a system 10 suitable for use herein. The system 10 includes a first computing device 12 and a second computing device 14 in electronic communication with one another via communication network 18, which can a wired and/or wireless connection. The system 10 also includes a first computer readable medium 16 associated with the first computing device 12 and a second computer readable medium 38 associated with the second computing device 14. The computer readable medium(s) 16 and/or 38 may be electronically coupled to the first and/or second computer by any suitable means known in the art. Each computer readable medium 16, 38 may include one or more digital files 20, 30 and 36 stored thereon, which are accessible by at least one of the computing devices 12, 14. The first and second computing devices 12, 14 and/or first and second computer readable media 16, 38 may be located remotely from one another, but need not necessarily be so.

As illustrated in FIG. 5, the first computer readable medium 16 includes a digital file 20 comprising a plurality of instances 22, 24, and 26 configured in a data structure. The first computer readable medium 16 may also include a second digital file 30 comprising one or more lists 32 and 34 of microarray probe set IDs associated with one or more periorbital dyschromia-relevant gene expression signatures. The lists 32 and 34 of probe set IDs of the second digital file 30 may represent one or more up and/or down-regulated genes associated with one or more types of periorbital dyschromia. For example, the first list 32 may represent the up-regulated genes and the second list 34 may represent the down-regulated genes of a gene expression signature. The computer readable medium 16 illustrated in FIG. 5 also includes a software application 28 that enables a user to read, write, manage and/or otherwise access the digital files 20 and/or 30. The instances 22, 24 and 26 and/or lists 32 and 34 may be stored in a data structure of the digital files 20 and/or 30 and the data arranged so that, when the digital file is accessed by the software application 28, a plurality of character strings are produced representing the instances or list of probe set IDs. Instead of probe set IDs, equivalent gene names and/or gene symbols (or another nomenclature) may be substituted. Additional data may also be stored with the instances, gene expression signatures and/or the digital file (e.g., metadata), which may include any associated information, for example, cell line or sample source, and microarray identification.

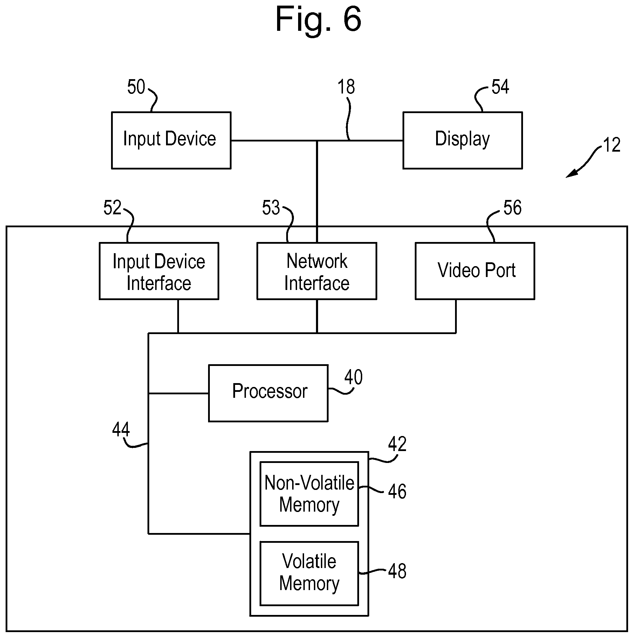

FIG. 6 illustrates an example of a computing device 12 suitable for use with the system 10 of FIG. 5. The computing device 12 may include one or more components commonly known for use with a computer including, without limitation, a processor 40 for executing stored instructions associated with one or more program applications or software; system memory 42; and a system bus 44 to provide an interface for system components (e.g., system memory 42 and processor 40). The system memory 42 may include non-volatile memory 46 (e.g., read only memory ("ROM") and the like) and/or volatile memory 48 (e.g., random access memory (RAM) and the like). The computing device 12 may include drives and associated computer-readable media to provide, e.g., non-volatile storage of data, data structures, data architecture, computer-executable instructions, and the like. Commands and information may be entered into the computing device 12 through one or more wired or wireless input devices 50, (e.g., keyboard, mouse, and/or touch screen), which may be in electronic communication with the processor 40 through an input device interface 52 coupled to the system bus 44. The computing device 12 may drive a separate or integral display device 54, which may also be connected to the system bus 44 via an interface, such as a video port 56. The computing device 12 may operate in a networked environment across network 18 using a wired and/or wireless network communications interface 58.

### CREATING A PLURALITY OF INSTANCES

In some embodiments, the methods comprise populating one or more digital files with a plurality of instances comprising data derived from gene expression profiling experiments, wherein one or more of the experiments comprise exposing, for example, keratinocyte cells (or other skin cells such as skin cells typically associated with the periorbital region, human skin equivalent cultures and *ex vivo* cultured human skin) to at least one perturbagen. In a particularly suitable example of an embodiment, an instance may consist of the rank ordered data for all of the probe sets on an AFFYMETRIX brand HG-U219 model GeneChip, wherein each probe on the chip has a unique probe set ID. The probe sets are rank ordered by the fold-change level of gene expression detected relative to controls in the same C-map batch (single instance/average of controls). The probe set identifiers are rank-ordered to reflect the most up-regulated to the most down-regulated.

Notably, even for non-differentially regulated genes, the signal values for a particular probe set are unlikely to be identical for a gene expression profile (e.g., associated with an instance or associated with a particular type of periorbital dyschromia) and a control profile. A fold-change different from 1 is calculated and can be used for comprehensive rank ordering. In accordance with methods disclosed by Lamb *et al.* (2006), data are adjusted using 2 thresholds to minimize the effects of genes that may have very low, noisy signal values. The thresholding is preferably performed before rank ordering. An example for illustrative purposes includes a process wherein a first threshold is set at 33%. If the signal for a probe set is below the threshold 33%, it is adjusted to to 33%. Ties for ranking are broken with a second threshold wherein the fold changes are recalculated and any values less than 5% of the gene expression values are set to the signal value at 5% threshhold. For any remaining ties, the order depends on the sorting algorithm used, but is essentially random. The probe sets in the middle of the list do not meaningfully contribute to an actual connectivity score.

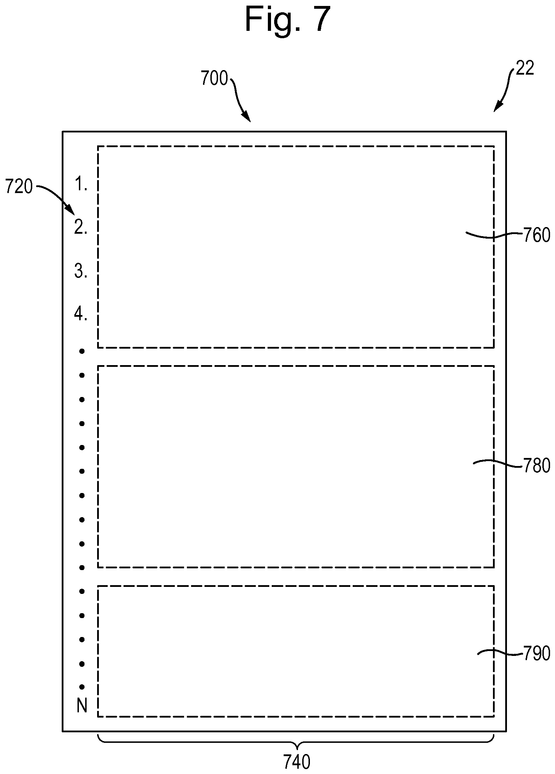

The rank ordered listing of probe IDs may be stored as an instance (e.g., 22 in FIG. 5) or a gene expression signature in a digital file (e.g., the first digital file 20 in FIG. 5). The probe IDs may be sorted into a list according to the level of gene expression regulation detected, wherein the list progresses from up-regulated to marginal or no regulation to down-regulated. Referring to FIG. 7, the data associated with an instance 22 (or gene expression profile associated with a type of periorbital dyschromia of interest) comprises the probe IDs 700 and a value 720 representing its ranking in the list (e.g., 1, 2, 3, 4 ... N, where N represents the total number of probes on the microarray). The ordered list 740 may generally comprise approximately three groupings of probe IDs: a first grouping 760 of probe IDs associated with up-regulated genes, a second group 780 of probe IDs associated with genes with marginal regulation or no detectable signal or noise, and a third group 790 of probe IDs associated with down-regulated genes. The most up-regulated genes are at or near the top of the ordered list 740 and the most down-regulated genes are at or near the bottom of the ordered list 740. The groupings are shown for illustration, but the lists for each instance 22 may be continuous and the number of regulated genes will depend on, e.g., the strength of the effect of the perturbagen associated with the instance. Other arrangements within the ordered list 740 may be provided. For example, the probe IDs associated with the down-regulated genes may be arranged at the top of the list 740. This instance data may also further comprise metadata such as perturbagen identification, perturbagen concentration, cell line or sample source, and microarray identification.

In some embodiments, one or more instances (or gene expression profiles) comprise at least about 1,000, 2,500, 5,000, 10,000, 20,000 or 50,000 identifiers and/or less than about 30,000, 25,000, 20,000 or 50,000 identifiers. In some embodiments, a database comprises at least about 50, 100, 250, 500, 1,000 or 5000 instances and/or less than about 50,000, 20,000, 15,000, 10,000, 7,500, 5,000, or 2,500 instances. Replicates of an instance may be created, and the same perturbagen may be used to derive a first instance from a particular cell type (e.g., keratinocyte cells) and a second instance from another target cell type or biological sample (e.g., fibroblasts, melanocytes, or complex tissue, such as, *ex vivo* human skin). In a very specific embodiment, an instance consists of the rank ordered data for all of the probe sets on the Affymetrix HG-U219 GeneChip wherein each probe on the chip has a unique probe set Identifier. The probe sets are rank ordered by the fold change relative to the controls in the same C-map batch (single instance/average of controls). The probe set Identifiers are rank-ordered to reflect the most up-regulated to the most down-regulated.

### CONSTRUCTING A GENE EXPRESSION SIGNATURE

A method of generating and/or identifying a gene expression signature representative of a type of periorbital dyschromia is provided. In certain embodiments, the method comprises (a) obtaining gene expression measurements from a test sample corresponding to a type of periorbital dyschromia; (b) identifying genes differentially expressed in the test sample by comparing the gene expression measurements of (a) with gene expression measurements for a control sample; (c) causing a computer to calculate a gene expression consistency value. The consistency value is representative of the significance of the difference in expression (b). The gene expression consistency value may be calculated by comparing log-odds ratios computed for the differentially expressed genes, and transforming the log-odds ratios using a sigmoid function. In certain embodiments, a one-tailed t-test against zero may be performed and log-odds ratios may be computed from the one-tailed t-test. The resulting gene expression consistency value is used to generate an ordered list of identifiers representing genes that are differentially expressed. The ordered list of identifiers is optionally associated with a numerical ranking for the identifier corresponding to its rank in the ordered list. The method may further comprise (d) creating an ordered list comprising identifiers representing consistently differentially expressed genes (i.e., genes differentially expressed in the tested biological conditions compared to the control sample), wherein the identifiers are ordered according to the gene expression consistency value computed in (c); and (e) storing the ordered list as a gene expression signature on at least one computer readable medium. The method optionally comprises using a programmable computer to perform one or more of steps (b), (c), (d), or (e).

Gene expression signatures may be generated from full thickness skin biopsies from skin exhibiting a periorbital dyschromia condition of interest compared to a control. For generation of an exemplary periorbital dyschromia gene expression signatures, biopsies are taken from the periorbital region and compared to non-affected skin sampled from the same subject (e.g., cheek, temple, forehead, chin, or an unaffected area of the periorbital region). It is to be appreciated that gene expression signatures may be generated by any suitable method known in the art.

The pathogenesis of periorbital dyschromia typically involves complex biological processes involving numerous known and unknown extrinsic and intrinsic factors, as well as responses to such factors that are subtle over a relatively short period of time but non-subtle over a longer period of time. This is in contrast to what is typically observed in drug development and drug screening methods, wherein a specific target, gene, or mechanism of action is of interest. Due to the unique screening challenges associated with periorbital dyschromia, the quality of the gene expression signature representing the condition of interest can be important for distinguishing between the gene expression data actually associated with a response to a perturbagen from the background expression data.

In various aspects, the gene expression signature references at least 2, 4, 5, 10, 20, 25, 30, or even at least 50 genes (e.g., 75 or more genes). Alternatively or in addition, the gene expression signature references no more than 10,000, 7,500, 5,000, 1,000, 800, 750, 700, 500, 50, 400, 350, 300, 250, 200, 150, 100, 70, 50, or even no more than 20 genes. For example, the gene expression signature optionally comprises identifiers corresponding to between about 5 and about 800 genes (e.g., between about 5 and about 400 genes, between about 10 and about 400 genes, between about 10 and about 200 genes, or between about 10 and about 140 genes). Exemplary periorbital dyschromia gene expression signatures comprise between about 100 and about 400 genes of similar numbers of up-regulated and/or down-regulated genes. For example, a suitable gene expression signature optionally comprises from about 100 to 150, from about 250 to 300, from about 300 to 350, or from about 350 to 400 up-regulated and down-regulated genes. It is to be appreciated that the number of genes will vary from biological condition to biological condition. When the biology is weaker, such as is the case typically with cosmetic condition phenotypes, fewer genes than those which may meet the statistical requisite for inclusion in the prior art, may be used to avoid adding genes that contribute to "noise." For example, where gene expression profiling analysis of a skin condition yields from between about 2,000 and 4,000 genes having a statistical p-value of less than 0.05 and approximately 1000 genes having a p-value of less than 0.001, a very strong biological response is indicated. A moderately strong biological response may yield approximately 800-2000 genes that have a statistical p-value of less than 0.05 combined with approximately 400-600 genes that have a p-value of less than 0.001. In these cases, a gene expression signature optionally comprises between about 100 and about 600 genes. Weaker biology is optionally represented by a gene expression signature comprising fewer genes, such as between about 20 and 100 genes. The invention further provides an immobilized array of oligonucleotides which hybridize to transcripts of between about 10 and about 400 genes, wherein the genes are selected from Tables 1 through 12, shown in Example 1 below.

In certain embodiments, a gene expression signature may be mapped onto a biological process grid or Gene Ontology to yield a physiological theme pattern as illustrated in Example 2 below. The broadest pattern would include all themes where genes are statistically clustered. A more circumscribed pattern includes, e.g., a subset of themes populated with the strongest-regulated genes, or a subset that is unique with respect to related disorders. Gene expression signatures derived from Gene Ontology and thematic pattern analysis will generally include fewer genes, and are a useful tool for differential diagnosis and screening for actives having very precise and targeted effects.

### COMPARING GENE EXPRESSION SIGNATURE(S) AND INSTANCES

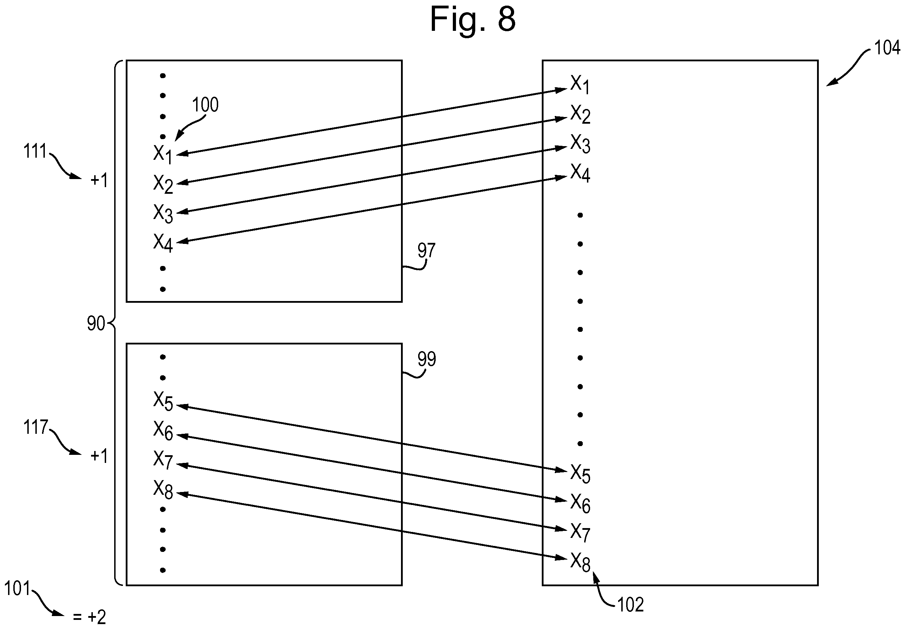

In certain embodiments, the method herein comprises causing a computer to query a data architecture of stored instances with a periorbital dyschromia gene expression signature, wherein each instance is associated with a perturbagen. The querying comprises comparing the periorbital dyschromia gene expression signature to each stored instance. This *in silico* method facilitates identification of perturbagens that induce a statistically significant change in expression of a statistically significant number of genes associated with one or more types of periorbital dyschromia, leading to the identification of potential new cosmetic agents for treating periorbital dyschromia or potential new uses of known cosmetic agents. In certain embodiments, the method comprises accessing with a computer a plurality of instances stored on at least one computer readable medium, accessing with a computer at least one periorbital dyschromia gene expression signature stored on the at least one computer readable medium, comparing with a computer the periorbital dyschromia gene expression signature to the plurality of instances, assigning with a computer a connectivity score to each of the plurality of instances, and identifying potential agents for treating the periorbital dyschromia (e.g., identifying at least one perturbagen associated with an instance having a negative connectivity score). The method further comprises formulating a personal care composition comprising the potential treatment agent. A connectivity score is a combination of an up-score and a down-score, wherein the up-score represents the correlation between the up-regulated genes of a gene expression signature and an instance and the down-score represents the correlation between the down-regulated genes of a gene expression signature and an instance. The up-score and down-score have, for example, values between +1 and -1. For an up-score (and down-score), a high positive value indicates that the corresponding perturbagen of an instance induced expression of genes corresponding to microarray probes specific for the up-regulated (or down-regulated) genes of the gene expression signature. A high negative value indicates that the corresponding perturbagen associated with the instance repressed (down-regulated) the expression of genes associated with microarray probes specific for the up-regulated (or down-regulated) genes of the gene signature. The up-score can be calculated by comparing each identifier of an up list of a gene expression signature comprising the up-regulated genes (e.g., Tables 1, 3, 5, 7, 9, and 11 and lists 93, 97, and 107 of FIGS. 8, 9 and 10) to an ordered instance list while the down-score can be calculated by comparing each identifier of a down list of a gene signature comprising the down-regulated genes (see, e.g., Tables 2, 4, 6, 8, 10 and 12 and down lists 95, 99, and 109 of FIGS. 8, 9 and 10) to an ordered instance list. In these embodiments, the gene expression signature comprises the combination of the up list and the down list.

In some embodiments, the connectivity score value may range from +2 (greatest positive connectivity) to -2 (greatest negative connectivity), wherein the connectivity score (e.g., 101, 103, and 105 of FIGS. 8, 9 and 10) is the combination of the up score (e.g., 111, 113, 115 of FIGS. 8, 9 and 10) and the down score (e.g., 117, 119, 121 of FIGS. 8, 9 and 10) derived by comparing each identifier of a gene signature to the identifiers of an ordered instance list. In other embodiments the connectivity range may be between +1 and -1. The strength of matching between a gene expression signature and an instance represented by the up scores and down scores and/or the connectivity score may be derived by one or more approaches known in the art and include, but are not limited to, parametric and non-parametric approaches. Examples of parametric approaches include Pearson correlation (or Pearson r) and cosine correlation. Examples of non-parametric approaches include Spearman's Rank (or rank-order) correlation, Kendall's Tau correlation, and the Gamma statistic. Optionally, in order to eliminate a requirement that all profiles be generated on the same microarray platform, a non-parametric, rank-based pattern matching strategy based on the Kolmogorov-Smirnov statistic (see M. Hollander et al. "Nonparametric Statistical Methods"; Wiley, New York, ed. 2, 1999)(see, e.g., pp. 178-185) is used. Where all expression profiles are derived from a single technology platform, similar results may be obtained using conventional measures of correlation, for example, the Pearson correlation coefficient.

In specific embodiments, the methods and systems herein may employ the nonparametric, rank-based pattern-matching strategy based on the Kolmogorov-Smirnov statistic, which has been refined for gene profiling data and is known as Gene Set Enrichment Analysis (GSEA) (see, e.g., Lamb *et al.* 2006 and Subramanian, A. et al. (2005) Proc.Natl.Acad Sci U.S.A, 102, 15545-15550). For each instance, a down score is calculated to reflect the match between the down-regulated genes of the query and the instance, and an up score is calculated to reflect the correlation between the up-regulated genes of the query and the instance. In certain embodiments the down-score and up-score each may range between -1 and +1. The combination represents the strength of the overall match between the query signature and the instance.

The combination of the up-score and down-score may be used to calculate an overall connectivity score for each instance, and in embodiments where up- and down-score ranges are set between -1 and +1, the connectivity score ranges from -2 to +2, and represents the strength of match between a query gene expression signature and the instance. The sign of the overall score is determined by whether the instance links positivity or negatively to the signature. Positive connectivity occurs when the perturbagen associated with an instance tends to up-regulate the genes in the up list of the signature and down-regulate the genes in the down list. Conversely, negative connectivity occurs when the perturbagen tends to reverse the up- and down- signature gene expression changes. The magnitude of the connectivity score is the sum of the absolute values of the up and down scores when the up and down scores have different signs. A high positive connectivity score predicts that the perturbagen will tend to induce the condition associated with the query gene expression signature, and a high negative connectivity score predicts that the perturbagen will tend to reverse the condition associated with the query gene expression signature. A zero score is assigned where the up- and down-scores have the same sign, indicating that a perturbagen did not have a consistent impact on the condition gene expression signature (e.g., up-regulating both the up and down lists).

According to Lamb *et al.* (2006), there is no standard for estimating statistical significance of connections observed. The power to detect connections may be greater for compounds with many replicates. Replicating in this context means that the same perturbagen is profiled multiple times. Profiling a perturbagen multiple times in each batch reduces batch to batch variation. Since microarray experiments tend to have strong batch effects, instances are optionally replicated in different batches (i.e., experiments) to increase confidence that connectivity scores are meaningful and reproducible.

Each instance may be rank ordered according to its connectivity score to the query gene expression signature, and the resulting rank ordered list displayed to a user using any suitable software and computer hardware allowing for visualization of data.

In some embodiments, the methods may comprise identifying from the displayed rank-ordered list of instances (i) the one or more perturbagens associated with the instances of interest (thereby correlating activation or inhibition of a plurality of genes listed in the query signature to the one or more perturbagens); (ii) the differentially expressed genes associated with any instances of interest (thereby correlating such genes with the one or more perturbagens, the periorbital dyschromia condition of interest, or both); (iii) the cells associated with any instance of interest (thereby correlating such cells with one or more of the differentially expressed genes, the one or more perturbagens, and the periorbital dyschromia condition of interest); or (iv) combinations thereof. The perturbagen(s) associated with an instance may be identified from the metadata stored in the database for that instance. However, one of skill in the art will appreciate that perturbagen data for an instance may be retrievably stored in and by other means. Because the identified perturbagens statistically correlate to activation or inhibition of genes listed in the query gene expression signature, and because the query gene expression signature is a proxy for a biological condition (e.g., periorbital dyschromia conditions of interest), the identified perturbagens may be candidates for new cosmetic agents, new uses of known cosmetic agents, or to validate known agents for known uses.

FIGS. 8, 9 and 10 schematically illustrate exemplary methods of querying instances 104 88 and 112 with one or more gene expression signatures 90, 94 and 108. Broadly, the methods comprise querying a data architecture of stored instances (e.g., skin instances) with one or more gene signatures (e.g., a periorbital dyschromia gene expression signature), and applying a statistical method to determine how strongly the gene expression signature genes match the regulated genes in an instance. Positive connectivity occurs when the genes in the up-regulated gene expression signature list are enriched among the up-regulated genes in an instance and the genes in the down-regulated gene expression signature list are enriched among the down-regulated genes in an instance. On the other hand, if the up-regulated genes of the gene expression signature are predominantly found among the down-regulated genes of the instance, and vice versa, this is scored as negative connectivity.

FIG. 8 schematically illustrates an extreme example of a positive connectivity between signature 90 and the instance 104. The instance 104 comprises probe IDs 102 ordered from most up-regulated (i.e., X₁) to most down-regulated (i.e., X₈). In this example, the probe IDs 100 (e.g., X₁, X₂ X₃, X₄, X₅, X₆, X₇, X₈) of the gene signature 90, comprising an up list 97 and a down list 99, have a one to one positive correspondence with the most up-regulated and down-regulated probe IDs 102 of the instance 104, respectively.

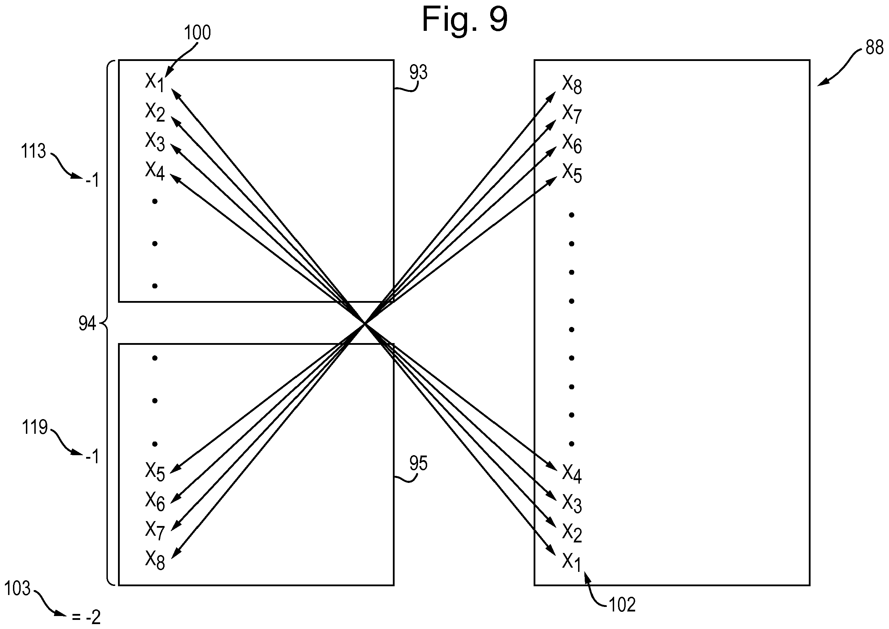

FIG. 9 schematically illustrates an extreme example of a negative connectivity between signature 94 and instance 88 comprising the probe IDs 100 and 102, respectively, wherein the probe IDs 102 of the instance 88 are ordered from most up-regulated (i.e., X₈) to most down-regulated (i.e., X₁). In this example, the probe IDs 100 of the up list 93 (i.e., X₁, X₂ X₃, X_{4...}) correspond exactly with the most down-regulated probe IDs 102 of the instance 88, and the probe IDs 100 of the down list 95 (i.e.,X₅, X₆, X₇, X_{8...}) correspond exactly to the most up-regulated probe IDs 102 of the instance 88.

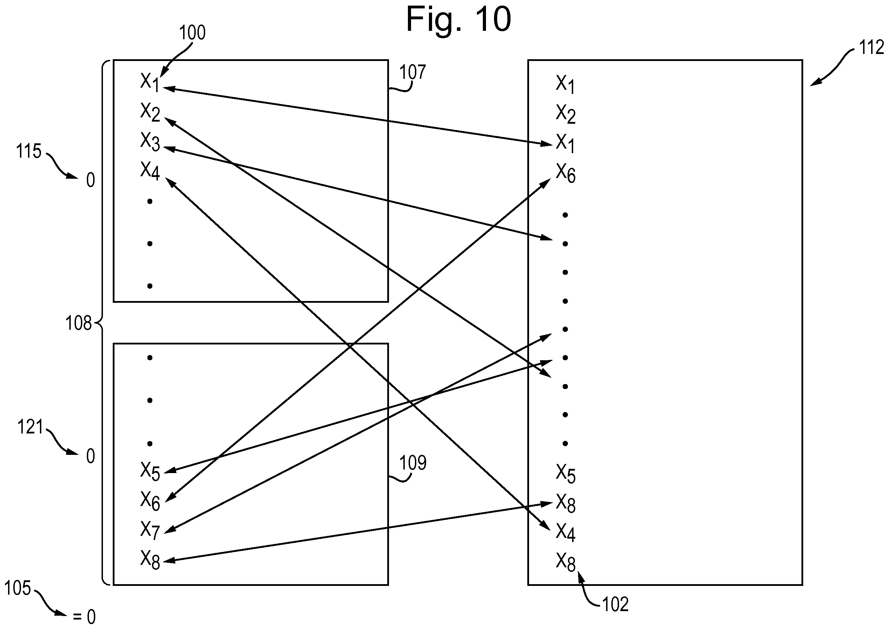

FIG. 10 schematically illustrates an extreme example of neutral connectivity, wherein there is no consistent enrichment of the up- and down-regulated genes of the signature 108 among the up- and down-regulated genes of the instance 112, either positive or negative. Hence the probe IDs 100 (e.g., X₁, X₂ X₃, X₄, X₅, X₆, X₇, X₈) of the gene signature 108 (comprising an up list 107 and a down list 109) are scattered with respect to rank with the probe IDs 102 of the instance 112, wherein the probe IDs 102 of the instance 112 are ordered from most up-regulated (i.e., X₁) to most down-regulated (i.e., X₈). While some examples herein may describe illustrate processes where a gene signature comprises both an "up list" and a "down list" representative of the most significantly up- and down-regulated genes of a skin condition, it is contemplated that a gene signature may comprise only an up list or a down list when the dominant biology associated with a condition of interest shows gene regulation in predominantly one direction.

### EVALUATING THE INFLUENCE OF PERTURBAGENS

The gene expression signatures described herein are useful for identifying connections between perturbagens and the appearance of periorbital dyschromia, i.e., determining whether a perturbagen modulates one or more aspects of skin health with respect to one or more types of periorbital dyschromia. For example, a periorbital dyschromia gene expression signature is useful for identifying agents that improve the appearance of one or more types of periorbital dyschromia, as well as evaluating candidate skin-active agents for activity against one or more types of periorbital dyschromia. Indeed, the materials and methods of the invention lend themselves to screening tens to hundreds of thousands of candidate active agents *in silico* to identify lead candidates for further evaluation using, e.g., *in vitro* and *ex vivo* methods. Connectivity mapping discovers functional connections between gene expression associated with a phenotype and cellular responses to perturbagens (see, e.g., Hughes et al., Cell, 102, 109-126 (2000); and Lamb et al., Science, 313, 1929-35 (2006)). In this regard, the systems and methods described herein can utilize connectivity mapping to predict the effectiveness of potential active agents for reducing or ameliorating the symptoms associated with the different types of periorbital dyschromia.

In certain embodiments, the method comprises querying a data architecture of stored instances with a periorbital dyschromia gene expression signature, wherien each stored instance is associated with a perturbagen. The querying comprises comparing the periorbital dyschromia gene expression signature to each stored skin instance (i.e., comparing each identifier in the gene expression signature list of the gene expression signature with the position of the same identifier in each instance list). Optionally, the method comprises querying a data architecture of stored instances associated with perturbagens that influence the biological conditions associated with one or more types of periorbital dyschromia. Also optionally, the comparison of the periorbital dyschromia gene expression signature to each stored skin instance comprises assigning a connectivity score to each of a plurality of instances. In various aspects, the method further comprises identifying an instance having a negative connectivity score (which represents a negative correlation between the gene expression signature and instance) and/or identifying an instance having a positive connectivity score (which represents a positive correlation between the gene expression signature and instance). The method also comprises formulating a composition for treating one or more types of periorbital dyschromia, the composition comprising a dermatologically acceptable carrier and the perturbagen associated with the identified instance.

### COSMETIC COMPOSITIONS AND PERSONAL CARE PRODUCTS

A method of formulating a skin care composition is also provided herein. The method comprises accessing a plurality of instances stored on at least one computer readable medium, wherein each instance is associated with a perturbagen (and optionally a skin cell type) and comprises an ordered list of a plurality of identifiers representing up-regulated genes and down-regulated genes. The method further comprises accessing at least one periorbital dyschromia gene expression signature stored on the computer readable medium. The periorbital dyschromia gene expression signature comprises one or more gene expression signature lists comprising a plurality of identifiers representing a plurality of up-regulated genes and a plurality of down-regulated genes associated with a type of periorbital dyschromia. The periorbital dyschromia gene expression signature is compared to the plurality of the instances, wherein the comparison comprises comparing each identifier in the one or more gene expression signature lists with the position of the same identifier in the ordered lists for each of the plurality of instances, and a connectivity score is assigned to each of the plurality of instances. The method further comprises formulating a skin care composition comprising a dermatologically acceptable carrier and at least one perturbagen, wherein the connectivity score of the instance associated with the at least one perturbagen is negative (i.e., there is a negative correlation between the instance and the query gene expression signature).

Generally, active agents identified for reducing the appearance of periorbital dyschromia may be applied in accordance with cosmetic compositions and formulation parameters well-known in the art. In certain embodiments, periorbital dyschromia may be generally treated by topical application of a suitable personal care product. Application of the personal care product may be limited to the periorbital region or may be applied to other portions of the face or even the entire face, for example, as part of a broader beauty regimen. The personal care product may be in the form of cosmetic composition comprising one or more test agents or compounds identified by a screening method described herein. In certain embodiments, the cosmetic composition may include a dermatological acceptable carrier, the test agent, and one or more optional ingredients of the kind commonly included in the particular cosmetic compositing being provided.

Dermatologically acceptable carriers should be safe for use in contact with human skin tissue. Suitable carriers may include water and/or water miscible solvents. The cosmetic composition may comprise from about 1% to about 95% by weight of water and/or water miscible solvent. The composition may comprise from about 1%, 3%, 5%, 10%, 15%, 20%, 25%, 30%, 35%, 40%, 45%, 50%, 55%, 60%, 65%, 70%, 75%, 80%, or 85% to about 90%, 85%, 80%, 75%, 70%, 65%, 60%, 55%, 50%, 45%, 40%, 35%, 30%, 25%, 20%, 15%, 10% , or 5% water and/or water miscible solvents. Suitable water miscible solvents include monohydric alcohols, dihydric alcohols, polyhydric alcohols, glycerol, glycols, polyalkylene glycols such as polyethylene glycol, and mixtures thereof. When the cosmetic composition is in the form of an emulsion, water and/or water miscible solvents are carriers typically associated with the aqueous phase.

Suitable carriers also include oils. The skin care composition may comprise from about 1% to about 95 % by weight of one or more oils. The composition may comprise from about 0.1%, 0.5%, 1%, 2%, 5%, 10%, 15%, 20%, 25%, 30%, 35%, 40%, 45%, 50%, 55%, 60%, 65%, 70%, 75%, 80%, 85%, or 90% to about 90%, 85%, 80%, 75%, 70%, 65%, 60%, 55%, 50%, 45%, 40%, 35%, 30%, 25%, 20%, 15%, 10%, 5%, or 3% of one or more oils. Oils may be used to solubilize, disperse, or carry materials that are not suitable for water or water soluble solvents. Suitable oils include silicones, hydrocarbons, esters, amides, ethers, and mixtures thereof. The oils may be volatile or nonvolatile.

Suitable silicone oils include polysiloxanes. Commercially available polysiloxanes include the polydimethylsiloxanes, which are also known as dimethicones, examples of which include the DM-Fluid series from Shin-Etsu, the Vicasil^{®} series sold by Momentive Performance Materials Inc., and the Dow Corning^{®} 200 series sold by Dow Corning Corporation. Specific examples of suitable polydimethylsiloxanes include Dow Corning^{®} 200 fluids (also sold as Xiameter^{®} PMX-200 Silicone Fluids) having viscosities of 0.65, 1.5, 50, 100, 350, 10,000, 12,500 100,000, and 300,000 centistokes.

Suitable hydrocarbon oils include straight, branched, or cyclic alkanes and alkenes. The chain length may be selected based on desired functional characteristics such as volatility. Suitable volatile hydrocarbons may have between 5-20 carbon atoms or, alternately, between 8-16 carbon atoms.

Other suitable oils include esters. The suitable esters typically contained at least 10 carbon atoms. These esters include esters with hydrocarbyl chains derived from fatty acids or alcohols (*e.g.*, mono-esters, polyhydric alcohol esters, and di- and tri-carboxylic acid esters). The hydrocarbyl radicals of the esters hereof may include or have covalently bonded thereto other compatible functionalities, such as amides and alkoxy moieties (*e.g*., ethoxy or ether linkages, etc.).

Other suitable oils include amides. Amides include compounds having an amide functional group while being liquid at 25 °C and insoluble in water. Suitable amides include N-acetyl-N-butylaminopropionate, isopropyl N-lauroylsarcosinate, and N,N,-diethyltoluamide. Other suitable amides are disclosed in U.S. Patent No. 6,872,401.

Other suitable oils include ethers. Suitable ethers include saturated and unsaturated fatty ethers of a polyhydric alcohol, and alkoxylated derivatives thereof. Exemplary ethers include C₄₋₂₀ alkyl ethers of polypropylene glycols, and di-C₈₋₃₀ alkyl ethers. Suitable examples of these materials include PPG-14 butyl ether, PPG-15 stearyl ether, dioctyl ether, dodecyl octyl ether, and mixtures thereof.

The cosmetic composition may comprise an emulsifier. An emulsifier is particularly suitable when the composition is in the form of an emulsion or if immiscible materials are being combined. The cosmetic composition may comprise from about 0.05%, 0.1%, 0.2%, 0.3%, 0.5%, or 1% to about 20%, 10%, 5%, 3%, 2%, or 1% emulsifier. Emulsifiers may be nonionic, anionic or cationic. Non-limiting examples of emulsifiers are disclosed in U.S. Patent 3,755,560, U.S. Patent 4,421,769, and McCutcheon's, Emulsifiers and Detergents, 2010 Annual Ed., published by M. C. Publishing Co.. Other suitable emulsifiers are further described in the Personal Care Product Council's International Cosmetic Ingredient Dictionary and Handbook, Thirteenth Edition, 2006, under the functional category of "Surfactants - Emulsifying Agents."

Linear or branched type silicone emulsifiers may also be used. Particularly useful polyether modified silicones include KF-6011, KF-6012, KF-6013, KF-6015, KF-6015, KF-6017, KF-6043, KF-6028, and KF-6038 from Shin Etsu. Also particularly useful are the polyglycerolated linear or branched siloxane emulsifiers including KF-6100, KF-6104, and KF-6105 from Shin Etsu. Emulsifiers also include emulsifying silicone elastomers. Suitable silicone elastomers may be in the powder form, or dispersed or solubilized in solvents such as volatile or nonvolatile silicones, or silicone compatible vehicles such as paraffinic hydrocarbons or esters. Suitable emulsifying silicone elastomers may include at least one polyalkyl ether or polyglycerolated unit.

Structuring agents may be used to increase viscosity, thicken, solidify, or provide solid or crystalline structure to the cosmetic composition. Structuring agents are typically grouped based on solubility, dispersibility, or phase compatibility. Examples of aqueous or water structuring agents include polymeric agents, natural or synthetic gums, polysaccharides, and the like. In one embodiment, the composition may comprises from about 0.0001%, 0.001%, 0.01%, 0.05%, 0.1%, 0.5%, 1%, 2%, 3%, 5% to about 25%, 20%, 10%, 7%, 5%, 4%, or 2%, by weight of the composition, of one or more structuring agents.

Polysaccharides and gums may be suitable aqueous phase thickening agents. Suitable classes of polymeric structuring agents include but are not limited to carboxylic acid polymers, polyacrylamide polymers, sulfonated polymers, high molecular weight polyalkylglycols or polyglycerins, copolymers thereof, hydrophobically modified derivatives thereof, and mixtures thereof. Silicone gums are another oil phase structuring agent. Another type of oily phase structuring agent includes silicone waxes. Silicone waxes may be referred to as alkyl silicone waxes which and are semi-solids or solids at room temperature. Other oil phase structuring agents may be one or more natural or synthetic waxes such as animal, vegetable, or mineral waxes.

The cosmetic compositions may be generally prepared by conventional methods known in the art of making topical cosmetic compositions and personal care products. Such methods typically involve mixing of ingredients in or more steps to a relatively uniform state, with or without heating, cooling, application of vacuum, and the like. Typically, emulsions are prepared by first mixing the aqueous phase materials separately from the fatty phase materials and then combining the two phases as appropriate to yield the desired continuous phase. The compositions are preferably prepared such as to optimize stability (physical stability, chemical stability, photostability, etc.) and/or delivery of active materials. The composition may be provided in a package sized to store a sufficient amount of the composition for a treatment period. The size, shape, and design of the package may vary widely. Certain package examples are described in USPNs D570,707; D391,162; D516,436; D535,191; D542,660; D547,193; D547,661; D558,591; D563,221; 2009/0017080; 2007/0205226; and 2007/0040306.

### EXAMPLES

### Example 1 - Deriving a Periorbital Dyschromia Gene Expression Signature.

In this example, gene expression profiles and phenotype themes were determined from biopsy samples of female test subjects aged 18 to 45 classified as having Type I, Type II or Type III periorbital dyschromia or no periorbital dyschromia ("No Dyschromia") by an expert grader. Thirteen subjects were classified as having Type I periorbital dyschromia, fourteen subjects were classified as having Type II periorbital dyschromia, seven subjects were classified as having Type III periorbital dyschromia, and 8 test subjects were classified as having No Dyschromia. 2 mm biopsies were taken from the lower eyelid of each test subject in the area approximately in line with the pupil where dyschromia appears and from a non-affected area (i.e., no skin discoloration) on the side of the face near the hairline adjacent to the lower eyelid. The biopsy samples may be collected, stored and sectioned by suitable conventional methods. A suitable example of a method of collecting biopsy samples and sectioning, storing and/or staining the samples is described in U.S. Provisional App. No. 61/798,208 filed by Osorio, et al., on March 15, 2013. In this example, the epidermis and dermis layers of the section biopsy samples were separated with a PALM Microbeam IV™ brand Laser-capture Miscrodissection ("LCM") system (available from Carl Zeiss MicroImaging GmbH, Germany) in accordance with the manufacturer's instructions.

After separating the epidermal and dermal layers of the sectioned biopsy samples, each layer was subjected to RNA extraction. In this example, RNA was extracted from each of the epidermis and dermis layers by utilizing an ARCTURS PICOPURE brand kit according to the manufacturers recommendation. RNA quantification and quality assurance was performed using AGILENCE 2100 bioanalyzer. Of course it is to be appreciated that any suitable means of extracting RNA from a tissue sample known in the art may be used. The extracted RNA was then run on a GeneTitan U219 brand microarray to identify the genes that were expressed. Two-sample t-tests were performed to examine differences between the periorbital dyschromia groups and the control group. A statistical analysis of the microarray data was performed to derive the periorbital dyschromia gene expression signatures. A general description of the statistical analysis is provided below. It is to be appreciated that while the statistical analysis below provides a suitable means of generating a gene signature from the microarray data, other statistical methods known in the art may also be suitable for use herein.

### Statistical Analysis

All the probe sets are sorted into sets of up-regulated and down-regulated sets using the statistical measure. In this example, a t-test was used to compute p-values, the values (positive and negative) of the t-statistic are used to sort the list since p-values are always positive. The sorted t-statistics will place the sets with the most significant p-values at the top and bottom of the list with the non-significant ones near the middle.

### Condition Signature Generation

### Filter non-expressed or noise genes using suitabledata organizing software

If a gene's expression mean value is too low (at bottom 30%), it is removed before any analysis starts. For gene signatures in this example, a gene's mean expression value must be in top 70%.

### Filter According to a Statistical Measure

Probes that are not statistically significant are eliminated using this filtering step. it may be desirable to use a suitable statistical measure such as, for example, p-values from a t-test, ANOVA, correlation coefficient, or other suitable model-based analysis. Limiting the gene signature list to genes that meet some reasonable cutoff (e.g., p ≤ 0.05, 0.01, 0.001, or even ≤ 0.0001 or less) for statistical significance compared to an appropriate control is important to allow selection of genes that are characteristic of the biological state of interest. This is preferable to using a fold change value, which does not take into account the noise around the measurements. For example, p-values may be chosen as the statistical measure and a cutoff value of p ≤ 0.05 may be chosen. The t-statistic was used in this example to select the probe sets in the signatures because it provides an indication of the directionality of the gene expression changes (i.e. up- or down-regulated) as well as statistical significance.

### Step 3: Filtering by fold change

Probes sets can be further filtered by fold changes. For example, fold change > 1.1 or 1.05 may used for both up-regulated genes and down-regulated genes ((mean of dyschromia/mean of non-dyschromia),).

### Step 4: Rank genes by p values and fold changes-

First, rank genes by p values ascendingly. If multiple genes have the same p value, then break/rank them by fold changes. For up-regulated signature genes, fold changes are ranked descendingly, and ascendingly for down-regulated signature genes. Select top ranked genes as CMap signature genes (this may be done twice to select up and down)

### Creation of the Gene Expression Signature

Using the filtered and sorted list created, a suitable number of probe sets from the top and bottom are selected to create a gene expression signature that preferably has approximately the same number of sets chosen from the top as chosen from the bottom. For example, the gene expression signature created may have at least 10, 50, 100, 200, or 300 and/or less than 800, 600, or about 400 genes corresponding to a probe set on the chip. The number of probe sets approximately corresponds to the number of genes, but a single gene may be represented by more than one probe set. It is understood that the phrase "number of genes" as used herein, corresponds generally with the phrase "number of probe sets." The number of genes included in the signature was based upon the observations in preliminary studies that indicated signatures with from 200 to 800 probe sets equally divided between up- and down-regulated genes provide stable results with regard to the top scoring chemical instances when using the signature to query the provided database.

U.S. Publication No. 2012/0283112 titled "Systems and Methods For Identifying Cosmetic Agents For Skin Care Compositions" filed by Binder, et al., on February 22, 2012; U.S. App. Serial No. 13/851,886, titled "Systems, Models and Methods for Identifying and Evaluating Skin-Active Agents Effective for Treating Conditions and Disorders of Skin Pigmentation," filed by Hakozaki, et al., on March 30, 2012; and U.S. Provisional App. No. 61/683,667, titeld "Systems, Models And Methods For Identifying And Evaluating Skin-Active Agents Effective For Treating An Array Of Skin Disorders" and filed on August 15, 2012 disclose suitable nonlimiting examples of methods of generating a gene expression profile.

Tables 1 through 12 below show gene expression signatures associated with each of Type I, Type II and Type III periorbital dyschromia, as compared to the gene expression signature for the No Dyschromia samples (control). The gene expression data was obtained by exracting RNA from lower eyelid biopsy samples obtained according to the biopsy method. Only genes that showed up-regulation or down-regulation with a p-value of less than 0.05 and fold change greater than 1.1 are shown. Table 1 shows the top 100 up-regulated genes expressed in the epidermis of subjects identified as having Type I periorbital dyschromia. Table 2 shows the top 100 down-regulated genes expressed in the epidermis of subjects identified as having Type I periorbital dyschromia. Table 3 shows the top 100 up-regulated genes expressed in the dermis of subjects identified as having Type I periorbital dyschromia. Table 4 shows the top 100 down-regulated genes expressed in the dermis of subjects identified as having Type I periorbital dyschromia. Table 5 shows the top 100 up-regulated genes expressed in the epidermis of subjects identified as having Type II periorbital dyschromia. Table 6 shows the top 100 down-regulated genes expressed in the epidermis of subjects identified as having Type II periorbital dyschromia. Table 7 shows the top 71 up-regulated genes expressed in the dermis of subjects identified as having Type II periorbital dyschromia. Table 8 shows the top 100 down-regulated genes expressed in the dermis of subjects identified as having Type II periorbital dyschromia. Table 9 shows the top 100 up-regulated genes expressed in the epidermis of subjects identified as having Type III periorbital dyschromia. Table 10 shows the top 100 down-regulated genes expressed in the epidermis of subjects identified as having Type III periorbital dyschromia. Table 11 shows the top 100 up-regulated genes expressed in the dermis of subjects identified as having Type III periorbital dyschromia. Table 12 show the top 100 down-regulated genes expressed in the dermis of subjects identified as having Type III periorbital dyschromia. The periorbital dyschromia gene expression signatures herein may optionally comprise between about 80% and about 100% of the up-regulated and/or down-regulated genes set forth in Tables 1 through 12, which represent the most up- and down-regulated genes common in each type of periorbital dyschromia.

**Table 1 - Type I Epidermis; Up-regulated**

| GeneTitan_ID | Gene | Title | Public ID | p |
|---|---|---|---|---|
| 11719428_a_at | BTBD7 | BTB (POZ) domain containing 7 | AI580162 | 0.000259 |
| 11724297_a_at | BMP7 | bone morphogenetic protein 7 | NM_001719.2 | 0.000396 |
| 11715192_s_art | C7orf46 | chromosome 7 open reading frame 46 | g188219621 | 0.000446 |
| 11724759_s_at | CALM1 | calmodulin 1 (phosphorylase kinase, delta) | NM_006888.3 | 0.000486 |
| 11735610_a_at | RPS6KA5 | ribosomal protein S6 kinase, 90kDa, polypeptide 5 | AF074393.1 | 0.000746 |
| 11758022_s_at | TNNI2 | troponin I type 2 (skeletal, fast) | AA728828 | 0.00151 |
| 11721260_a_at | WDR47 | WD repeat domain 47 | NM_001142550.1 | 0.001528 |
| 11727979_a_at | MAN2B2 | mannosidase, alpha, class 2B, member 2 | BC094773.1 | 0.002024 |
| 11753967_a_at | SLC6A2 | solute carrier family 6 (neurotransmitter transporter, noradrenalin), member 2 | AK301811.1 | 0.002393 |
| 11722090_a_at | EFNA4 | ephrin-A4 | NM_005227.2 | 0.002687 |
| 11758092_s_at | EFNA5 | ephrin-A5 | BE464799 | 0.002776 |
| 11717146_at | PTPN1 | protein tyrosine phosphatase, non-receptor type 1 | NM_002827.2 | 0.002876 |
| 11716283_at | PAPD7 | PAP associated domain containing 7 | NM_006999.3 | 0.003497 |
| 11746140_a_at | ARHGEF26 | Rho guanine nucleotide exchange factor (GEF) 26 | AF415176.1 | 0.003751 |
| 11724038_a_at | PTGS2 | prostaglandin-endoperoxide synthase 2 (prostaglandin G/H synthase and cyclooxygenase) | BC013734.1 | 0.003867 |
| 11723156_a_at | LSP1 | lymphocyte-specific protein 1 | NM_001013254.1 | 0.004011 |
| 11729840_s_at | ZCCHC2 | zinc finger, CCHC domain containing 2 | NM_017742.4 | 0.004086 |
| 11754302_a_at | ATP2A2 | ATPase, Ca++ transporting, cardiac muscle, slow twitch 2 | BM676899 | 0.004156 |
| 11754447_a_at | RPS6KA5 | ribosomal protein S6 kinase, 90kDa, polypeptide 5 | BM968829 | 0.00436 |
| 11739724_a_at | ATP2B2 | ATPase, Ca++ transporting, plasma membrane 2 | AL138283 | 0.004438 |
| 11723075_a_at | BCL9L | B-cell CLL/lymphoma 9-like | AY296059.1 | 0.00493 |
| 11717385_a_at | MT1G | metallothionein 1G | NM_005950.1 | 0.005447 |
| 11758557_s_at | ZFP36L1 | zinc finger protein 36, C3H type-like 1 | AI758505 | 0.005544 |
| 11724628_a_at | MAT1A | methionine adenosyltransferase I, alpha | NM_000429.2 | 0.005576 |
| 11744955_a_at | ANXA1 | annexin A1 | AK296808.1 | 0.005629 |
| 11718966_at | NUFIP2 | nuclear fragile X mental retardation protein interacting protein 2 | BU533767 | 0.006124 |
| 11744953_a_at | ANXA1 | annexin A1 | BC034157.1 | 0.007553 |
| 11717387_x_at | MT1G | metallothionein 1G | NM_005950.1 | 0.007682 |
| 11727642_a_at | TRERF1 | transcriptional regulating factor 1 | AF111801.1 | 0.0078 |
| 11745806_a_at | AMMECR1L | AMME chromosomal region gene 1-like | AK095871.1 | 0.008163 |
| 11717514_a_at | ANXA1 | annexin A1 | NM_000700.1 | 0.008725 |
| 11744954_x_at | ANXA1 | annexin A1 | BC034157.1 | 0.009223 |
| 11743864_a_at | ATP2B1 | ATPase, Ca++ transporting, plasma membrane 1 | S49852.1 | 0.009249 |
| 11717927_at | FOXK2 | forkhead box K2 | NM_004514.3 | 0.009268 |
| 11717386_s_at | MT1G | metallothionein 1G | NM_005950.1 | 0.009421 |
| 11730080_x_at | IL28RA | interleukin 28 receptor, alpha (interferon, lambda receptor) | NM_170743.2 | 0.009429 |
| 11763297_x_at | CCDC76 | coiled-coil domain containing 76 | BQ614335 | 0.010042 |
| 11726119_at | RPGR | retinitis pigmentosa GTPase regulator | AK291832.1 | 0.01012 |
| 11739805_a_at | RASAL2 | RAS protein activator like 2 | AK075169.1 | 0.010243 |
| 11715190_s_at | C7orf46 | chromosome 7 open reading frame 46 | g188219617 | 0.010448 |
| 11726351_at | EFNA5 | ephrin-A5 | CB240929 | 0.010459 |
| 11738035_s_at | RTN4 | reticulon 4 | AK302741.1 | 0.010618 |
| 11724104_s_at | SGK3 | serum/glucocorticoid regulated kinase family, member 3 | NM_001033578.1 | 0.010735 |
| 11729396_a_at | NEK1 | NIMA (never in mitosis gene a)-related kinase 1 | Z25431.1 | 0.010781 |
| 11723592_at | LRRC8C | leucine rich repeat containing 8 family, member C | NM_032270.4 | 0.011015 |
| 11757982_s_at | KIF21A | kinesin family member 21A | N39407 | 0.011191 |
| 11731899_s_at | PPAT | phosphoribosyl pyrophosphate amidotransferase | D13757.1 | 0.01137 |
| 11726633_s_at | TRIM8 | tripartite motif-containing 8 | BC021925.1 | 0.011932 |
| 11725675_a_at | RORA | RAR-related orphan receptor A | AA034012 | 0.012081 |
| 11745021_a_at | MYC | v-myc myelocytomatosis viral oncogene homolog (avian) | K02276.1 | 0.012276 |
| 11758246_s_at | ARL4D | ADP-ribosylation factor-like 4D | BM719529 | 0.012438 |
| 11726634_a_at | MYST3 | MYST histone acetyltransferase (monocytic leukemia) 3 | NM_001099412.1 | 0.012468 |
| 11735421_a_at | NKD2 | naked cuticle homolog 2 (Drosophila) | AF358137.1 | 0.013343 |
| 11723821_a_at | SMURF2 | SMAD specific E3 ubiquitin protein ligase 2 | AY014180.1 | 0.013348 |
| 11733165_a_at | YIPF5 | Yip1 domain family, member 5 | NM_001024947.2 | 0.013814 |
| 11723169_s_at | FOXN2 | forkhead box N2 | NM_002158.3 | 0.014398 |
| 11728958_x_at | E2F8 | E2F transcription factor 8 | NM_024680.2 | 0.014717 |
| 11762525_s_at | | | AF339086.1 | 0.015079 |
| 11731645_a_at | CAMKK2 | calcium/calmodulin-dependent protein kinase kinase 2, beta | BC026060.2 | 0.016269 |
| 11730893_a_at | UBA6 | ubiquitin-like modifier activating enzyme 6 | EF623993.1 | 0.016784 |
| 11730360_at | CCDC126 | coiled-coil domain containing 126 | NM_138771.3 | 0.016798 |
| 11736426_s_at | CLIP4 | CAP-GLY domain containing linker protein family, member 4 | BM994685 | 0.017149 |
| 11725820_s_at | PAQR3 | progestin and adipoQ receptor family member III | NM_001040202.1 | 0.01721 |
| 11743411_a_at | RBM25 | RNA binding motif protein 25 | BG251218 | 0.017641 |
| 11718873_a_at | ATP2A2 | ATPase, Ca++ transporting, cardiac muscle, slow twitch 2 | NM_170665.3 | 0.018009 |
| 11739797_a_at | RFX2 | regulatory factor X, 2 (influences HLA class II expression) | NM_134433.2 | 0.018119 |
| 11739408_at | MLL3 | myeloid/lymphoid or mixed-lineage leukemia 3 | DN917896 | 0.018481 |
| 11734164_a_at | ARHGEF26 | Rho guanine nucleotide exchange factor (GEF) 26 | NM_015595.3 | 0.018897 |
| 11721039_a_at | SOLH | small optic lobes homolog (Drosophila) | NM_005632.2 | 0.019352 |
| 11721053_s_at | KLHDC5 | kelch domain containing 5 | NM_020782.1 | 0.01948 |
| 11753735_x_at | TMSB4X | thymosin beta 4, X-linked | BC101792.1 | 0.019698 |
| 11748149_a_at | FNBP1 | formin binding protein 1 | AK293743.1 | 0.019714 |
| 11728706_x_at | EMP2 | epithelial membrane protein 2 | DB374012 | 0.020127 |
| 11717989_a_at | SUN1 | Sad1 and UNC84 domain containing 1 | NM_001130965.1 | 0.020293 |
| 11722448_at | KCNMB4 | potassium large conductance calcium-activated channel, subfamily M, beta member 4 | AF160967.1 | 0.020548 |
| 11727512_at | UBN2 | ubinuclein 2 | CA775887 | 0.020981 |
| 11759962_at | TPRKB | TP53RK binding protein | AY643713.1 | 0.021007 |
| 11728603_a_at | CLDN23 | claudin 23 | NM_194284.2 | 0.0211 |
| 11743497_at | BMP2 | bone morphogenetic protein 2 | BX101090 | 0.021788 |
| 11718874_s_at | ATP2A2 | ATPase, Ca++ transporting, cardiac muscle, slow twitch 2 | NM_170665.3 | 0.0218 |
| 11721216_s_at | TMEM106B | transmembrane protein 106B | AA789109 | 0.021968 |
| 11718006_a_at | MYLIP | myosin regulatory light chain interacting protein | BC002860.2 | 0.02198 |
| 11719104_s_at | CPNE3 | copine III | BC066597.1 | 0.022194 |
| 11723130_a_at | ARHGEF7 | Rho guanine nucleotide exchange factor (GEF) 7 | NM_003899.3 | 0.022803 |
| 11757700_a_at | NDFIP2 | Nedd4 family interacting protein 2 | AA521251 | 0.022979 |
| 11718081_a_at | ATP2B4 | ATPase, Ca++ transporting, plasma membrane 4 | NM_001001396.1 | 0.023666 |
| 11748034_a_at | CMAH | cytidine monophosphate-N-acetylneuraminic acid hydroxylase (CMP-N-acetylneuraminate monooxygenase)pseudogene | D86324.1 | 0.02381 |
| 11740148_x_at | ZNF429 | zinc finger protein 429 | NM_001001415.2 | 0.023983 |
| 11716408_a_at | MET | met proto-oncogene (hepatocyte growth factor receptor) | NM_001127500.1 | 0.024171 |
| 11720082_at | CBX6 | chromobox homolog 6 | NM_014292.3 | 0.024178 |
| 11733076_x_at | PPP1R12A | protein phosphatase 1, regulatory (inhibitor) subunit 12A | AK314193.1 | 0.024224 |
| 11743280_a_at | WNK1 | WNK lysine deficient protein kinase 1 | BC013629.2 | 0.024246 |
| 11719028_a_at | PSD3 | pleckstrin and Sec7 domain containing 3 | DB314358 | 0.024644 |
| 11757869_s_at | AKAP13 | A kinase (PRKA) anchor protein 13 | BE504033 | 0.024753 |
| 11722290_a_at | ZBTB43 | zinc finger and BTB domain containing 43 | AI745225 | 0.024875 |
| 11743865_s_at | ATP2B1 | Ca++ transporting, plasma membrane 1 | AI337321 | 0.025057 |
| 11734994_s_at | SKI | v-ski sarcoma viral oncogene homolog (avian) | NM_003036.3 | 0.025378 |
| 11731857_x_at | MT1H | metallothionein 1H | NM_005951.2 | 0.026268 |
| 11758247_x_at | ARL4D | ADP-ribosylation factor-like 4D | BM719529 | 0.026757 |
| 11731787_x_at | ERC1 | ELKS/RAB6-interacting/CAST family member 1 | NM_178037.1 | 0.026811 |

**Table 2 - Type I Epidermis; Down-regulated**

| GeneTitan_ID | Gene | Title | Public ID | p |
|---|---|---|---|---|
| 11763233_x_at | TRAC | T cell receptor alpha constant | EU427374.1 | 0.000086 |
| 11750712_a_at | SEMA4A | sema domain, immunoglobulin domain (Ig), transmembrane domain (TM) and short cytoplasmic domain, (semaphorin) 4A | AK296693.1 | 0.000205 |
| 11743350_a_at | C15orf48 | chromosome 15 open reading frame 48 | CA309087 | 0.000277 |
| 11723817_at | ARHGAP29 | Rho GTPase activating protein 29 | BU620659 | 0.000376 |
| 11723854_at | SAMD9 | sterile alpha motif domain containing 9 | NM_017654.2 | 0.000398 |
| 11754862_a_at | RARRES2 | retinoic acid receptor responder (tazarotene induced) 2 | AK092804.1 | 0.000534 |
| 11729515_a_at | SLC26A9 | solute carrier family 26, member 9 | NM_052934.3 | 0.001204 |
| 11748896_s_at | CCRL1 | chemokine (C-C motif) receptor-like 1 | AK304461.1 | 0.001209 |
| 11761960_x_at | TRAV20 | T cell receptor alpha variable 20 | AY532913.1 | 0.001367 |
| 11741285_a_at | CCRL1 | chemokine (C-C motif) receptor-like 1 | BC069438.1 | 0.00144 |
| 11754482_a_at | PSMB1 | proteasome (prosome macropain) subunit, beta type, 1 | BQ006450 | 0.001693 |
| 11754184_a_at | ALDH1A3 | aldehyde dehydrogenase 1 family, member A3 | BX538027.1 | 0.001883 |
| 11726829_at | TYW1B | tRNA-yW synthesizing protein 1 homolog B (S. cerevisiae) | NM_001145440.1 | 0.001956 |
| 11725368_at | LRG1 | leucine-rich alpha-2-glycoprotein 1 | NM_052972.2 | 0.001997 |
| 11723561_x_at | C11orf75 | chromosome 11 open reading frame 75 | NM_020179.2 | 0.002048 |
| 11734507_s_at | MECOM | MDS1 and EVI1 complex locus | AK292865.1 | 0.002142 |
| 11716805_s_at | PLEKHA2 | pleckstrin homology domain containing, family A (phosphoinositide binding specific) member 2 | NM_021623.1 | 0.002256 |
| 11754057_x_at | CRABP2 | cellular retinoic acid binding protein 2 | BT019827.1 | 0.00239 |
| 11737108_a_at | CCRL1 | chemokine (C-C motif) receptor-like 1 | NM_178445.1 | 0.002438 |
| 11720080_at | NTRK2 | neurotrophic tyrosine kinase, receptor, type 2 | NM_001007097.1 | 0.002467 |
| 11715767_s_at | ACAA2 | acetyl-CoA acyltransferase 2 | NM_006111.2 | 0.002477 |
| 11715670_a_at | IFITM1 | interferon induced transmembrane protein 1 (9-27) | NM_003641.3 | 0.002619 |
| 11735937_a_at | CD48 | CD48 molecule | NM_001778.2 | 0.002662 |
| 11746503_a_at | EHF | efts homologous factor | AF203977.1 | 0.00321 |
| 11736806_at | GABRA4 | gamma-aminobutyric acid (GABA) A receptor, alpha 4 | NM_000809.2 | 0.003627 |
| 11720157_at | GDA | guanine deaminase | AK295716.1 | 0.003854 |
| 11720132_a_at | SPIRE1 | spire homolog 1 (Drosophila) | BC125206.1 | 0.003917 |
| 11725832_s_at | OTUB2 | OTU domain, ubiquitin aldehyde binding 2 | NM_023112.3 | 0.004105 |
| 11717763_a_at | MGLL | monoglyceride lipase | BC006230.2 | 0.004157 |
| 11756683_a_at | CD1E | CDle molecule | AK311643.1 | 0.004158 |
| 11728008_x_at | FUT3 | fucosyltransferase 3 (galactoside 3(4)-L-fucosyltransferase, Lewis blood group) | NM_000149.3 | 0.004308 |
| 11750244_a_at | MGLL | monoglyceride lipase | AK304844.1 | 0.004332 |
| 11724346_a_at | IFIH1 | interferon induced with helicase C domain 1 | NM_022168.2 | 0.004365 |
| 11750245_x_at | MGLL | monoglyceride lipase | AK304844.1 | 0.004459 |
| 11758377_s_at | TLR1 | toll-like receptor 1 | BU623316 | 0.004771 |
| 11740897_a_at | TREX2 | three prime repair exonuclease 2 | NM_080701.3 | 0.004847 |
| 11718077_s_at | MAPKAPK3 | mitogen-activated protein kinase-activated protein kinase 3 | NM_004635.3 | 0.004908 |
| 11763697_s_at | SNHG9 | small nucleolar RNA host gene 9 (non-protein coding) | AW958849 | 0.004956 |
| 11719591_s_at | GLTP | glycolipid transfer protein | NM_016433.3 | 0.005188 |
| 11715671_x_at | IFITM1 | interferon induced transmembrane protein 1 (9-27) | NM_003641.3 | 0.005216 |
| 11725641_at | EFHD2 | EF-hand domain family, member D2 | CB240768 | 0.005381 |
| 11727633_at | SLC16A10 | solute carrier family 16, member 10 (aromatic amino acid transporter) | BC066985.1 | 0.005429 |
| 11717764_x_at | MGLL | monoglyceride lipase | BC006230.2 | 0.005967 |
| 11750324_a_at | GAS7 | growth arrest-specific 7 | AK293755.1 | 0.006052 |
| 11735833_a_at | KIAA1199 | KIAA1199 | NM_018689.1 | 0.006555 |
| 11757367_s_at | HSPA7 | heat shock 70kDa protein 7 (HSP70B) | BM677874 | 0.006575 |
| 11741263_s_at | LTBP1 | latent transforming growth factor beta binding protein 1 | M34057.1 | 0.006584 |
| 11723235_a_at | IF144L | interferon-induced protein 44-like | AB000115.1 | 0.006657 |
| 11729692_a_at | SERPINB3 | serpin peptidase inhibitor, clade B (ovalbumin), member 3 | EU852041.1 | 0.006806 |
| 11727385_a_at | PCCA | propionyl CoA carboxylase, alpha polypeptide | NM_000282.2 | 0.006811 |
| 11729742_x_at | IFI27L2 | interferon, alpha-inducible protein 27-like 2 | NM_032036.2 | 0.006823 |
| 11731973_at | SCNN1G | sodium channel, nonvoltage-gated 1, gamma | NM_001039.3 | 0.00716 |
| 11743404_at | ZMAT2 | zinc finger, matrin-type 2 | BM450158 | 0.007256 |
| 11736058_s_at | C10orf32 | chromosome 10 open reading frame 32 | NM_001136200.1 | 0.007371 |
| 11715239_x_at | IFITM3 | interferon induced transmembrane protein 3 (1-8U) | g148612841 | 0.007374 |
| 11726145_at | CA6 | carbonic anhydrase VI | NM_001215.2 | 0.007409 |
| 11718850_a_at | SRPK1 | SRSF protein kinase 1 | AK299591.1 | 0.007419 |
| 11729693_at | SERPINB3 | serpin peptidase inhibitor, clade B (ovalbumin), member 3 | EU852041.1 | 0.007514 |
| 11755950_a_at | CCDC71 | coiled-coil domain containing 71 | AK098658.1 | 0.007621 |
| 11740349_at | RNASE7 | ribonuclease, RNase A family, 7 | BC112334.1 | 0.007692 |
| 11757300_s_at | ELOVL5 | ELOVL family member 5, elongation of long chain fatty acids (FEN1/Elo2, SUR4/Elo3-like, yeast) | AL576414 | 0.007702 |
| 11725310_at | CRISP3 | cysteine-rich secretory protein 3 | NM_006061.1 | 0.007915 |
| 11723490_at | GCLM | glutamate-cysteine ligase, modifier subunit | BC041809.1 | 0.008288 |
| 11757595_x_at | CRABP2 | cellular retinoic acid binding protein 2 | BU631189 | 0.008352 |
| 11752101_s_at | EIF2S1 | eukaryotic translation initiation factor 2, subunit 1 alpha, 35kDa | BC002513.2 | 0.008373 |
| 11737496_a_at | CD200R1 | CD200 receptor 1 | NM_170780.2 | 0.008378 |
| 11749745_a_at | SRP68 | signal recognition particle 68kDa | AK301100.1 | 0.008462 |
| 11718142_a_at | TTC27 | tetratricopeptide repeat domain 27 | NM_017735.3 | 0.00876 |
| 11737432_a_at | PAPL | iron/zinc purple acid phosphatase-like protein | BC136722.1 | 0.008796 |
| 11753762_x_at | KLK6 | kallikrein-related peptidase 6 | AY457039.1 | 0.008902 |
| 11725875_at | WDR66 | WD repeat domain 66 | NM_144668.4 | 0.008916 |
| 11729694_s_at | SERPINB4 | serpin peptidase inhibitor, clade B (ovalbumin), member 4 | EU852041.1 | 0.009199 |
| 11763184_at | IDE | insulin-degrading enzyme | BQ006777 | 0.009364 |
| 11737743_a_at | ARSF | arylsulfatase F | NM_004042.3 | 0.009395 |
| 11724785_x_at | MRPS18C | mitochondrial ribosomal protein S18C | BC005186.1 | 0.009553 |
| 11723899_a_at | DHRS9 | dehydrogenase/reductase (SDR family) member 9 | NM_005771.4 | 0.009689 |
| 11743805_s_at | MRPL42 | mitochondrial ribosomal protein L42 | DB379276 | 0.009847 |
| 11758083_s_at | HPGD | hydroxyprostaglandin dehydrogenase 15-(NAD) | AI743714 | 0.009851 |
| 11727995_a_at | SPINK5 | serine peptidase inhibitor, Kazal type 5 | DQ149928.1 | 0.010277 |
| 11727208_x_at | DDHD1 | DDHD domain containing 1 | NM_030637.1 | 0.010297 |
| 11737431_x_at | PAPL | iron/zinc purple acid phosphatase-like protein | NM_001004318.2 | 0.010375 |
| 11720510_a_at | APOBEC3G | apolipoprotein B mRNA editing enzyme, catalytic polypeptide-like 3G | NM_021822.2 | 0.010471 |
| 11719503_a_at | DHX36 | DEAH (Asp-Glu-Ala-His) box polypeptide 36 | NM_020865.2 | 0.010702 |
| 11753152_x_at | CFLAR | CASP8 and FADD-like apoptosis regulator | AK297387.1 | 0.010705 |
| 11726289_at | GRAMD3 | GRAM domain containing 3 | BC008590.1 | 0.010722 |
| 11722661_at | NFE2L3 | nuclear factor (erythroid-derived 2)-like 3 | NM_004289.6 | 0.010893 |
| 11716743_s_at | TJP2 | tight junction protein 2 (zona occludens 2) | NM_004817.2 | 0.011228 |
| 11726894_a_at | IRAK3 | interleukin-1 receptor-associated kinase 3 | BG929347 | 0.011364 |
| 11753202_s_at | SERPINB4 | serpin peptidase inhibitor, clade B (ovalbumin), member 4 | AB046400.1 | 0.011604 |
| 11739292_at | EHF | ets homologous factor | NM_012153.3 | 0.012013 |
| 11748253_a_at | SLC5A1 | solute carrier family 5 (sodium/glucose cotransporter), member 1 | AK297665.1 | 0.01209 |
| 11723953_a_at | CLINT1 | clathrin interactor 1 | NM_014666.2 | 0.01254 |
| 11736117_a_at | ZFAND5 | zinc finger, AN1-type domain 5 | AF062346.1 | 0.012593 |
| 11724795_at | ZG16B | zymogen granule protein 16 homolog B (rat) | NM_145252.2 | 0.013029 |
| 11717765_a_at | MGLL | monoglyceride lipase | NM_007283.5 | 0.01317 |
| 11744194_a_at | ABCC3 | ATP-binding cassette, subfamily C (CFTR/MRP), member 3 | CB055248 | 0.013406 |
| 11746088_a_at | IFI44 | interferon-induced protein 44 | DB350079 | 0.013466 |
| 11733533_at | CYP4F22 | cytochrome P450, family 4, subfamily F, polypeptide | NM_173483.3 | 0.013708 |
| 11746581_a_at | PCCA | propionyl CoA carboxylase, alpha polypeptide | AK298318.1 | 0.013745 |
| 11717981_a_at | ACP5 | acid phosphatase 5, tartrate resistant | NM_001611.3 | 0.014935 |

**Table 3 - Type I Dermis; Up-regulated**

| GeneTitan_ID | Gene | Title | Public ID | p |
|---|---|---|---|---|
| 11733167_at | LRRN4CL | LRRN4 C-terminal like | BC053902.1 | 0.000112 |
| 11720616_a_at | DNM1 | dynamin 1 | NM_001005336.1 | 0.000113 |
| 11758194_s_at | DPP4 | dipeptidyl-peptidase 4 | AI768728 | 0.000125 |
| 11759481_at | COPB1 | Coatomer protein complex, subunit beta 1 | AU143964 | 0.000148 |
| 11718627_at | TRAK1 | trafficking protein, kinesin binding 1 | CA415544 | 0.000173 |
| 11739544_a_at | C19orf12 | chromosome 19 open reading frame 12 | BX328123 | 0.000234 |
| 11743191_a_at | NTM | neurotrimin | AI343272 | 0.000278 |
| 11731649_x_at | NTM | neurotrimin | AY358331.1 | 0.000343 |
| 11723111_a_at | EMILIN2 | elastin microfibril interfacer 2 | NM_032048.2 | 0.000384 |
| 11719422_s_at | ABCC1 | ATP-binding cassette, subfamily C (CFTR/MRP), member 1 | NM_004996.3 | 0.000385 |
| 11739527_a_at | SECTM1 | secreted and transmembrane 1 | CR614987.1 | 0.000402 |
| 11758810_at | COL14A1 | collagen, type XIV, alpha 1 | NM_021110.1 | 0.000451 |
| 11724441_x_at | PTGIS | prostaglandin I2 (prostacyclin) synthase | NM_000961.3 | 0.000458 |
| 11747944_a_at | PPFIA2 | protein tyrosine phosphatase, receptor type, f polypeptide (PTPRF), interacting protein (liprin), alpha 2 | AK296380.1 | 0.000483 |
| 11728451_a_at | PCOLCE2 | procollagen C-endopeptidase enhancer 2 | NM_013363.2 | 0.000493 |
| 11736086_a_at | HHIP | hedgehog interacting protein | NM_022475.1 | 0.000511 |
| 11758252_s_at | HSD3B7 | hydroxy-delta-5-steroid dehydrogenase, 3 beta- and steroid delta-isomerase 7 | CB115219 | 0.000562 |
| 11756706_a_at | DPP4 | dipeptidyl-peptidase 4 | AK314798.1 | 0.000563 |
| 11720690_a_at | C2orf18 | chromosome 2 open reading frame 18 | NM_017877.3 | 0.000604 |
| 11724619_at | RSPO3 | R-spondin 3 homolog (Xenopus laevis) | NM_032784.3 | 0.000676 |
| 11743447_a_at | BICD2 | bicaudal D homolog 2 (Drosophila) | AW409827 | 0.000721 |
| 11725773_a_at | TBC1D24 | TBC1 domain family, member 24 | NM_020705.1 | 0.000726 |
| 11715704_x_at | ITGA5 | integrin, alpha 5 (fibronectin receptor, alpha polypeptide) | NM_002205.2 | 0.000766 |
| 11756007_a_at | HHIP | hedgehog interacting protein | AK024645.1 | 0.000889 |
| 11741377_a_at | MMP2 | matrix metallopeptidase 2 (gelatinase A, 72kDa gelatinase, 72kDa type IV collagenase) | NM_001127891.1 | 0.000928 |
| 11734550_x_at | TGFBI | transforming growth factor, beta-induced, 68kDa | NM_000358.2 | 0.000963 |
| 11730405_at | MEX3B | mex-3 homolog B (C. elegans) | BC111545.1 | 0.000968 |
| 11731648_a_at | NTM | neurotrimin | AY358331.1 | 0.000985 |
| 11740103_a_at | MAFG | v-maf musculoaponeurotic fibrosarcoma oncogene homolog G (avian) | BX427058 | 0.001077 |
| 11727783_s_at | TPM4 | tropomyosin 4 | NM_003290.2 | 0.001163 |
| 11718269_x_at | ANGPTL2 | angiopoietin-like 2 | AY358274.1 | 0.001231 |
| 11725897_at | TUBB1 | tubulin, beta 1 | BC033679.1 | 0.001234 |
| 11717803_a_at | NTN4 | netrin 4 | NM_021229.3 | 0.001243 |
| 11754476_x_at | DNM1 | dynamin 1 | BQ183716 | 0.00125 |
| 11746893_a_at | MPP1 | membrane protein, palmitoylated 1, 55kDa | AK304538.1 | 0.001254 |
| 11753088_a_at | MCTP1 | multiple C2 domains, transmembrane 1 | AK297325.1 | 0.001305 |
| 11717568_s_at | NQO1 | NAD(P)H dehydrogenase, quinone 1 | NM_000903.2 | 0.001408 |
| 11720051_at | SPOCK1 | sparc/osteonectin, cwcv and kazal-like domains proteoglycan (testican) 1 | NM_004598.3 | 0.001423 |
| 11757921_s_at | COL14A1 | collagen, type XIV, alpha 1 | AI248460 | 0.001424 |
| 11725923_s_at | ASAP2 | ArfGAP with SH3 domain, ankyrin repeat and PH domain 2 | NM_001135191.1 | 0.001457 |
| 11723225_a_at | CLDN11 | claudin 11 | BC013577.1 | 0.001495 |
| 11754368_a_at | FBN1 | fibrillin 1 | AB208840.1 | 0.001626 |
| 11758062_s_at | STK32B | serine/threonine kinase 32B | AI401203 | 0.001735 |
| 11740358_a_at | LILRB5 | leukocyte immunoglobulin-like receptor, subfamily B (with TM and ITIM domains), member 5 | NM_001081443.1 | 0.001775 |
| 11746200_s_at | EHD2 | EH-domain containing 2 | AK097126.1 | 0.001844 |
| 11747945_x_at | PPFIA2 | protein tyrosine phosphatase, receptor type, f polypeptide (PTPRF), interacting protein (liprin), alpha 2 | AK296380.1 | 0.002036 |
| 11720811_a_at | PAMR1 | peptidase domain containing associated with muscle regeneration 1 | NM_015430.2 | 0.002139 |
| 11731716_at | CCBP2 | chemokine binding protein 2 | NM_001296.4 | 0.002156 |
| 11754706_a_at | HHIP | hedgehog interacting protein | AK098525.1 | 0.002166 |
| 11722940_a_at | SRGAP2 | SLIT-ROBO Rho GTPase activating protein 2 | NM_001042758.1 | 0.00222 |
| 11724848_a_at | DIXDC1 | DIX domain containing 1 | DB358954 | 0.002285 |
| 11718842_a_at | C16orf62 | chromosome 16 open reading frame 62 | BC058845.1 | 0.002303 |
| 11734549_s_at | TGFBI | transforming growth factor, beta-induced, 68kDa | NM_000358.2 | 0.002382 |
| 11734548_a_at | TGFBI | transforming growth factor, beta-induced, 68kDa | NM_000358.2 | 0.002425 |
| 11731650_a_at | NTM | neurotrimin | NM_001048209.1 | 0.002477 |
| 11738845_x_at | NTM | neurotrimin | NM_001144059.1 | 0.002642 |
| 11726905_a_at | ARHGAP20 | Rho GTPase activating protein 20 | NM_020809.2 | 0.002644 |
| 11724735_a_at | PDPN | podoplanin | BC014668.1 | 0.002735 |
| 11755796_a_at | ADAM9 | ADAM metallopeptidase domain 9 | BC143924.1 | 0.002822 |
| 11726017_a_at | C17orf63 | chromosome 17 open reading frame 63 | AU253346 | 0.002907 |
| 11741286_a_at | CCRL1 | chemokine (C-C motif) receptor-like 1 | AF110640.1 | 0.002908 |
| 11743910_at | FAM69A | family with sequence similarity 69, member A | BQ015316 | 0.002927 |
| 11756911_a_at | C1QTNF3 | C1q and tumor necrosis factor related protein 3 | BX640995.1 | 0.002969 |
| 11746361_a_at | C7orf58 | chromosome 7 open reading frame 58 | BC030538.2 | 0.002971 |
| 11715703_s_at | ITGA5 | integrin, alpha 5 (fibronectin receptor, alpha polypeptide) | NM_002205.2 | 0.002995 |
| 11735913_s_at | TNXB | tenascin XB | BC125114.1 | 0.003064 |
| 11730236_s_at | MYADM | myeloid-associated differentiation marker | AY358582.1 | 0.003084 |
| 11718267_a_at | ANGPTL2 | angiopoietin-like 2 | NM_012098.2 | 0.003114 |
| 11723217_x_at | SFXN3 | sideroflexin 3 | NM_030971.3 | 0.003152 |
| 11720286_a_at | TRAK1 | trafficking protein, kinesin binding 1 | BC015922.1 | 0.003236 |
| 11717133_a_at | MAFG | v-maf musculoaponeurotic fibrosarcoma oncogene homolog G (avian) | BF340448 | 0.003272 |
| 11717340_at | PTGFRN | prostaglandin F2 receptor negative regulator | NM_020440.2 | 0.003311 |
| 11729541_a_at | CAMKK2 | calcium/calmodulin-dependent protein kinase kinase 2, beta | AB081337.1 | 0.003359 |
| 11718268_a_at | ANGPTL2 | angiopoietin-like 2 | AY358274.1 | 0.003373 |
| 11717413_a_at | WIPI1 | WD repeat domain, phosphoinositide interacting 1 | NM_017983.5 | 0.003406 |
| 11716226_a_at | LIMA1 | LIM domain and actin binding 1 | BC136763.1 | 0.003456 |
| 11740588_at | BDKRB2 | bradykinin receptor B2 | NM_000623.3 | 0.003464 |
| 11741128_a_at | CAPN2 | calpain 2, (m/II) large subunit | NM_001146068.1 | 0.003473 |
| 11717891_a_at | ECM1 | extracellular matrix protein 1 | BC023505.2 | 0.003509 |
| 11730385_at | GEM2 | gremlin 2 | BG150451 | 0.003541 |
| 11756245_s_at | ANXA5 | annexin A5 | CR607543.1 | 0.003586 |
| 11721499_x_at | CTSA | cathepsin A | NM_001127695.1 | 0.003681 |
| 11717757_s_at | RALA | v-ral simian leukemia viral oncogene homolog A (ras related) | AA548928 | 0.003773 |
| 11723075_a_at | BCL9L | B-cell CLL/lymphoma 9-like | AY296059.1 | 0.003835 |
| 11748650_a_at | ADAM33 | ADAM metallopeptidase domain 33 | BC125113.1 | 0.003846 |
| 11758676_s_at | RHOQ | ras homolog gene family, member Q | R23125 | 0.003853 |
| 11724260_a_at | TRIO | triple functional domain (PTPRF interacting) | AF091395.1 | 0.004049 |
| 11724541_s_at | VWF | von Willebrand factor | NM_000552.3 | 0.004078 |
| 11716549_s_at | ISLR | immunoglobulin superfamily containing leucine-rich | NM_005545.3 | 0.0042 |
| 11724228_at | RBMS1 | RNA binding motif, single stranded interacting protein 1 | BC080620.1 | 0.004204 |
| 11752423_a_at | F13A1 | coagulation factor XIII, A1 polypeptide | AK304335.1 | 0.004293 |
| 11757340_s_at | RHOQ | ras homolog gene family, member Q | BM677515 | 0.004317 |
| 11750650_a_at | PAMR1 | peptidase domain containing associated with muscle regeneration 1 | AK297092.1 | 0.004367 |
| 11735263_s_at | SCN2A | sodium channel, voltage-gated, type II, alpha subunit | NM_001040142.1 | 0.004453 |
| 11731682_at | CD70 | CD70 molecule | NM_001252.3 | 0.004492 |
| 11737108_a_at | CCRL1 | chemokine (C-C motif) receptor-like 1 | NM_178445.1 | 0.004501 |
| 11743251_s_at | MMP2 | matrix metallopeptidase 2 (gelatinase A, 72kDa gelatinase, 72kDa type IV collagenase) | BX357054 | 0.004507 |
| 11727782_a_at | TPM4 | tropomyosin 4 | NM_003290.2 | 0.004515 |
| | FAP | fibroblast activation protein, | AL832166.1 | 0.004528 |
| 11725868_at | SSC5D | scavenger receptor cysteine-rich glycoprotein | NM_001144950.1 | 0.004768 |

**Table 4 - Type I Dermis; Down-regulated**

| GeneTitan_ID | Gene | Title | Public ID | p |
|---|---|---|---|---|
| 11718273_a_at | EIF3L | eukaryotic translation initiation factor 3, subunit L | NM_016091.2 | 0.000001 |
| 11729152_a_at | EIF3M | eukaryotic translation initiation factor 3, subunit M | NM_006360.3 | 0.000006 |
| 11755203_x_at | RPL21 | ribosomal protein L21 | BX647669.1 | 0.000006 |
| 11757356_x_at | RPL30 | ribosomal protein L30 | BM855760 | 0.000008 |
| 11717236_x_at | RPS7 | ribosomal protein S7 | NM_001011.3 | 0.000009 |
| 11745362_x_at | RPS11 | ribosomal protein S11 | BC100025.1 | 0.000009 |
| 11757363_x_at | RPS15A | ribosomal protein S15a | D313157 | 0.000009 |
| 200062_PM_s_at | RPL30 | ribosomal protein L30 | L05095.1 | 0.000009 |
| 11716092_x_at | CKS1B | CDC28 protein kinase regulatory subunit 1B | NM_001826.2 | 0.00001 |
| 11743094_at | SPRR4 | small proline-rich protein 4 | BC069445.1 | 0.000011 |
| 11757421_x_at | RPL31 | ribosomal protein L31 | CD687230 | 0.000011 |
| 11718274_s_at | EIF3L | eukaryotic translation initiation factor 3, subunit L | NM_016091.2 | 0.000012 |
| 11715376_a_at | RPS11 | ribosomal protein S11 | NM_001015.3 | 0.000015 |
| 11757773_x_at | NCRNA00275 | non-protein coding RNA 275 | BF185165 | 0.000015 |
| 11753659_x_at | RPL30 | ribosomal protein L30 | BC095426.1 | 0.000016 |
| 11753694_x_at | RPS15A | ribosomal protein S15a | AB062400.1 | 0.000017 |
| 11755956_x_at | POLE3 | polymerase (DNA directed), epsilon 3 (p17 subunit) | AF070640.1 | 0.000017 |
| 200063_PM_s_at | NPM1 | nucleophosmin (nucleolar phosphoprotein B23, numatrin) | BC002398.1 | 0.000017 |
| 11740643_a_at | CYP4F8 | cytochrome P450, family 4, subfamily F, polypeptide 8 | AF133298.1 | 0.00002 |
| 11757305_x_at | RPSAP58 | ribosomal protein SA pseudogene 58 | BI762726 | 0.000021 |
| 11715958_s_at | RPL7 | ribosomal protein L7 | NM_000971.3 | 0.000023 |
| 11719783_at | RPS23 | ribosomal protein S23 | D14530.1 | 0.000027 |
| 11745154_a_at | NCL | nucleolin | BC006516.2 | 0.000027 |
| 11720183_s_at | EEF1B2 | eukaryotic translation elongation factor 1 beta 2 | NM_001959.3 | 0.000029 |
| 11753691_x_at | RPL24 | ribosomal protein L24 | CR456729.1 | 0.000031 |
| 200013_PM_at | RPL24 | ribosomal protein L24 | NM_000986.1 | 0.000034 |
| 11718275_x_at | EIF3L | eukaryotic translation initiation factor 3, subunit L | NM_016091.2 | 0.000037 |
| 11744326_s_at | RPL37 | ribosomal protein L37 | BC079477.1 | 0.000037 |
| 11757264_s_at | RPS3 | ribosomal protein S3 | BU588459 | 0.000038 |
| 200010_PM_at | RPL11 | ribosomal protein L11 | NM_000975.1 | 0.000039 |
| 11757027_x_at | RPL31 | ribosomal protein L31 | CR600452.1 | 0.000042 |
| 200018_PM_at | RPS13 | ribosomal protein S13 | NM_001017.1 | 0.000046 |
| 11757375_x_at | RPS15 | ribosomal protein S15 | AI625563 | 0.000047 |
| 11754031_s_at | CKS1B | CDC28 protein kinase regulatory subunit 1B | BT007196.1 | 0.000051 |
| 11715733_a_at | NIPSNAP1 | nipsnap homolog 1 (C. elegans) | NM_003634.2 | 0.000054 |
| 11740644_x_at | CYP4F8 | cytochrome P450, family 4, subfamily F, polypeptide 8 | AF133298.1 | 0.000061 |
| 11736188_a_at | ORMDL3 | ORM1-like 3 (S. cerevisiae) | NM_139280.1 | 0.000071 |
| 11730527_a_at | DAPK2 | death-associated protein kinase 2 | AF052941.1 | 0.000088 |
| 11723312_a_at | PXMP2 | peroxisomal membrane protein 2, 22kDa | NM_018663.1 | 0.000092 |
| 11734833_s_at | TAF9B | TAF9B RNA polymerase II, TATA box binding protein (TBP)-associated factor, 31kDa | NM_015975.4 | 0.000093 |
| 11732205_x_at | NAP1L1 | nucleosome assembly protein 1-like 1 | BX413854 | 0.000099 |
| 11752912_x_at | EIF3M | eukaryotic translation initiation factor 3, subunit M | AK292139.1 | 0.000099 |
| 11756437_x_at | RPS618 | ribosomal protein S18 | BQ057441 | 0.0001 |
| 11729011_at | CDH22 | cadherin 22, type 2 | NM_021248.1 | 0.000107 |
| 11734329_at | TNN | tenascin N | NM_022093.1 | 0.000107 |
| 11749558_a_at | CYP4F8 | cytochrome P450, family 4, subfamily F, polypeptide 8 | AK300530.1 | 0.000119 |
| 200029_PM_at | RPL19 | ribosomal protein L19 | NM_000981.1 | 0.000121 |
| 11757386_x_at | NPM1 | nucleophosmin (nucleolar phosphoprotein B23, numatrin) | AL563600 | 0.000122 |
| 11733774_a_at | RPL37 | ribosomal protein L37 | NM_000997.4 | 0.000123 |
| 11729427_a_at | GLI1 | GLI family zinc finger 1 | NM_005269.2 | 0.000126 |
| 11754066_x_at | NPM1 | nucleophosmin (nucleolar phosphoprotein B23, numatrin) | BT007011.1 | 0.000128 |
| 11715626_a_at | RPL11 | ribosomal protein L11 | NM_000975.2 | 0.00013 |
| 11757489_x_at | RPL22 | ribosomal protein L22 | AW268528 | 0.00013 |
| 11757355_x_at | RPL41 | ribosomal protein L41 | BU958994 | 0.000138 |
| 11752726_x_at | GNB2L1 | guanine nucleotide binding protein (G protein), beta polypeptide 2-like 1 | AY159316.1 | 0.000144 |
| 11728288_a_at | KRT15 | keratin 15 | NM_002275.3 | 0.000153 |
| 11756783_a_at | TF | transferrin | BC045772.1 | 0.000155 |
| 11757331_x_at | RPL13A | ribosomal protein L13a | BF688481 | 0.000157 |
| 11744365_a_at | NCRNA00275 | non-protein coding RNA 275 | AY513722.1 | 0.000159 |
| 11739727_x_at | NAP1L1 | nucleosome assembly protein 1-like 1 | BE965760 | 0.00016 |
| 200074_PM_s_at | RPL14 | ribosomal protein L14 | U16738.1 | 0.000161 |
| 200089_PM_s_at | RPL4 | ribosomal protein L4 | AI953886 | 0.000162 |
| 11734331_a_at | TNN | tenascin N | BC136619.1 | 0.000163 |
| 11757906_x_at | RPL10 | ribosomal protein L10 | AL558950 | 0.000167 |
| 11715645_s_at | C22orf28 | chromosome 22 open reading frame 28 | NM_014306.4 | 0.00018 |
| 11756875_x_at | COMMD6 | COMM domain containing 6 | CR603325.1 | 0.000184 |
| 11722318_a_at | EFNB3 | ephrin-B3 | NM_001406.3 | 0.000198 |
| 11756878_a_at | FBL | fibrillarin | CR593763.1 | 0.000198 |
| 11736721_x_at | RPL32 | ribosomal protein L32 | NM_001007073.1 | 0.000199 |
| 11720184_x_at | EEF1B2 | eukaryotic translation elongation factor 1 beta 2 | NTM_001959.3 | 0.0002 |
| 11749786_x_at | HNRNPF | heterogeneous nuclear ribonucleoprotein F | AK296696.1 | 0.000201 |
| 11717058_x_at | RPL5 | ribosomal protein L5 | NM_000969.3 | 0.00021 |
| 11752911_a_at | EIF3M | eukaryotic translation initiation factor 3, subunit M | AK292139.1 | 0.000211 |
| 11730790_x_at | NPM1 | nucleophosmin (nucleolar phosphoprotein B23, numatrin) | AK290652.1 | 0.000213 |
| 11753680_x_at | RPL21 | ribosomal protein L21 | CR457032.1 | 0.000213 |
| 200022_PM_at | RPL18 | ribosomal protein L18 | NM_000979.1 | 0.000213 |
| 200014_PM_s_at | HNRNPC | heterogeneous nuclear ribonucleoprotein C (C1/C2) | NM_004500.1 | 0.000215 |
| 11742667_x_at | RPS28 | ribosomal protein S28 | NM_001031.4 | 0.000242 |
| 200082_PM_s_at | RPS7 | ribosomal protein S7 | AI805587 | 0.00025 |
| 11728380_x_at | NACA2 | nascent polypeptide-associated complex alpha subunit 2 | NM_199290.2 | 0.000252 |
| 11756140_s_at | RPL4 | ribosomal protein L4 | BX447218 | 0.000254 |
| 11716304_a_at | ABHD14B | abhydrolase domain containing 14B | NM_032750.2 | 0.000267 |
| 11758357_x_at | RPL9 | ribosomal protein L9 | BF172613 | 0.000272 |
| 11715280_s_at | KRT17 | keratin 17 | g197383031 | 0.000274 |
| 11739813_a_at | FZD1 | frizzled homolog 1 (Drosophila) | BF675672 | 0.000295 |
| 11721885_s_at | CDC42 | cell division cycle 42 (GTP binding protein, 25kDa) | NM_001039802.1 | 0.000307 |
| 11720954_s_at | RPL30 | ribosomal protein L30 | NM_000989.2 | 0.000309 |
| 11743688_at | GLI2 | GLI family zinc finger 2 | AB209354.1 | 0.000312 |
| 11720599_s_at | SUB1 | SUB1 homolog (S. cerevisiae) | NM_006713.3 | 0.000315 |
| 11725875_at | WDR66 | WD repeat domain 66 | NM_144668.4 | 0.000318 |
| 11733496_x_at | COMMD6 | COMM domain containing 6 | AA535445 | 0.000323 |
| 11727795_x_at | EIF3E | eukaryotic translation initiation factor 3, subunit | NM_001568.2 | 0.000327 |
| 11756215_x_at | UBA52 | ubiquitin A-52 residue ribosomal protein fusion product 1 | BU619323 | 0.000331 |
| 11757059_x_at | RPL36A | ribosomal protein L36a | CR617894.1 | 0.000331 |
| 200012_PM_x_at | RPL21 | ribosomal protein L21 | NM_000982.1 | 0.000335 |
| 11718344_a_at | CNOT7 | CCR4-NOT transcription complex, subunit 7 | NM_013354.5 | 0.00035 |
| 11717235_s_at | RPS7 | ribosomal protein S7 | NM_001011.3 | 0.000352 |
| 11757113_a_at | SNHG1 | small nucleolar RNA host gene 1 (non-protein coding) | BE836747 | 0.000365 |
| 11743679_a_at | PTCH1 | patched 1 | DB299015 | 0.000374 |
| 11744366_a_at | NCRNA00275 | non-protein coding RNA 275 | CR936805.1 | 0.000378 |

**Table 5 - Type II Epidermis; Up-regulated**

| GeneTitan_ID | Gene | Title | Public ID | p |
|---|---|---|---|---|
| 11729461_a_at | CTNS | cystinosis, nephropathic | NM_001031681.2 | 0.000105 |
| 11737824_a_at | STX16 | syntaxin 16 | NM_001134773.1 | 0.000753 |
| 11731828_at | GPC2 | glypican 2 | NM_152742.1 | 0.000819 |
| 11757259_x_at | SCARNA9L | small Cajal body-specific RNA 9-like (retrotransposed) | NR_023358.1 | 0.000852 |
| 11728498_a_at | SVIL | supervillin | NM_003174.3 | 0.001387 |
| 11733298_a_at | VIPR1 | vasoactive intestinal peptide receptor 1 | NM_004624.3 | 0.00166 |
| 11754972_s_at | BAZ2A | bromodomain adjacent to zinc finger domain, 2A | AK127775.1 | 0.001742 |
| 11732899_s_at | SULT1A1 | sulfotransferase family, cytosolic, 1A, phenol-preferring, member 1 | NM_177528.1 | 0.001887 |
| 11716708_a_at | DDR1 | discoidin domain receptor tyrosine kinase 1 | NM_013993.2 | 0.001925 |
| 11757623_s_at | RNF5 | ring finger protein 5 | AA923467 | 0.00209 |
| 11715799_s_at | BAT2L1 | HLA-B associated transcript 2-like 1 | NM_013318.3 | 0.002325 |
| 11756190_a_at | CLK3 | CDC-like kinase 3 | CD743118 | 0.002384 |
| 11726634_a_at | MYST3 | MYST histone acetyltransferase (monocytic leukemia) 3 | NM_001099412.1 | 0.002824 |
| 11752331_s_at | SULT1A4 | sulfotransferase family, cytosolic, 1A, phenol-preferring, member 4 | BC111011.1 | 0.002837 |
| 11731093_s_at | BRD1 | bromodomain containing 1 | NM_014577.1 | 0.002905 |
| 11744831_a_at | RPAIN | RPA interacting protein | AY775316.1 | 0.002976 |
| 11744173_x_at | DNAJC4 | DnaJ (Hsp40) homolog, subfamily C, member 4 | BQ267791 | 0.00329 |
| 11723546_s_at | PLD1 | phospholipase D1, phosphatidylcholine-specific | BF434088 | 0.003418 |
| 11732589_a_at | ZNF467 | zinc finger protein 467 | NM_207336.1 | 0.004153 |
| 11721165_a_at | KHNYN | KH and NYN domain containing | NM_015299.2 | 0.00435 |
| 11730324_s_at | SLC38A9 | solute carrier family 38, member 9 | NM_173514.2 | 0.004534 |
| 11739669_at | SS18L1 | synovial sarcoma translocation gene on chromosome 18-like 1 | AB014593.1 | 0.004553 |
| 11758140_s_at | CPSF6 | cleavage and polyadenylation specific factor 6, 68kDa | BU689332 | 0.004569 |
| 11721912_at | MDM | Mdm4 p53 binding protein homolog (mouse) | NM_002393.3 | 0.004611 |
| 11744830_x_at | NPIPL3 | nuclear pore complex interacting protein-like 3 | AK303166.1 | 0.004653 |
| 11729100_a_at | TTC18 | tetratricopeptide repeat domain 18 | NM_145170.3 | 0.004727 |
| 11757896_s_at | C1orf63 | chromosome 1 open reading frame 63 | R81538 | 0.004766 |
| 11715976_a_at | VGLL4 | vestigial like 4 (Drosophila) | NM_001128219.1 | 0.004868 |
| 11729196_a_at | STX16 | syntaxin 16 | BE782754 | 0.004954 |
| 11758055_x_at | RGPD8 | RANBP2-like and GRIP domain containing 8 | BQ005433 | 0.005005 |
| 11721624_s_at | WSB1 | WD repeat and SOCS box-containing 1 | NM_015626.8 | 0.005017 |
| 11720589_s_at | PHF21A | PHD finger protein 21A | BU733437 | 0.005058 |
| 11720895_at | SOS1 | son of sevenless homolog 1 (Drosophila) | BM970418 | 0.005891 |
| 11761133_at | KDM5C | lysine (K)-specific demethylase 5C | EF613277.1 | 0.006011 |
| 11726189_x_at | HCFC1R1 | host cell factor C1 regulator 1 (XPO1 dependent) | NM_017885.2 | 0.006166 |
| 11721598_a_at | EFS | embryonal Fyn-associated substrate | NM_032459.1 | 0.006547 |
| 11745431_a_at | SVIL | supervillin | BC092440.1 | 0.006666 |
| 11740447_x_at | BTN3A1 | butyrophilin, subfamily 3, member A1 | NM_194441.2 | 0.006686 |
| 11726515_a_at | CLK4 | CDC-like kinase 4 | AF294429.1 | 0.006691 |
| 11746529_x_at | TNFRSF14 | tumor necrosis factor receptor superfamily, member 14 (herpesvirus entry mediator) | BC029848.1 | 0.006897 |
| 11759308_s_at | MAGI1 | membrane associated guanylate kinase, WW and PDZ domain containing 1 | AL050184.1 | 0.006929 |
| 11749473_a_at | MEF2D | myocyte enhancer factor 2D | BC040949.1 | 0.007372 |
| 11719128_a_at | LMF2 | lipase maturation factor 2 | NM_033200.1 | 0.007569 |
| 11762365_x_at | KIAA0415 | KIAA0415 | AB007875.1 | 0.007605 |
| 11716129_at | IGF2R | insulin-like growth factor 2 receptor | NM_000876.2 | 0.007692 |
| 11717989_a_at | SUN1 | Sad1 and UNC84 domain containing 1 | NM_001130965.1 | 0.007807 |
| 11755196_a_at | CORO6 | coronin 6 | AK092430.1 | 0.007848 |
| 11755758_s_at | NLRC5 | NLR family, CARD domain containing 5 | AK090439.1 | 0.00788 |
| 11716283_at | PAPD7 | PAP associated domain containing 7 | NM_006999.3 | 0.008191 |
| 11730449_a_at | DHRS12 | dehydrogenase/reductase (SDR family) member 12 | NM_024705.1 | 0.008218 |
| 11718728_a_at | | zinc finger protein 655 | NM_001083956.1 | 0.008286 |
| 11718820_at | TSC1 | tuberous sclerosis 1 | NM_000368.3 | 0.008558 |
| 11729483_a_at | KLF8 | Kruppel-like factor 8 | NM_007250.4 | 0.008607 |
| 11754192_s_at | SRSF11 | serine/arginine-rich splicing factor 11 | CR614713.1 | 0.009136 |
| 11758557_s_at | ZFP36L1 | zinc finger protein 36, C3H type-like 1 | AI758505 | 0.009209 |
| 11723598_x_at | MAP2K7 | mitogen-activated protein kinase kinase 7 | NM_145185.2 | 0.009355 |
| 11754541_a_at | CCDC45 | coiled-coil domain containing 45 | AW167096 | 0.00982 |
| 11757630_s_at | HERPUD2 | HERPUD family member 2 | AA709265 | 0.010052 |
| 11718536_s_at | NKTR | natural killer-tumor recognition sequence | NM_005385.3 | 0.010291 |
| 11755674_s_at | RALGAPA1 | Ral GTPase activating protein, alpha subunit 1 (catalytic) | DQ786317.1 | 0.010335 |
| 11757591_s_at | PAN3 | PAN3 poly(A) specific ribonuclease subunit homolog (S. cerevisiae) | DB314869 | 0.010593 |
| 11724312_a_at | SH3BP2 | SH3-domain binding protein 2 | NM_001145855.1 | 0.010664 |
| 11757197_s_at | NCRNA00201 | non-protein coding RNA 201 | NR_026778.1 | 0.010882 |
| 11759150_at | CNOT4 | CCR4-NOT transcription complex, subunit 4 | BC035590.1 | 0.011523 |
| 11718939_s_at | TNFAIP3 | tumor necrosis factor, alpha-induced protein 3 | NM_006290.2 | 0.011563 |
| 11738035_s_at | RTN4 | reticulon 4 | AK302741.1 | 0.011615 |
| 11719084_a_at | SMARCC2 | SWI/SNF related, matrix associated, actin dependent regulator of chromatin, subfamily c, member 2 | BF663402 | 0.011872 |
| 11736501_x_at | SS18 | synovial sarcoma translocation, chromosome 18 | NM_005637.2 | 0.011911 |
| 11755811_a_at | ZNF266 | zinc finger protein 266 | AL833503.1 | 0.012172 |
| 11720362_at | PHIP | pleckstrin homology domain interacting protein | CR600369.1 | 0.012352 |
| 11724758_s_at | GPBP1L1 | GC-rich promoter binding protein 1-like 1 | NM_021639.4 | 0.012429 |
| 11754821_s_at | SLC38A1 | solute carrier family 38, member 1 | AI476037 | 0.012431 |
| 11758907_at | ZNF827 | zinc finger protein 827 | AA031947 | 0.012473 |
| 11736498_a_at | TNRC6B | trinucleotide repeat containing 6B | NM_015088.2 | 0.012866 |
| 11716010_s_at | DYNC1LI2 | dynein, cytoplasmic 1, light intermediate chain 2 | NM_006141.2 | 0.01317 |
| 11745723_a_at | MALAT1 | metastasis associated lung adenocarcinoma transcript 1 (non-protein coding) | BX538238.1 | 0.013758 |
| 11758584_s_at | STYX | serine/threonine/tyrosine interacting protein | N34305 | 0.013878 |
| 11757558_s_at | LONRF1 | LON peptidase N-terminal domain and ring finger 1 | BF680438 | 0.014197 |
| 11750922_x_at | AMT | aminomethyltransferase | AK296177.1 | 0.0143 |
| 11718558_s_at | MKRN1 | makorin ring finger protein 1 | NM_001145125.1 | 0.014465 |
| 11723112_a_at | CCDC84 | coiled-coil domain containing 84 | NM_198489.1 | 0.014471 |
| 11757808_s_at | RERE | arginine-glutamic acid dipeptide (RE) repeats | BM706668 | 0.014565 |
| 11763191_at | PRICKLE3 | prickle homolog 3 (Drosophila) | AK303308.1 | 0.014697 |
| 11757821_s_at | LDB1 | LIM domain binding 1 | AW271288 | 0.014809 |
| 11755194_s_at | CCNL2 | cyclin L2 | AK000685.1 | 0.014941 |
| 11720122_at | GIGYF1 | GRB10 interacting GYF protein 1 | NM_022574.4 | 0.014956 |
| 11763351_at | LOC286052 | hypothetical protein LOC286052 | CK819455 | 0.014973 |
| 11722752_a_at | C14orf43 | chromosome 14 open reading frame 43 | NM_194278.3 | 0.015053 |
| 11757958_s_at | POGZ | pogo transposable element with ZNF domain | AI374931 | 0.015063 |
| 11734056_at | PTGR2 | prostaglandin reductase 2 | NM_152444.2 | 0.015377 |
| 11722715_at | STK35 | serine/threonine kinase 35 | NM_080836.2 | 0.015673 |
| 11722134_a_at | TNFRSF25 | tumor necrosis factor receptor superfamily, member 25 | NM_148965.1 | 0.015688 |
| 11715192_s_at | C7orf46 | chromosome 7 open reading frame 46 | g188219621 | 0.015694 |
| 11720795_s_at | NUPL1 | nucleoporin like 1 | NM_014089.3 | 0.016154 |
| 11729510_a_at | WDR33 | WD repeat domain 33 | NM_001006623.1 | 0.016469 |
| 11724066_s_at | HCFC1R1 | host cell factor C1 regulator 1 (XPO1 dependent) | NM_001002018.1 | 0.016491 |
| 11718534_at | NKTR | natural killer-tumor recognition sequence | AI361805 | 0.016611 |
| 11741625_a_at | SLC22A23 | solute carrier family 22, member 23 | NM_021945.5 | 0.016631 |
| 11722305_at | ARHGAP23 | Rho GTPase activating protein 23 | NM_020876.1 | 0.01665 |
| 11764248_s_at | LDLRAD3 | low density lipoprotein receptor class A domain containing 3 | AW043782 | 0.016656 |

**Table 6 - Type II Epidermis; Down-regulated**

| GeneTitan_ID | Gene | Title | PublicID | p |
|---|---|---|---|---|
| 11719408_a_at | HIPK2 | homeodomain interacting protein kinase 2 | BM679184 | 0.000155 |
| 11739028_s_at | CLTC | clathrin, heavy chain (Hc) | BX395378 | 0.00019 |
| 11747337_x_at | EIF31 | eukaryotic translation initiation factor 3, subunit I | U36764.1 | 0.000378 |
| 11749845_a_at | TBC1D22A | TBC1 domain family, member 22A | AK301445.1 | 0.000577 |
| 11723960_at | SCFD2 | sec1 family domain containing 2 | BC032453.1 | 0.000699 |
| 11717105_a_at | PSMB5 | proteasome (prosome, macropain) subunit, beta type, 5 | NM_001144932.1 | 0.00071 |
| 11716545_x_at | PSMC1 | proteasome (prosome, macropain) 26S subunit, ATPase, 1 | NM_002802.2 | 0.00077 |
| 11730754_s_at | AGPAT5 | 1-acylglycerol-3-phosphate O-acyltransferase 5 (lysophosphatidic acid acyltransferase, epsilon) | CB306609 | 0.00077 |
| 11750994_a_at | SYAP1 | synapse associated protein 1 | AK295322.1 | 0.000788 |
| 11754977_x_at | CTSB | cathepsin B | CR614817.1 | 0.000808 |
| 200096_PM_s_at | ATP6V0E1 | ATPase, H+ transporting, lysosomal 9kDa, V0 subunit e1 | AI862255 | 0.000889 |
| 11738899_a_at | SERPINB12 | serpin peptidase inhibitor, clade B (ovalbumin), member 12 | NM_080474.1 | 0.000913 |
| 11747533_a_at | GRSF1 | G-rich RNA sequence binding factor 1 | AK298883.1 | 0.000973 |
| 11733918_a_at | PSMD14 | proteasome (prosome, macropain) 26S subunit, non-ATPase, 14 | BC066336.1 | 0.001091 |
| 11753572_a_at | TMEM85 | transmembrane protein 85 | AY336092.1 | 0.001121 |
| 11738988_a_at | GANAB | glucosidase, alpha; neutral AB | AK302752.1 | 0.001195 |
| 11751303_s_at | GORASP2 | golgi reassembly stacking protein 2, 55kDa | AK293640.1 | 0.001298 |
| 11719482_a_at | MRPL21 | mitochondrial ribosomal protein L21 | NM_181515.1 | 0.001366 |
| 11715943_x_at | PSMB1 | proteasome (prosome, macropain) subunit, beta type, 1 | NM_002793.3 | 0.001475 |
| 11749935_s_at | TPM3 | tropomyosin 3 | AK298678.1 | 0.001523 |
| 11736759_s_at | SDHC | succinate dehydrogenase complex, subunit C, integral membrane protein, 15kDa | AB211234.1 | 0.001635 |
| 11731397_a_at | YWHAB | tyrosine 3-monooxygenase/tryptophan 5-monooxygenase activation protein, beta polypeptide | AI866370 | 0.001646 |
| 11751557_s_at | MED27 | mediator complex subunit 27 | AK298436.1 | 0.001692 |
| 11747146_s_at | TMBIM6 | transmembrane BAX inhibitor motif containing 6 | AK304577.1 | 0.001727 |
| 11716972_s_at | NSF | N-ethylmaleimide-sensitive factor | NM_006178.2 | 0.001747 |
| 11754009_a_at | PSMB5 | proteasome (prosome, macropain) subunit, beta type, 5 | BT006777.1 | 0.001747 |
| 11717459_a_at | MRPL39 | mitochondrial ribosomal protein L39 | NM_017446.3 | 0.001824 |
| 11742273_a_at | MRPL33 | mitochondrial ribosomal protein L33 | AF420602.1 | 0.001825 |
| 11739599_a_at | ZNF398 | zinc finger protein 398 | BU736496 | 0.001891 |
| 11747534_a_at | RSU1 | Ras suppressor protein 1 | BC008691.1 | 0.002033 |
| 11747507_x_at | NAP1L4 | nucleosome assembly protein 1-like 4 | AK316548.1 | 0.002069 |
| 11747506_a_at | NAP1L4 | nucleosome assembly protein 1-like 4 | AK316548.1 | 0.002092 |
| 11748665_a_at | PICALM | phosphatidylinositol binding clathrin assembly protein | AK300275.1 | 0.002111 |
| 11718035_at | PPIL1 | peptidylprolyl isomerase (cyclophilin)-like 1 | AF151882.1 | 0.002124 |
| 11751336_x_at | MKRN1 | makorin ring finger protein 1 | AK297361.1 | 0.002138 |
| 11720264_at | ASCC3 | activating signal cointegrator 1 complex subunit 3 | NM_006828.2 | 0.002301 |
| 11725037_a_at | SEC23IP | SEC23 interacting protein | AK000698.1 | 0.002534 |
| 11715772_x_at | MRPL13 | mitochondrial ribosomal protein L13 | NM_014078.4 | 0.002536 |
| 11716173_a_at | P4HB | prolyl 4-hydroxylase, beta polypeptide | AK296206.1 | 0.002544 |
| 11717106_x_at | PSMB5 | proteasome (prosome, macropain) subunit, beta type, 5 | NM_001144932.1 | 0.002579 |
| 200059_PM_s_at | RHOA | ras homolog gene family, member A | BC001360.1 | 0.002604 |
| 11746655_a_at | ACAA1 | acetyl-CoA acyltransferase 1 | AK303251.1 | 0.002689 |
| 11739201_a_at | ATP5G3 | ATP synthase, H+ transporting, mitochondrial Fo complex, subunit C3 (subunit 9) | BE736890 | 0.002714 |
| 11728637_a_at | ATP5A1 | ATP synthase, H+ transporting, mitochondrial F1 complex, alpha subunit 1, cardiac muscle | NM_001001937.1 | 0.002718 |
| 11756013_a_at | BCL2L10 | BCL2-like 10 (apoptosis facilitator) | BC143227.1 | 0.002726 |
| 11754271_a_at | PSMB4 | proteasome (prosome, macropain) subunit, beta type, 4 | B849884 | 0.002734 |
| 11731415_a_at | PSMD6 | proteasome (prosome, macropain) 26S subunit, non-ATPase, 6 | NM_014814.1 | 0.002807 |
| 11715840_s_at | SDHC | succinate dehydrogenase complex, subunit C, integral membrane protein, 15kDa | BC020808.1 | 0.002866 |
| 11755266_x_at | SUCLA2 | succinate-CoA ligase, ADP-forming, beta subunit | AK001458.1 | 0.003146 |
| 11750876_a_at | SCFD1 | sec1 family domain containing 1 | AK301406.1 | 0.003266 |
| 11752770_a_at | SCARCE1 | SWI/SNF related, matrix associated, actin dependent regulator of chromatin, subfamily e, member 1 | AK294666.1 | 0.003311 |
| 11758800_x_at | SERBP1 | SERPINE1 mRNA binding protein 1 | AF151813.1 | 0.003464 |
| 11751835_a_at | LTV1 | LTV1 homolog (S. cerevisiae) | AY326463.1 | 0.003472 |
| 11758319_x_at | UBC | ubiquitin C | B672950 | 0.003583 |
| 11729168_x_at | DCTD | dCMP deaminase | BC001286.1 | 0.003633 |
| 11717159_a_at | NDUFB33 | NADH dehydrogenase (ubiquinone) 1 beta subcomplex, 3, 12kDa | NM_002491.2 | 0.003697 |
| 11744181_a_at | FARS2 | phenylalanyl-tRNA synthetase 2, mitochondrial | BG192794 | 0.003735 |
| 11751412_x_at | ARL1 | ADP-ribosylation factor-like 1 | AK301701.1 | 0.003805 |
| 11734682_a_at | PSMA7 | proteasome (prosome, macropain) subunit, alpha type, 7 | NM_002792.2 | 0.003878 |
| 11750059_a_at | MLX | MAX-like protein X | AK296114.1 | 0.003884 |
| 11715718_a_at | ZNHIT1 | zinc finger, HIT-type containing 1 | NM_006349.2 | 0.003894 |
| 11751360_x_at | REXO2 | RNA exonuclease 2 homolog (S. cerevisiae) | BC107887.1 | 0.003907 |
| 11747349_s_at | PSAT1 | phosphoserine aminotransferase 1 | BT006840.1 | 0.003982 |
| 11763975_a_at | MRPS11 | mitochondrial ribosomal protein S11 | DB346141 | 0.004073 |
| 11716381_x_at | BRP44 | brain protein 44 | NM_015415.2 | 0.00408 |
| 11751523_a_at | TMED5 | transmembrane emp24 protein transport domain containing 5 | AK293308.1 | 0.004086 |
| 11753974_s_at | SNRPG | small nuclear ribonucleoprotein polypeptide G | CR456918.1 | 0.004159 |
| 11732216_s_at | PEF1 | penta-EF-hand domain containing 1 | CR542139.1 | 0.004221 |
| 11718978_x_at | CAPN2 | calpain 2, (m/II) large subunit | BC021303.2 | 0.004238 |
| 11763422_a_at | ATP6V0D1 | ATPase, H+ transporting, lysosomal 38kDa, V0 subunit d1 | BX397389 | 0.004241 |
| 11754060_a_at | DAD1 | defender against cell death 1 | CR542204.1 | 0.004283 |
| 11743905_a_at | SPCS1 | signal peptidase complex subunit 1 homolog (S. cerevisiae) | BE782150 | 0.004361 |
| 11715732_at | PSMB3 | proteasome (prosome, macropain) subunit, beta type, 3 | NM_002795.2 | 0.004369 |
| 11751505_a_at | YIPF1 | Yip1 domain family, member 1 | AK300240.1 | 0.004409 |
| 11742925_a_at | C11orf59 | chromosome 11 open reading frame 59 | CR457247.1 | 0.004503 |
| 11715417_s_at | SKP1 | S-phase kinase-associated protein 1 | BC020798.1 | 0.004548 |
| 11750438_x_at | PGAM1 | phosphoglycerate mutase 1 (brain) | AK296619.1 | 0.004563 |
| 11752939_x_at | PGK1 | phosphoglycerate kinase 1 | AK298855.1 | 0.004575 |
| 11723478_s_at | CDC123 | cell division cycle 123 homolog (S. cerevisiae) | NM_006023.2 | 0.004585 |
| 11743034_x_at | RDH11 | retinol dehydrogenase 11 (all-trans/9-cis/11-cis) | AK289427.1 | 0.004593 |
| 11754086_x_at | VAPA | VAMP (vesicle-associated membrane protein)-associated protein A, 33kDa | BT019618.1 | 0.004657 |
| 11749303_s_at | HNRNPD | heterogeneous nuclear ribonucleoprotein D (AU-rich element RNA binding protein 1, 37kDa) | AK300149.1 | 0.004671 |
| 11753142_a_at | PSMD11 | proteasome (prosome, macropain) 26S subunit, non-ATPase, 11 | AK300342.1 | 0.004673 |
| 11747719_a_at | KIAA0391 | KIAA0391 | AK304066.1 | 0.004719 |
| 11748974_s_at | CWF19L1 | CWF19-like 1, cell cycle control (S. pombe) | AK295303.1 | 0.004746 |
| 11751133_a_at | ATP6V1B2 | ATPase, H+ transporting, lysosomal 56/58kDa, V1 subunit B2 | AK298819.1 | 0.004759 |
| 11715552_a_at | IMMT | inner membrane protein, mitochondrial | NM_006839.2 | 0.004766 |
| 11747365_a_at | QARS | glutaminyl-tRNA synthetase | AK302867.1 | 0.004855 |
| 11758248_s_at | SDHD | succinate dehydrogenase complex, subunit D, integral membrane protein | BF696015 | 0.004909 |
| 11753592_x_at | EEF1G | eukaryotic translation elongation factor 1 gamma | AK299876.1 | 0.004961 |
| 11715369_s_at | NDUFA4 | NADH dehydrogenase (ubiquinone) 1 alpha subcomplex, 4, 9kDa | BC105295.1 | 0.005125 |
| 11715499_x_at | CBX3 | chromobox homolog 3 | U26312.1 | 0.005141 |
| 11715874_s_at | ATP5H | ATP synthase, H+ transporting, mitochondrial Fo complex, subunit d | NM_006356.2 | 0.005143 |
| 11758311_s_at | SDHD | succinate dehydrogenase complex, subunit D, integral membrane protein | B697775 | 0.005199 |
| 11715883_x_at | DAP3 | death associated protein 3 | NM_004632.2 | 0.005251 |
| 11754030_a_at | PABPC4 | poly(A) binding protein, cytoplasmic 4 (inducible form) | BC118568.1 | 0.00537 |
| 11749682_s_at | EXOC5 | exocyst complex component 5 | AK303531.1 | 0.005371 |
| 11718142_a_at | TTC27 | tetratricopeptide repeat domain 27 | NM_017735.3 | 0.005424 |
| 11754067_a_at | TXNDC9 | thioredoxin domain containing 9 | CR456935.1 | 0.005482 |
| 11716509_a_at | AKR1A1 | aldo-keto reductase family 1, member A1 (aldehyde reductase) | NM_006066.2 | 0.005483 |

**Table 7 - Type II Dermis; Up-regulated**

| GeneTitan_ID | Gene | Title | Public ID | p |
|---|---|---|---|---|
| 11715351_at | COL1A1 | collagen, type I, alpha 1 | NM_000088.3 | 0.000139 |
| 11715352_x_at | COL1A1 | collagen, type I, alpha 1 | NM_000088.3 | 0.000375 |
| 11734105_a_at | PNMAL2 | PNMA-like 2 | AB033009.1 | 0.001583 |
| 11763844_s_at | UBXN6 | UBX domain protein 6 | CR590857.1 | 0.001819 |
| 11756896_a_at | COL6A6 | collagen, type VI, alpha 6 | AL713792.1 | 0.002188 |
| 11715284_x_at | C15orf40 | chromosome 15 open reading frame 40 | g237858663 | 0.003648 |
| 11715852_at | PDGFRB | platelet-derived growth factor receptor, beta polypeptide | NM_002609.3 | 0.005919 |
| 11715888_s_at | PIP4K2B | phosphatidylinositol-5-phosphate 4-kinase, type II, beta | NM_003559.4 | 0.006199 |
| 11724481_a_at | C5orf13 | chromosome 5 open reading frame 13 | NM_004772.2 | 0.006404 |
| 11758388_s_at | LHX8 | LIM homeobox 8 | DB302169 | 0.006562 |
| 11727836_a_at | GPR78 | G protein-coupled receptor 78 | NM_001014447.1 | 0.007388 |
| 11729827_at | FAM110B | family with sequence similarity 110, member B | BC024294.1 | 0.009523 |
| 11744562_x_at | FAM176B | family with sequence similarity 176, member B | BC071697.1 | 0.009813 |
| 11725867_s_at | EBF3 | early B-cell factor 3 | NM_001005463.1 | 0.009883 |
| 11749069_a_at | PAQR4 | progestin and adipoQ receptor family member IV | AK295348.1 | 0.010054 |
| 11723068_at | CRHBP | corticotropin releasing hormone binding protein | NM_001882.3 | 0.011101 |
| 11723174_a_at | FNDC1 | fibronectin type III domain containing 1 | NM_032532.2 | 0.011173 |
| 11717274_s_at | COL5A1 | collagen, type V, alpha 1 | BQ007762 | 0.012177 |
| 11729541_a_at | CAMKK2 | calcium/calmodulin-dependent protein kinase kinase 2, beta | AB081337.1 | 0.012395 |
| 11724848_a_at | DIXDC1 | DIX domain containing 1 | DB358954 | 0.013451 |
| 11726830_at | ANTXR1 | anthrax toxin receptor 1 | NM_018153.3 | 0.013945 |
| 11727296_s_at | TGFB3 | transforming growth factor, beta 3 | NM_003239.2 | 0.014199 |
| 11759905_a_at | EXD3 | exonuclease 3'-5' domain containing 3 | BC110879.1 | 0.01495 |
| 11721372_at | TCF7L1 | transcription factor 7-like 1 (T-cell specific, HMG-box) | NM_031283.1 | 0.01515 |
| 11755955_a_at | FAP | fibroblast activation protein, alpha | AL832166.1 | 0.015704 |
| 11725989_x_at | MMP14 | matrix metallopeptidase 14 (membrane-inserted) | NM_004995.2 | 0.019095 |
| 11717272_at | COL5A1 | collagen, type V, alpha 1 | AB371583.1 | 0.020092 |
| 11739544_a_at | C19orf12 | chromosome 19 open reading frame 12 | BX328123 | 0.021 |
| 11727867_a_at | CLEC3B | C-type lectin domain family 3, member B | NM_003278.2 | 0.024069 |
| 11731645_a_at | CAMKK2 | calcium/calmodulin-dependent protein kinase kinase 2, beta | BC026060.2 | 0.024228 |
| 11729101_a_at | AKR1C2 | aldo-keto reductase family 1, member C2 (dihydrodiol dehydrogenase 2; bile acid binding protein; 3-alpha hydroxysteroid dehydrogenase, type III) | NM_205845.1 | 0.024814 |
| 11726474_a_at | HES4 | hairy and enhancer of split 4 (Drosophila) | NM_021170.3 | 0.025047 |
| 11725224_a_at | ZNF193 | zinc finger protein 193 | NM_006299.3 | 0.026826 |
| 11715350_a_at | COL1A1 | collagen, type I, alpha 1 | BC036531.2 | 0.026857 |
| 11741377_a_at | MMP2 | matrix metallopeptidase 2 (gelatinase A, 72kDa gelatinase, 72kDa type IV MMP2 collagenase) | NM_001127891.1 | 0.02687 |
| 11722292_a_at | NYNRIN | NYN domain and retroviral integrase containing | NM_025081.2 | 0.027121 |
| 11730404_at | MEX3B | mex-3 homolog B (C. elegans) | NM_032246.3 | 0.027161 |
| 11759126_a_at | THRA | thyroid hormone receptor, alpha (erythroblastic leukemia viral (v-erb-a) oncogene homolog, avian) | CB054873 | |
| 11744348_x_at | COL6A2 | collagen, type VI, alpha 2 | BC002484.2 | 0.027551 |
| 11720845_a_at | CD248 | CD248 molecule, endosialin | NM_020404.2 | 0.02836 |
| 11720372_at | TESC | tescalcin | NM_017899.2 | 0.028677 |
| 11752890_a_at | SNTA1 | syntrophin, alpha 1 (dystrophin-associated protein A1, 59kDa, acidic component) | AK301800.1 | 0.029777 |
| 11717273_at | COL5A1 | collagen, type V, alpha 1 | BQ007762 | 0.030174 |
| 11754184_a_at | ALDH1A3 | aldehyde dehydrogenase 1 family, member A3 | BX538027.1 | 0.030605 |
| 11727155_a_at | TRIOBP | TRIO and F-actin binding protein | NM_007032.5 | 0.030721 |
| 11727031_a_at | SQSTM1 | sequestosome 1 | NM_003900.4 | 0.031363 |
| 11720846_at | CD248 | CD248 molecule, endosialin | NM_020404.2 | 0.031411 |
| 11761938_a_at | TRIO | triple functional domain (PTPRF interacting) | AB115332.1 | 0.032433 |
| 11718096_a_at | MEF2A | myocyte enhancer factor 2A | BC013437.2 | 0.03254 |
| 11734906_a_at | NOVA1 | neuro-oncological ventral antigen 1 | NM_002515.2 | 0.032886 |
| 11724619_at | RSPO3 | R-spondin 3 homolog (Xenopus laevis) | NM_032784.3 | 0.033442 |
| 11726188_at | SHISA3 | shisa homolog 3 (Xenopus laevis) | NM_001080505.1 | 0.033466 |
| 11729644_a_at | GPX8 | glutathione peroxidase 8 (putative) | AK074216.1 | 0.033979 |
| 11756359_s_at | ADRA2C | adrenergic, alpha-2C-, receptor | CR590957.1 | 0.034296 |
| 11747064_x_at | ANXA11 | annexin A11 | AK301047.1 | 0.034459 |
| 11732785_a_at | C16orf45 | chromosome 16 open reading frame 45 | NM_001142469.1 | 0.035589 |
| 11727030_s_at | MAP1A | microtubule-associated protein 1A | NM_002373.5 | 0.036125 |
| 11748738_a_at | SEMA3E | sema domain, immunoglobulin domain (Ig), short basic domain, secreted, (semaphorin) 3E | AK303925.1 | 0.036605 |
| 11721995_a_at | LRRC32 | leucine rich repeat containing 32 | NM_001128922.1 | 0.038953 |
| 11726189_x_at | HCFC1R1 | host cell factor C1 regulator 1 (XPO1 dependent) | NM_017885.2 | 0.039807 |
| 11754792_a_at | RGMA | RGM domain family, member A | AK125204.1 | 0.040435 |
| 11717123_a_at | PPPIR12B | protein phosphatase 1, regulatory (inhibitor) subunit 12B | NM_032105.1 | 0.043242 |
| 11724260_a_at | TRIO | triple functional domain (PTPRF interacting) | AF091395.1 | 0.044219 |
| 11759962_at | TPRKB | TP53RK binding protein | AY643713.1 | 0.045288 |
| 11733167_at | LRRN4CL | LRRN4 C-terminal like | BC053902.1 | 0.045766 |
| 11721703_s_at | TNRC18 | trinucleotide repeat containing 18 | NM_001080495.2 | 0.045935 |
| 11725568_a_at | ATP8A1 | ATPase, aminophospholipid transporter (APLT), class I, type 8A, member 1 | NM_001105529.1 | 0.046173 |
| 11741562_a_at | MME | membrane metallo-endopeptidase | NM_007287.2 | 0.046616 |
| 11745820_s_at | PLAGAL1 | pleiomorphic adenoma gene-like 1 | BQ026948 | 0.046856 |
| 11743696_at | CLEC14A | C-type lectin domain family 14, member A | CA412481 | 0.047788 |
| 11720277_a_at | OLFML2A | olfactomedin-like 2A | NM_182487.2 | 0.049248 |

**Table 8 - Type II Dermis; Down-regulated**

| GeneTitan_ID | Gene | Title | Public ID | p |
|---|---|---|---|---|
| 11749128_x_at | MAP7 | microtubule-associated protein 7 | AK299355.1 | 0.000033 |
| 11720184_x_at | EEF1B2 | eukaryotic translation elongation factor 1 beta 2 | NM_001959.3 | 0.000101 |
| 11724155_at | SULT1E1 | sulfotransferase family 1E, estrogen-preferring, member 1 | U08098.1 | 0.000109 |
| 200062_PM_s_at | RPL30 | ribosomal protein L30 | L05095.1 | 0.000123 |
| 11742734_s_at | WDR3 | WD repeat domain 3 | AK292438.1 | 0.000139 |
| 11732205_x_at | NAP1L1 | nucleosome assembly protein 1-like 1 | BX413854 | 0.000141 |
| 11743604_s_at | RRM1 | ribonucleotide reductase M1 | BE618815 | 0.000164 |
| 11723197_at | HNRNPA3 | heterogeneous nuclear ribonucleoprotein A3 | B2434302 | 0.000178 |
| 11732684_a_at | ABCA12 | ATP-binding cassette, sub-family A (ABC1), member 12 | AF418105.1 | 0.000237 |
| 11739308_s_at | DLG1 | discs, large homolog 1 (Drosophila) | BM681931 | 0.000254 |
| 11725875_at | WDR66 | WD repeat domain 66 | NM_144668.4 | 0.000271 |
| 200082_PM_s_at | RPS7 | ribosomal protein S7 | AI805587 | 0.00036 |
| 200081_PM_s_at | RPS6 | ribosomal protein S6 | BE741754 | 0.000371 |
| 200063_PM_s_at | NPM1 | nucleophosmin (nucleolar phosphoprotein B23, numatrin) | BC002398.1 | 0.000404 |
| 11757356_x_at | RPL30 | ribosomal protein L30 | BM855760 | 0.000405 |
| 11719783_at | RPS23 | ribosomal protein S23 | D14530.1 | 0.000416 |
| 11724156_at | SULT1E1 | sulfotransferase family 1E, estrogen-preferring, member 1 | NM_005420.2 | 0.000461 |
| 11720183_s_at | EEF1B2 | eukaryotic translation elongation factor 1 beta 2 | NM_001959.3 | 0.000477 |
| 11745154_a_at | NCL | nucleolin | BC006516.2 | 0.000485 |
| 11715958_s_at | RPL7 | ribosomal protein L7 | NM_000971.3 | 0.000491 |
| 11749558_a_at | CYP4F8 | cytochrome P450, family 4, subfamily F, polypeptide 8 | AK300530.1 | 0.000497 |
| 11755203_x_at | RPL21 | ribosomal protein L21 | BX647669.1 | 0.000511 |
| 11728022_a_at | TMEM45A | transmembrane protein 45A | NM_018004.1 | 0.000533 |
| 11718273_a_at | EIF3L | eukaryotic translation initiation factor 3, subunit L | NM_016091.2 | 0.000658 |
| 11757399_s_at | PRKDC | protein kinase, DNA-activated, catalytic polypeptide | A760328 | 0.000698 |
| 11736055_at | C10orf32 | chromosome 10 open reading frame 32 | BG696280 | 0.000732 |
| 11749267_a_at | SRD5A1 | steroid-5-alpha-reductase, alpha polypeptide 1 (3-oxo-5 alpha-steroid delta 4-dehydrogenase alpha 1) | AK315996.1 | 0.000744 |
| 11716946_s_at | TM9SF3 | transmembrane 9 superfamily member 3 | AF269150.1 | 0.000758 |
| 11726461_a_at | PDCD2 | programmed cell death 2 | NM_144781.1 | 0.000759 |
| 11763318_s_at | CSNK1A1 | casein kinase 1, alpha 1 | BC040473.1 | 0.000792 |
| 11727794_s_at | EIF3E | eukaryotic translation initiation factor 3, subunit E | NM_001568.2 | 0.000802 |
| 11731690_a_at | PIK3C2G | phosphoinositide-3-kinase, class 2, gamma polypeptide | NM_004570.4 | 0.000846 |
| 11748052_x_at | EI24 | etoposide induced 2.4 mRNA | AK316539.1 | 0.000851 |
| 11743094_at | SPRR4 | small proline-rich protein 4 | BC069445.1 | 0.000998 |
| 11717236_x_at | RPS7 | ribosomal protein S7 | NM_001011.3 | 0.001062 |
| 11718719_at | KIAA1797 | KIAA1797 | NM_017794.3 | 0.001102 |
| 11752908_a_at | TCEA1 | transcription elongation factor A (SII), 1 | AK297729.1 | 0.001141 |
| 11753659_x_at | RPL30 | ribosomal protein L30 | BC095426.1 | 0.001161 |
| 11717058_x_at | RPL5 | ribosomal protein L5 | NM_000969.3 | 0.001214 |
| 11736057_s_at | C10orf32 | chromosome 10 open reading frame 32 | B685637 | 0.00123 |
| 11742991_a_at | PSAT1 | phosphoserine aminotransferase 1 | AK295222.1 | 0.001244 |
| 11758357_x_at | RPL9 | ribosomal protein L9 | B172613 | 0.00126 |
| 200010_PM_at | RPL11 | ribosomal protein L11 | NM_000975.1 | 0.001282 |
| 11749776_a_at | TFAP2C | transcription factor AP-2 gamma (activating enhancer binding protein 2 gamma) | AK301572.1 | 0.001283 |
| 11727995_a_at | SPINK5 | serine peptidase inhibitor, Kazal type 5 | DQ149928.1 | 0.001284 |
| 11722185_at | C14orf147 | chromosome 14 open reading frame 147 | NM_138288.3 | 0.001309 |
| 11750883_a_at | EIF2A | eukaryotic translation initiation factor 2A, 65kDa | AF109358.1 | 0.001327 |
| 200017_PM_at | RPS27A | ribosomal protein S27a | NM_002954.1 | 0.001414 |
| 11749559_x_at | CYP4F8 | cytochrome P450, family 4, subfamily F, polypeptide 8 | AK300530.1 | 0.001416 |
| 11722308_a_at | TP63 | tumor protein p63 | NM_003722.4 | 0.001433 |
| 11754066_x_at | NPM1 | nucleophosmin (nucleolar phosphoprotein B23, numatrin) | BT007011.1 | 0.001448 |
| 11743603_a_at | RRM1 | ribonucleotide reductase M1 | BE618815 | 0.001462 |
| 11754963_a_at | SPINK5 | serine peptidase inhibitor, Kazal type 5 | Auk301660.1 | 0.001558 |
| 11747333_a_at | HSD17B4 | hydroxysteroid (17-beta) dehydrogenase 4 | AK295440.1 | 0.001571 |
| 11757386_x_at | NPM1 | nucleophosmin (nucleolar phosphoprotein B23, numatrin) | AL563600 | 0.00169 |
| 11751437_a_at | CYP4F8 | cytochrome P450, family 4, subfamily F, polypeptide 8 | AK300539.1 | 0.001708 |
| 11726258_at | RNF141 | ring finger protein 141 | BX503543 | 0.001716 |
| 11720766_a_at | METTL9 | methyltransferase like 9 | AK074529.1 | 0.001743 |
| 11743729_at | CCDC47 | coiled-coil domain containing 47 | A575693 | 0.001771 |
| 11749786_x_at | HNRNPF | heterogeneous nuclear ribonucleoprotein F | AK296696.1 | 0.001879 |
| 11729152_a_at | EIF3M | eukaryotic translation initiation factor 3, subunit M | NM_006360.3 | 0.001895 |
| 11737634_a_at | UGT2A1 | UDP glucuronosyltransferase 2 family, polypeptide A1 | NM_006798.2 | 0.001986 |
| 11748044_a_at | SCEL | sciellin | BC020726.1 | 0.002038 |
| 11752912_x_at | EIF3M | eukaryotic translation initiation factor 3, subunit M | AK292139.1 | 0.002066 |
| 11736309_a_at | CSNK1A1 | casein kinase 1, alpha 1 | L37042.1 | 0.002093 |
| 11727421_a_at | CANX | calnexin | C243867 | 0.002106 |
| 11727425_s_at | CANX | calnexin | M94859.1 | 0.002182 |
| 11757305_x_at | RPSAP58 | ibosomal protein SA pseudogene 58 | BI762726 | 0.002211 |
| 200087_PM_s_at | TMED2 | transmembrane emp24 domain trafficking protein 2 | AK024976.1 | 0.002219 |
| 11718030_at | RAB11A | RAB11A, member RAS oncogene family | NM_004663.3 | 0.002258 |
| 11742887_a_at | BAG5 | BCL2-associated athanogene 5 | BQ008934 | 0.00227 |
| 11746199_a_at | METTL9 | methyltransferase like 9 | AA524199 | 0.002298 |
| 11756182_s_at | PSAT1 | phosphoserine aminotransferase 1 | AA173918 | 0.002312 |
| 11727658_s_at | KLK10 | kallikrein-related peptidase 10 | AF024605.1 | 0.002335 |
| 11732204_a_at | NAP1L1 | nucleosome assembly protein 1-like 1 | BX413854 | 0.002341 |
| 11744334_x_at | RPS17 | ribosomal protein S17 | BC071928.1 | 0.002403 |
| 11748536_a_at | DIMT1L | DIM1 dimethyladenosine transferase 1-like (S. cerevisiae) | BC002841.2 | 0.002406 |
| 11745155_s_at | NCL | nucleolin | BC006516.2 | 0.002458 |
| 11730790_x_at | NPM1 | nucleophosmin (nucleolar phosphoprotein B23, numatrin) | AK290652.1 | 0.002509 |
| 11740643_a_at | CYP4F8 | cytochrome P450, family 4, subfamily F, polypeptide 8 | AF133298.1 | 0.002616 |
| 11755057_s_at | ATP2C1 | ATPase, Ca++ transporting, type 2C, member 1 | AB037768.1 | 0.002687 |
| 11742992_s_at | PSAT1 | phosphoserine aminotransferase 1 | AK295222.1 | 0.002716 |
| 11757424_x_at | RPL37 | ribosomal protein L37 | F34903 | 0.002789 |
| 11723312_a_at | PXMP2 | peroxisomal membrane protein 2, 22kDa | NM_018663.1 | 0.002819 |
| 11756137_x_at | BTF3 | basic transcription factor 3 | CA772090 | 0.002824 |
| 11754054_x_at | RPL3 | ribosomal protein L3 | L22453.1 | 0.002905 |
| 11750667_a_at | RRM1 | ribonucleotide reductase M1 | AK297988.1 | 0.00292 |
| 11751326_a_at | RAB11A | RAB11A, member RAS oncogene family | AK300008.1 | 0.002998 |
| 11749040_a_at | PGM2 | phosphoglucomutase 2 | AK297752.1 | 0.003008 |
| 11715376_a_at | RPS11 | ribosomal protein S11 | NM_001015.3 | 0.003035 |
| 11715849_a_at | DDX47 | DEAD (Asp-Glu-Ala-Asp) box polypeptide 47 | NM_016355.3 | 0.003047 |
| 11726829_at | TYW1B | tRNA-yW synthesizing protein 1 homolog B (S. cerevisiae) | NM_001145440.1 | 0.003195 |
| 11756267_x_at | C14orf166 | chromosome 14 open reading frame 166 | BX349547 | 0.003217 |
| 11756437_x_at | RPS18 | ribosomal protein S18 | BQ057441 | 0.003368 |
| 11715648_x_at | ADIPOR1 | adiponectin receptor 1 | AY424279.1 | 0.003389 |
| 11747662_x_at | PTGES3 | prostaglandin E synthase 3 (cytosolic) | AK298147.1 | 0.003391 |
| 11749546_a_at | SLC39A6 | solute carrier family 39 (zinc transporter), member 6 | AK301539.1 | 0.003463 |
| 11745720_s_at | PDIA6 | protein disulfide isomerase family A, member 6 | D49489.1 | 0.00351 |
| 200074_PM_s_at | RPL14 | ribosomal protein L14 | U16738.1 | 0.003587 |
| 11746023_a_at | PGD | phosphogluconate dehydrogenase | AK304423.1 | 0.003624 |

**Table 9 - Type III Epidermis; Up-regulated**

| GeneTitan_ID | Gene | Title | Public ID | p |
|---|---|---|---|---|
| 11725675_a_at | RORA | RAR-related orphan receptor A | AA034012 | 0.00064 |
| 11732366_a_at | SCAPER | S-phase cyclin A-associated protein in the ER | BC015212.1 | 0.001096 |
| 11739639_at | CDK12 | cyclin-dependent kinase 12 | AW968504 | 0.001477 |
| 11745215_a_at | KBTBD4 | kelch repeat and BTB (POZ) domain containing 4 | CR457270.1 | 0.001496 |
| 11730873_a_at | RASSF5 | Ras association (RalGDS/AF-6) domain family member 5 | NM_182665.2 | 0.001694 |
| 11718966_at | NUFIP2 | nuclear fragile X mental retardation protein interacting protein 2 | BU533767 | 0.001959 |
| 11718513_x_at | TSPAN14 | tetraspanin 14 | NM_030927.2 | 0.002425 |
| 11744585_a_at | ATRN | attractin | AK302730.1 | 0.003547 |
| 11755420_a_at | KDM4B | lysine (K)-specific demethylase 4B | AK126854.1 | 0.003701 |
| 11720895_at | SOS1 | son of sevenless homolog 1 (Drosophila) | BM970418 | 0.003776 |
| 11734873_a_at | SCAPER | S-phase cyclin A-associated protein in the ER | NM_020843.2 | 0.00429 |
| 11759897_x_at | OFD1 | oral-facial-digital syndrome 1 | BC042830.1 | 0.004666 |
| 11754251_a_at | USP36 | ubiquitin specific peptidase 36 | AK022913.1 | 0.005023 |
| 11736059_a_at | KIF5B | kinesin family member 5B | BC065267.1 | 0.005318 |
| 11754824_a_at | HSPC159 | galectin-related protein | DB323149 | 0.005358 |
| 11757991_s_at | ANKRD12 | ankyrin repeat domain 12 | AA399583 | 0.006238 |
| 11744468_at | SYNCRIP | synaptotagmin binding, cytoplasmic RNA interacting protein | AK056188.1 | 0.007087 |
| 11754992_a_at | CHD1 | chromodomain helicase DNA binding protein 1 | BE535223 | 0.007613 |
| 11716283_at | PAPD7 | PAP associated domain containing 7 | NM_006999.3 | 0.00802 |
| 11722973_s_at | FOXK1 | forkhead box K1 | | 0.008695 |
| 11748149_a_at | FNBP1 | formin binding protein 1 | AK293743.1 | 0.009063 |
| 11722125_a_at | C3orf19 | chromosome 3 open reading frame 19 | AL526467 | 0.009976 |
| 11720007_a_at | STEAP4 | STEAP family member 4 | NM_024636.2 | 0.011197 |
| 11731506_a_at | RAD23B | RAD23 homolog B (S. cerevisiae) | NM_002874.3 | 0.011526 |
| 11724759_s_at | CALM1 | calmodulin 1 (phosphorylase kinase, delta) | NM_006888.3 | 0.011622 |
| 11718161_at | KLF13 | Kruppel-like factor 13 | AF132599.1 | 0.012064 |
| 11726305_at | C10orf84 | chromosome 10 open reading frame 84 | BC023577.2 | 0.012188 |
| 11723962_at | KIAA1143 | KIAA1143 | BC008468.1 | 0.012597 |
| 11740956_x_at | PLEKHN1 | in homology domain containing, family N member 1 | NM_032129.1 | 0.013256 |
| 11722305_at | ARHGAP23 | Rho GTPase activating protein 23 | NM_020876.1 | 0.01353 |
| 11726022_a_at | FAM177A1 | family with sequence similarity 177, member A1 | BC029559.1 | 0.01364 |
| 11754010_x_at | GOLGA2 | golgin A2 | BT007248.1 | 0.013918 |
| 11729523_a_at | NLRC5 | ily, CARD domain containing 5 | NM_032206.3 | 0.014235 |
| 11723113_a_at | CENPC1 | centromere protein C 1 | BC041117.1 | 0.0145 |
| 11754616_a_at | UPF1 | UPF1 regulator of nonsense transcripts homolog (yeast) | AI690963 | 0.014647 |
| 11722291_s_at | ZBTB43 | zinc finger and BTB domain containing 43 | AI745225 | 0.014686 |
| 11739805_a_at | RASAL2 | RAS protein activator like 2 | AK075169.1 | 0.014761 |
| 11723502_at | PRLR | prolactin receptor | AI435838 | 0.014945 |
| 11726244_a_at | RORA | RAR-related orphan receptor A | U04898.1 | 0.01551 |
| 11736104_a_at | ZNF750 | zinc finger protein 750 | BC109037.1 | 0.015749 |
| 11723184_x_at | CNOT6L | CCR4-NOT transcription complex, subunit 6-like | BQ025327 | 0.015988 |
| 11755058_a_at | BAZ1A | bromodomain adjacent to zinc finger domain, 1A | BC020636.1 | 0.016704 |
| 11759600_at | SFRS18 | Splicing factor, arginine/serine-rich 18 | AK027751.1 | 0.016758 |
| 11744829_s_at | HLA-E | major histocompatibility complex, class I, E | AK296822.1 | 0.016828 |
| 11719447_s_at | GBP2 | guanylate binding protein 2, interferon-inducible | BC073163.1 | 0.016832 |
| 11720541_at | HSPC159 | galectin-related protein | NM_014181.2 | 0.017088 |
| 11719028_a_at | PSD3 | pleckstrin and Sec7 domain containing 3 | DB314358 | 0.017377 |
| 11754462_a_at | RSPRY1 | ring finger and SPRY domain containing 1 | AU253443 | 0.017904 |
| 11725676_a_at | RORA | RAR-related orphan receptor A | NM_002943.3 | 0.018226 |
| 11722290_a_at | ZBTB43 | zinc finger and BTB domain containing 43 | AI745225 | 0.018329 |
| 11723821_a_at | SMURF2 | SMAD specific E3 ubiquitin protein ligase 2 | AY014180.1 | 0.018461 |
| 11720111_at | SNTB2 | syntrophin, beta 2 (dystrophin-associated protein A1, 59kDa, basic component 2) | BC036429.1 | 0.018669 |
| 11736432_x_at | PPP4R2 | protein phosphatase 4, regulatory subunit 2 | BC128136.1 | 0.01869 |
| 11744000_a_at | NFKBIA | nuclear factor of kappa light polypeptide gene enhancer in B-cells inhibitor, alpha | BX367826 | 0.018695 |
| 11759512_x_at | CWC25 | CWC25 spliceosome-associated protein homolog (S. cerevisiae) | CR748127 | 0.018768 |
| 11716095_s_at | KLF6 | Kruppel-like factor 6 | CD366698 | 0.019227 |
| 11754447_a_at | RPS6KA5 | ribosomal protein S6 kinase, 90kDa, polypeptide 5 | BM968829 | 0.019584 |
| 11719085_a_at | SMARCC2 | SWI/SNF related, matrix associated, actin dependent regulator of chromatin, subfamily c, member 2 | AL544435 | 0.01997 |
| 11727506_x_at | RAB21 | RAB21, member RAS oncogene family | BC021901.1 | 0.020551 |
| 11720276_s_at | TREX1 | three prime repair exonuclease 1 | NM_016381.3 | 0.020671 |
| 11724549_a_at | RSBN1 | round spermatid basic protein 1 | AK292552.1 | 0.020675 |
| 11731645_a_at | CAMKK2 | calcium/calmodulin-dependent protein kinase kinase 2, beta | BC026060.2 | 0.020762 |
| 11737413_at | MICALCL | MICAL C-terminal like | NM_032867.2 | 0.020903 |
| 11715938_x_at | KHDRBS1 | KH domain containing, RNA binding, signal transduction associated 1 | BC000717.1 | 0.021833 |
| 11747192_x_at | NFIC | nuclear factor I/C (CCAAT-binding transcription factor) | AK289885.1 | 0.022039 |
| 11729396_a_at | NEK1 | NIMA (never in mitosis gene a)-related kinase 1 | Z25431.1 | 0.022138 |
| 11727064_a_at | ANKRD11 | ankyrin repeat domain 11 | BU674634 | 0.022386 |
| 11752626_a_at | PBX1 | pre-B-cell leukemia homeobox 1 | AK299673.1 | 0.022397 |
| 11721119_a_at | ANKHD1-EIF4EBP3 | ANKHD1-EIF4EBP3 readthrough | AF217646.1 | 0.022562 |
| 11743648_a_at | DCAF6 | DDB1 and CUL4 associated factor 6 | BF672818 | 0.022893 |
| 11740362_a_at | FOXN3 | forkhead box N3 | U68723.1 | 0.023025 |
| 11718869_x_at | PALMD | palmdelphin | CF552454 | 0.023068 |
| 11727604_a_at | EPB41L4A | erythrocyte membrane protein band 4.1 like 4A | NM_022140.3 | 0.023101 |
| 11726633_s_at | TRIM8 | tripartite motif-containing 8 | BC021925.1 | 0.023187 |
| 11732370_a_at | CUX1 | cut-like homeobox 1 | NM_181552.2 | 0.023258 |
| 11726113_a_at | FAM46B | family with sequence similarity 46, member B | NM_052943.3 | 0.023515 |
| 11729100_a_at | TTC18 | tetratricopeptide repeat domain 18 | NM_145170.3 | 0.023862 |
| 11729259_a_at | ZNF644 | zinc finger protein 644 | BQ014639 | 0.023971 |
| 11745806_a_at | AMMECR1L | AMME chromosomal region gene 1-like | AK095871.1 | 0.024186 |
| 11717894_s_at | PTP4A1 | protein tyrosine phosphatase type IVA, member 1 | BC023975.2 | 0.024306 |
| 11728765_a_at | PVRL4 | poliovirus receptor-related 4 | BC010423.1 | 0.024371 |
| 11740747_a_at | DNMT3A | DNA (cytosine-5-) methyltransferase 3 alpha | AF480163.1 | 0.024617 |
| 11718868_a_at | PALMD | palmdelphin | CF552454 | 0.025394 |
| 11758133_s_at | COL4A3BP | collagen, type IV, alpha 3 (Goodpasture antigen) binding protein | BE046819 | 0.026883 |
| 11749969_a_at | TSPAN5 | tetraspanin 5 | AK295385.1 | 0.027108 |
| 11733899_a_at | TROVE2 | TROVE domain family, member 2 | BX445026 | 0.027171 |
| 11747743_x_at | MTF2 | metal response element binding transcription factor 2 | AK302776.1 | 0.027946 |
| 11746790_a_at | BECN1 | beclin 1, autophagy related | AK298619.1 | 0.027999 |
| 11731573_a_at | FRMD4B | FERM domain containing 4B | AU147415 | 0.028099 |
| 11724271_a_at | HLF | hepatic leukemia factor | EL952952 | 0.028251 |
| 11728683_x_at | KRR1 | KRR1, small subunit (SSU) processome component, homolog (yeast) | U55766.1 | 0.02827 |
| 11758327_s_at | BAZ1A | bromodomain adjacent to zinc finger domain, 1A | BF852255 | 0.02853 |
| 11743300_a_at | SRP72 | signal recognition particle 72kDa | AK225430.1 | 0.028582 |
| 11719103_at | CPNE3 | copine III | CB250550 | 0.028667 |
| 11755895_a_at | FAM129A | family with sequence similarity 129, member A | AK095547.1 | 0.029014 |
| 11721326_at | C3orf14 | chromosome 3 open reading frame 14 | AF236158.1 | 0.029423 |
| 11738035_s_at | RTN4 | reticulon 4 | AK302741.1 | 0.029458 |
| 11746122_s_at | ZC3H11A | zinc finger CCCH-type containing 11A | DA094705 | 0.029486 |
| 11724085_at | DAPL1 | death associated protein-like 1 | NM_001017920.2 | 0.030615 |
| 11735181_a_at | DLX2 | distal-less homeobox 2 | NM_004405.3 | 0.030619 |

**Table 10 - Type III Epidermis; Down-regulated**

| GeneTitan_ID | Gene | Title | Public ID | p |
|---|---|---|---|---|
| 11743060_s_at | COMMD10 | COMM domain containing 10 | AL572695 | 0.000211 |
| 11720515_s_at | C9orf150 | chromosome 9 open reading frame 150 | NM_203403.1 | 0.000414 |
| 11716897_x_at | PPIE | peptidylprolyl isomerase E (cyclophilin E) | NM_006112.2 | 0.000535 |
| 11725787_a_at | C4orf43 | chromosome 4 open reading frame 43 | NM_018352.2 | 0.000571 |
| 11729680_a_at | KHK | ketohexokinase (fructokinase) | CR456801.1 | 0.000981 |
| 11745494_x_at | ERCC8 | excision repair cross-complementing rodent repair deficiency, complementation group 8 | U28413.1 | 0.001042 |
| 11745948_a_at | CHEK1 | CHK1 checkpoint homolog (S. pombe) | AK299783.1 | 0.0011 |
| 11758965_at | ATG4C | ATG4 autophagy related 4 homolog C (S. cerevisiae) | AK027773.1 | 0.001439 |
| 11724079_s_at | E2F2 | E2F transcription factor 2 | NM_004091.2 | 0.001667 |
| 11718280_s_at | TRIAP1 | TP53 regulated inhibitor of apoptosis 1 | NM_016399.2 | 0.001683 |
| 11720459_s_at | CAPRIN1 | cell cycle associated protein 1 | BQ002768 | 0.001735 |
| 11720615_a_at | TUBG2 | tubulin, gamma 2 | NM_016437.2 | 0.00174 |
| 11757673_x_at | RPL39 | ribosomal protein L39 | BX435916 | 0.001879 |
| 11720398_a_at | NBN | nibrin | BC146797.1 | 0.002186 |
| 11734661_a_at | CLSTN3 | calsyntenin 3 | NM_014718.3 | 0.0022 |
| 11726529_s_at | BRCC3 | BRCA1/BRCA2-containing complex, subunit 3 | NM_024332.2 | 0.002236 |
| 11758291_s_at | MRPS10 | mitochondrial ribosomal protein S10 | BF701142 | 0.002439 |
| 11726660_a_at | GPN3 | GPN-loop GTPase 3 | AY359078.1 | 0.002577 |
| 11734619_x_at | ALOX15B | arachidonate 15-lipoxygenase, type B | NM_001141.2 | 0.002749 |
| 11719482_a_at | MRPK21 | mitochondrial ribosomal protein L21 | NM_181515.1 | 0.002874 |
| 11716896_a_at | PPIE | peptidylprolyl isomerase E (cyclophilin E) | NM_006112.2 | 0.002936 |
| 11757498_s_at | TMEM106C | transmembrane protein 106C | AI278554 | 0.003263 |
| 11722842_s_at | ENAH | enabled homolog (Drosophila) | BC095481.1 | 0.003561 |
| 11722415_a_at | HBS1L | HBS1-like (S. cerevisiae) | BC040849.1 | 0.003563 |
| 11742050_a_at | API5 | apoptosis inhibitor 5 | AK294724.1 | 0.003612 |
| 11719499_at | MAOB | monoamine oxidase B | NM_000898.4 | 0.003642 |
| 11717099_at | HIST1H2BK | histone cluster 1, H2bk | NM_080593.1 | 0.003648 |
| 11744666_at | FAN1 | FAN CD2/FANCI-associated nuclease 1 | BC047882.1 | 0.003687 |
| 11756910_x_at | FANCD2 | Fanconi anemia, complementation group D2 | AL832427.1 | 0.00395 |
| 11758535_s_at | GPAM | glycerol-3-phosphate acyltransferase, mitochondrial | AI074401 | 0.003969 |
| 11719920_at | FXC1 | fracture callus 1 homolog (rat) | BC011014.1 | 0.004197 |
| 11719571_a_at | RCHY1 | ring finger and CHY zinc finger domain containing 1 | BC047393.1 | 0.004345 |
| 11763339_a_at | SIVA1 | SIVA1, apoptosis-inducing factor | AK128704.1 | 0.004492 |
| 11740706_a_at | NFRKB | nuclear factor related to kappaB binding protein | NM_006165.3 | 0.004845 |
| 11730410_a_at | PXMP4 | peroxisomal membrane protein 4, 24kDa | NM_007238.4 | 0.005105 |
| 11739973_s_at | NUAK1 | NUAK family, SNF1-like kinase, 1 | BU686994 | 0.005564 |
| 11757687_x_at | DAD1 | defender against cell death 1 | BU535881 | 0.005572 |
| 11721430_a_at | SYBU | syntabulin (syntaxin-interacting) | NM_001099744.1 | 0.005683 |
| 11722555_s_at | HADH | hydroxyacyl-CoA dehydrogenase | CR591982.1 | 0.005987 |
| 11736367_a_at | MCM10 | minichromosome maintenance complex component 10 | NM_182751.1 | 0.006448 |
| 11739660_x_at | PPCS | phosphopantothenoylcysteine synthetase | NM_001077447.1 | 0.0065 |
| 11757604_a_at | SAMM50 | sorting and assembly machinery component 50 homolog (S. cerevisiae) | BQ186212 | 0.006505 |
| 11722398_s_at | RWDD4 | RWD domain containing 4 | NM_152682.2 | 0.00654 |
| 11733866_a_at | RARS2 | arginyl-tRNA synthetase 2, mitochondrial | NM_020320.3 | 0.006608 |
| 11722351_at | SRSF8 | serine/arginine-rich splicing factor 8 | NM_032102.2 | 0.006693 |
| 11726320_at | ERO1L | ERO1-like (S. cerevisiae) | NM_014584.1 | 0.006774 |
| 11744384_x_at | USMG5 | up-regulated during skeletal muscle growth 5 homolog (mouse) | BC072683.1 | 0.006853 |
| 11733975_a_at | DDHD2 | DDHD domain containing 2 | BU631346 | 0.007241 |
| 11727533_a_at | FEZ2 | fasciculation and elongation protein zeta 2 (zygin II) | NM_001042548.1 | 0.007345 |
| 11723462_a_at | PHKB | phosphorylase kinase, beta | NM_001031835.2 | 0.007666 |
| 11718475_s_at | IDH1 | isocitrate dehydrogenase 1 (NADP+), soluble | NM_005896.2 | 0.007717 |
| 11744822_a_at | NDUFB2 | NADH dehydrogenase (ubiquinone) 1 beta subcomplex, 2, 8kDa | BC063026.1 | 0.007876 |
| 11757589_a_at | NDUFA12 | NADH dehydrogenase (ubiquinone) 1 alpha subcomplex, 12 | BU537124 | 0.008475 |
| 11726186_x_at | C12orf48 | chromosome 12 open reading frame 48 | NM_017915.2 | 0.008601 |
| 11739972_at | NUAK1 | NUAK family, SNF1-like kinase, 1 | BU686994 | 0.008782 |
| 11755294_x_at | NEB | nebulin | BC063136.1 | 0.008985 |
| 11740962_a_at | UBA5 | ubiquitin-like modifier activating enzyme 5 | NM_198329.2 | 0.009092 |
| 11753867_a_at | NDUFA1 | NADH dehydrogenase (ubiquinone) 1 alpha subcomplex, 1, 7.5kDa | AB451304.1 | 0.009324 |
| 11757665_x_at | NDUFS5 | NADH dehydrogenase (ubiquinone) Fe-S protein 5, 15kDa (NADH-coenzyme Q reductase) | AA977996 | 0.009487 |
| 11757684_a_at | TPD52L2 | tumor protein D52-like 2 | AI806821 | 0.009563 |
| 11731068_s_at | FIGNL1 | fidgetin-like 1 | NM_022116.3 | 0.009587 |
| 11743064_at | CDC6 | cell division cycle 6 homolog (S. cerevisiae) | CR598029.1 | 0.009677 |
| 11746036_s_at | CBR1 | carbonyl reductase 1 | AK311219.1 | 0.009851 |
| 11729763_a_at | LSM10 | LSM10, U7 small nuclear RNA associated | NM_032881.1 | |
| 11719268_at | TNNC1 | troponin C type 1 (slow) | NM_003280.2 | 0.010462 |
| 11758199_s_at | RAD23B | RAD23 homolog B (S. cerevisiae) | BG571600 | 0.010584 |
| 11723291_a_at | NDUFA1 | NADH dehydrogenase (ubiquinone) 1 alpha subcomplex, 1, 7.5kDa | NM_004541.3 | 0.010596 |
| 11715771_a_at | MRPL13 | mitochondrial ribosomal protein L13 | NTM_014078.4 | 0.010834 |
| 11746174_s_at | IDH1 | isocitrate dehydrogenase 1 (NADP+), soluble | BC012846.1 | 0.011295 |
| 11724432_x_at | TRAPPC2 | trafficking protein particle complex 2 | NM_001011658.2 | 0.01144 |
| 11746489_x_at | GPAA1 | glycosylphosphatidylinositol anchor attachment protein 1 homolog (yeast) | BC006383.2 | 0.011462 |
| 11724120_a_at | TRIM59 | tripartite motif-containing 59 | NM_173084.2 | 0.01188 |
| 11764061_s_at | NDUFB3 | NADH dehydrogenase (ubiquinone) 1 beta subcomplex, 3, 12kDa | AA887183 | 0.012144 |
| 11758083_s_at | HPGD | hydroxyprostaglandin dehydrogenase 15-(NAD) | AI743714 | 0.012171 |
| 11729715_a_at | CBR1 | carbonyl reductase 1 | NM_001757.2 | 0.012312 |
| 11734864_x_at | SARNP | SAP domain containing ribonucleoprotein | NM_033082.3 | 0.012428 |
| 11717314_a_at | HAUS1 | HAUS augmin-like complex, subunit 1 | NM_138443.3 | 0.012462 |
| 11751523_a_at | TMED5 | transmembrane emp24 protein transport domain containing 5 | AK293308.1 | 0.012569 |
| 11754800_s_at | GFM1 | G elongation factor, mitochondrial 1 | AK092293.1 | 0.012699 |
| 11746042_s_at | TRA2B | transformer 2 beta homolog (Drosophila) | AK098191.1 | 0.01285 |
| 11736741_a_at | MKI67 | antigen identified by monoclonal antibody Ki-67 | NM_001145966.1 | 0.012861 |
| 11729333_at | PADI1 | peptidyl arginine deiminase, type I | NM_013358.2 | 0.013091 |
| 11751291_a_at | SFXN4 | sideroflexin 4 | AY269785.1 | 0.013333 |
| 11717991_a_at | SIDT2 | SID1 transmembrane family, member 2 | NM_001040455.1 | 0.013344 |
| 11748896_s_at | CCRL1 | chemokine (C-C motif) receptor-like 1 | AK304461.1 | 0.013521 |
| 11716395_a_at | GPR56 | G protein-coupled receptor 56 | NM_001145774.1 | 0.013559 |
| 11729716_s_at | CBR1 | carbonyl reductase 1 | NM_001757.2 | 0.01399 |
| 11716063_at | TNC | tenascin C | NM_002160.2 | 0.014267 |
| 11758011_x_at | EEF1A1 | eukaryotic translation elongation factor 1 alpha 1 | BI495952 | 0.014362 |
| 11720317_a_at | DAD1 | defender against cell death 1 | NM_001344.2 | 0.014754 |
| 11720186_s_at | MAD2L1 | MAD2 mitotic arrest deficient-like 1 (yeast) | NM_002358.3 | 0.014969 |
| 11725960_s_at | CALM3 | calmodulin 3 (phosphorylase kinase, delta) | NM_005184.2 | 0.015262 |
| 11730753_at | AGPAT5 | 1-acylglycerol-3-phosphate O-acyltransferase 5 (lysophosphatidic acid acyltransferase, epsilon) | NM_018361.3 | 0.015361 |
| 11735839_at | STX19 | syntaxin 19 | NM_001001850.1 | 0.015426 |
| 11746655_a_at | ACAA1 | acetyl-CoA acyltransferase 1 | AK303251.1 | 0.015895 |
| 11744002_s_at | MTHFD2 | methylenetetrahydrofolate dehydrogenase (NADP+ dependent) 2, methenyltetrahydrofolate cyclohydrolase | BG026531 | 0.015966 |
| 11721296_a_at | NDUFB1 | NADH dehydrogenase (ubiquinone) 1 beta subcomplex, 1, 7kDa | NM_004545.3 | 0.016187 |
| 11725125_a_at | NEB | nebulin | NM_004543.3 | 0.016335 |
| 11716624_s_at | XPO1 | exportin 1 (CRM1 homolog, yeast) | NM_003400.3 | 0.016341 |
| 11759922_a_at | PARD3 | par-3 partitioning defective 3 homolog (C. elegans) | BC071566.1 | 0.016372 |

**Table 11 - Type III Dermis; Up-regulated**

| GeneTitan_ID | Gene | Title | Public ID | p |
|---|---|---|---|---|
| 11733167_at | LRRN4CL | LRRN4 C-terminal like | BC053902.1 | 0.000101 |
| 11716549_s_at | ISLR | immunoglobulin superfamily containing leucine-rich repeat | NM_005545.3 | 0.00021 |
| 11743191_a_at | NTM | neurotrimin | AI343272 | 0.000486 |
| 11725753_a_at | GRIA3 | glutamate receptor, ionotrophic, AMPA 3 | U10301.1 | 0.000741 |
| 11741377_a_at | MMP2 | matrix metallopeptidase 2 (gelatinase A, 72kDa gelatinase, 72kDa type IV collagenase) | NM_001127891.1 | 0.001476 |
| 11717765_a_at | MGLL | monoglyceride lipase | NM_007283.5 | 0.001622 |
| 11721372_at | TCF7L1 | transcription factor 7-like 1 (T-cell specific, HMG-box) | NM_031283.1 | 0.001646 |
| 11722839_at | LYAR | Ly1 antibody reactive homolog (mouse) | AW958593 | 0.001955 |
| 11762135_at | PTPRK | protein tyrosine phosphatase, receptor type, K | BC063596.1 | 0.002158 |
| 11721467_s_at | CD276 | CD276 molecule | NM_001024736.1 | 0.002325 |
| 11761134_at | MYST3 | MYST histone acetyltransferase (monocytic leukemia) 3 | BC142959.1 | 0.003233 |
| 11720440_at | OLFML2B | olfactomedin-like 2B | NM_015441.1 | 0.003454 |
| 1174543I_a_at | SVIL | supervillin | BC092440.1 | 0.003484 |
| 11739746_s_at | SVIL | supervillin | CD366976 | 0.003636 |
| 11757808_s_at | RERE | arginine-glutamic acid dipeptide (RE) repeats | BM706668 | 0.003739 |
| 11725937_a_at | LGALS3 | lectin, galactoside-binding, soluble, 3 | BC053667.1 | 0.003825 |
| 11720274_x_at | ALKBH6 | alkB, alkylation repair homolog 6 (E. coli) | NM_032878.3 | 0.003968 |
| 11755955_a_at | FAP | fibroblast activation protein, alpha | AL832166.1 | 0.003989 |
| 11724619_at | RSPO3 | RSPO3 laevis) R-spondin 3 homolog (Xenopus laevis) | NM_032784.3 | 0.004121 |
| 11729170_x_at | DUSP10 | dual specificity phosphatase 10 | AF179212.1 | 0.004193 |
| 11752038_a_at | AQPEP | laeverin | BC068560.1 | 0.004865 |
| 11720846_at | CD248 | CD248 molecule, endosialin | NM_020404.2 | 0.005206 |
| 11731143_a_at | GPR133 | G protein-coupled receptor 133 | NM_198827.3 | 0.005394 |
| 11728451_a_at | PCOLCE2 | procollagen C-endopeptidase enhancer 2 | NM_013363.2 | 0.005523 |
| 11731682_at | CD70 | CD70 molecule | NM_001252.3 | 0.005777 |
| 11716226_a_at | LIMA1 | LIM domain and actin binding 1 | BC136763.1 | 0.006288 |
| 11750244_a_at | MGLL | monoglyceride lipase | AK304844.1 | 0.00649 |
| 11762370_x_at | BNC1 | basonuclin 1 | L03427.1 | 0.006531 |
| 11729101_a_at | AKR1C2 | aldo-keto reductase family 1, member C2 (dihydrodiol dehydrogenase 2; bile acid binding protein; 3-alpha hydroxysteroid dehydrogenase, type III) | NM_205845.1 | 0.006587 |
| 11731649_x_at | NTM | neurotrimin | AY358331.1 | 0.006934 |
| 11716238_at | ARHGAP1 | Rho GTPase activating protein 1 | NM_004308.2 | 0.006953 |
| 11728498_a_at | SVIL | supervillin | NM_003174.3 | 0.00696 |
| 11725517_x_at | ABCG1 | ATP-binding cassette, sub-family G (WHITE), member 1 | NM_207627.1 | 0.007347 |
| 11728605_s_at | LIMS1 | LIM and senescent cell antigen-like domains 1 | NM_033514.2 | 0.007353 |
| 11752843_x_at | SQSTM1 | sequestosome 1 | AK304877.1 | 0.007432 |
| 11757557_s_at | CADM1 | cell adhesion molecule 1 | H23245 | 0.007475 |
| 11718269_x_at | ANGPTL2 | angiopoietin-like 2 | AY358274.1 | 0.007782 |
| 11747944_a_at | PPFIA2 | protein tyrosine phosphatase, receptor type, f polypeptide (PTPRF), interacting protein (liprin), alpha 2 | AK296380.1 | 0.008378 |
| 11761149_a_at | C5orf45 | chromosome 5 open reading frame 45 | AK293901.1 | 0.008409 |
| 11737357_a_at | CNGA3 | cyclic nucleotide gated channel alpha 3 | NM_001298.2 | 0.008783 |
| 11743250_a_at | MMP2 | matrix metallopeptidase 2 (gelatinase A, 72kDa gelatinase, 72kDa type IV collagenase) | BX357054 | 0.009096 |
| 11725515_a_at | ABCG1 | ATP-binding cassette, sub-family G (WHITE), member 1 | NM_207627.1 | 0.009131 |
| 11759362_x_at | PHKG1 | phosphorylase kinase, gamma 1 (muscle) | BC051327.1 | 0.009194 |
| 11717802_s_at | ATF5 | activating transcription factor 5 | BE300055 | 0.009354 |
| 11723070_a_at | CYTL1 | cytokine-like 1 | NM_018659.2 | 0.009527 |
| 11731650_a_at | NTM | neurotrimin | NM_001048209.1 | 0.009599 |
| 11720845_a_at | CD248 | CD248 molecule, endosialin | NM_020404.2 | 0.009838 |
| 11716322_s_at | PRKCDBP | protein kinase C, delta binding protein | NM_145040.2 | 0.009887 |
| 11718658_s_at | CD34 | CD34 molecule | NM_001773.2 | 0.010659 |
| 11747945_x_at | PPFIA2 | protein tyrosine phosphatase, receptor type, f polypeptide (PTPRF), interacting protein (liprin), alpha 2 | AK296380.1 | 0.010696 |
| 11717764_x_at | MGLL | monoglyceride lipase | BC006230.2 | 0.010744 |
| 11743251_s_at | MMP2 | matrix metallopeptidase 2 (gelatinase A, 72kDa gelatinase, 72kDa type IV collagenase) | BX357054 | 0.010854 |
| 11731303_a_at | DUSP10 | dual specificity phosphatase 10 | BC020608.1 | 0.010964 |
| 11719737_a_at | FAM134B | family with sequence similarity 134, member B | BC053326.1 | 0.011265 |
| 11758143_s_at | DUSP8 | dual specificity phosphatase 8 | BE350906 | 0.011421 |
| 11724441_x_at | PTGIS | prostaglandin I2 (prostacyclin) synthase | NM_000961.3 | 0.011582 |
| 11737583_s_at | SGCD | sarcoglycan, delta (35kDa dystrophin-associated glycoprotein) | NM_001128209.1 | 0.01169 |
| 11729541_a_at | CAMKK2 | calcium/calmodulin-dependent protein kinase kinase 2, beta | AB081337.1 | 0.011881 |
| 11737108_a_at | CCRL1 | chemokine (C-C motif) receptor-like 1 | NM_178445.1 | 0.012 |
| 11721507_at | DVL3 | dishevelled, dsh homolog 3 (Drosophila) | NM_004423.3 | 0.012974 |
| 11750245_x_at | MGLL | monoglyceride lipase | AK304844.1 | 0.013107 |
| 11737946_a_at | XPNPEP2 | X-prolyl aminopeptidase (aminopeptidase P) 2, membrane-bound | BC143901.1 | 0.01347 |
| 11727155_a_at | TRIOBP | TRIO and F-actin binding protein | NM_007032.5 | 0.013526 |
| 1172044l_x_at | OLFML2B | olfactomedin-like 2B | NM_01544101 | 0.01374 |
| 11727773_at | LARP6 | La ribonucleoprotein domain family, member 6 | NM_197958.1 | 0.013995 |
| 11728499_x_at | SVIL | supervillin | NM_003174.3 | 0.01402 |
| 11745659_s_at | POM121 | POM121 membrane glycoprotein | BC130587.1 | 0.014097 |
| 11752562_x_at | CDH13 | cadherin 13, H-cadherin (heart) | AK294277.1 | 0.014197 |
| 11720617_at | TRIM9 | tripartite motif-containing 9 | NM_015163.5 | 0.014562 |
| 11757548_s_at | ADAMTSL1 | ADAMTS-like 1 | DB329733 | 0.014859 |
| 11753179_s_at | FAM134B | family with sequence similarity 134, member B | BC030517.1 | 0.014991 |
| 11729285_a_at | NFU1 | NFU1 iron-sulfur cluster scaffold homolog (S. cerevisiae) | NM_001002755.1 | 0.01519 |
| 11741286_a_at | CCRL1 | chemokine (C-C motif) receptor-like 1 | AF110640.1 | 0.015787 |
| 11732315_a_at | SGCD | sarcoglycan, delta (35kDa dystrophin-associated glycoprotein) | AF010236.1 | 0.015795 |
| 11715852_at | PDGFRB | platelet-derived growth factor receptor, beta polypeptide | NM_002609.3 | 0.016136 |
| 11730404_at | MEX3B | mex-3 homolog B (C. elegans) | NM_032246.3 | 0.0163 |
| 11751986_at | MMP19 | matrix metallopeptidase 19 | U38320.1 | 0.016486 |
| 11731122_a_at | VASH2 | vasohibin 2 | BC051856.1 | 0.016505 |
| 11732785_a_at | C16orf45 | chromosome 16 open reading frame 45 | NM_001142469.1 | 0.017241 |
| 11757765_s_at | SGCD | sarcoglycan, delta (35kDa dystrophin-associated glycoprotein) | AA401248 | 0.017347 |
| 11743143_at | COX11 | COX11 cytochrome c oxidase assembly homolog (yeast) | AK293851.1 | 0.017504 |
| 11724142_s_at | RAB11FIP2 | RAB11 family interacting protein 2 (class I) | DB356544 | 0.017868 |
| 11723075_a_at | BCL9L | B-cell CLL/lymphoma 9-like | AY296059.1 | 0.017989 |
| 11747704_a_at | CLDN11 | claudin 11 | AK294087.1 | 0.017998 |
| 11716376_at | SERPINA5 | serpin peptidase inhibitor, clade A (alpha-1 antiproteinase, antitrypsin), member 5 | NM_000624.4 | 0.018046 |
| 11756879_a_at | STARD9 | StAR-related lipid transfer (START) domain containing 9 | CR936665.1 | 0.018874 |
| 11733166_at | LRRN4CL | LRRN4 C-terminal like | NM_203422.1 | 0.018933 |
| 11720163_at | VEGFC | vascular endothelial growth factor C | NM_005429.2 | 0.018951 |
| 11754821_s_at | SLC38A1 | solute carrier family 38, member 1 | AI476037 | 0.019062 |
| 11720082_at | CBX6 | chromobox homolog 6 | NM_014292.3 | 0.020169 |
| 11762231_x_at | BBS1 | Bardet-Biedl syndrome 1 | AK294962.1 | 0.020213 |
| 11732462_at | ADAMTSL1 | ADAMTS-like 1 | AK123028.1 | 0.020317 |
| 11761563_x_at | HEATR1 | HEAT repeat containing 1 | BC062442.1 | 0.0204 |
| 11727714_at | KCNJ12 | potassium inwardly-rectifying channel, subfamily J, member 12 | NM_021012.4 | 0.020553 |
| 11727780_a_at | SCARA5 | scavenger receptor class A, member 5 (putative) | NM_173833.4 | 0.020636 |
| 11749436_a_at | NFIC | nuclear factor I/C (CCAAT-binding transcription factor) | AK297825.1 | 0.020877 |
| 11731209_s_at | C15orf59 | chromosome 15 open reading frame 59 | NM_001039614.1 | 0.021422 |
| 11727125_a_at | PVRL3 | poliovirus receptor-related 3 | BE544927 | 0.021561 |
| 11744741_at | LOH3CR2A | loss of heterozygosity, 3, chromosomal region 2, gene A | AF086709.2 | 0.021592 |
| 11717891_a_at | ECM1 | extracellular matrix protein 1 | BC023505.2 | 0.021868 |

**Table 12 - Type III Dermis; Down-regulated**

| GeneTitan_ID | Gene | Title | Public ID | p |
|---|---|---|---|---|
| 11727158_a_at | STRBP | spermatid perinuclear RNA binding protein | NM_018387.3 | 0.000131 |
| 11756850_x_at | CCT8 | chaperonin containing TCP1, subunit 8 (theta) | CR612497.1 | 0.000172 |
| 11754000_x_at | CD58 | CD58 molecule | CR456939.1 | 0.000222 |
| 11737761_a_at | HSD17B4 | hydroxysteroid (17-beta) dehydrogenase 4 | NM_000414.2 | 0.000267 |
| 11754276_a_at | RAD23B | RAD23 homolog B (S. cerevisiae) | BG501496 | 0.000343 |
| 11743094_at | SPRR4 | small proline-rich protein 4 | BC069445.1 | 0.000362 |
| 11724156_at | SULT1E1 | sulfotransferase family 1E, estrogen-preferring, member 1 | NM_005420.2 | 0.000395 |
| 11749267_a_at | SRD5A1 | steroid-5-alpha-reductase, alpha polypeptide 1 (3-oxo-5 alpha-steroid delta 4-dehydrogenase alpha 1) | AK315996.1 | 0.000399 |
| 11720183_s_at | EEF1B2 | eukaryotic translation elongation factor 1 beta 2 | NM_001959.3 | 0.000428 |
| 11737053_s_at | HSPD1 | heat shock 60kDa protein 1 (chaperonin) | NM_002156.4 | 0.000467 |
| 11740377_a_at | PXMP4 | peroxisomal membrane protein 4, 24kDa | AK297018.1 | 0.000581 |
| 11726318_s_at | EEF1G | eukaryotic translation elongation factor 1 gamma | NM_001404.4 | 0.000583 |
| 11720184_x_at | EEF1B2 | eukaryotic translation elongation factor 1 beta 2 | NM_001959.3 | 0.000595 |
| 11746149_x_at | BCHE | butyrylcholinesterase | M16474.1 | 0.000611 |
| 11737762_x_at | HSD17B4 | hydroxysteroid (17-beta) dehydrogenase 4 | NM_000414.2 | 0.000646 |
| 11739725_a_at | TC2N | tandem C2 domains, nuclear | NM_001128595.1 | 0.000648 |
| 11754918_s_at | HMGCS1 | 3-hydroxy-3-methylglutaryl-CoA synthase 1 (soluble) | AK095492.1 | 0.000648 |
| 11741799_a_at | BCOR | BCL6 corepressor | AF317391.1 | 0.000649 |
| 11736831_a_at | SEC23B | Sec23 homolog B (S. cerevisiae) | NM_032986.3 | 0.000656 |
| 11744777_s_at | DPY30 | dpy-30 homolog (C. elegans) | BC015970.1 | 0.000676 |
| 11754418_s_at | G3BP1 | GTPase activating protein (SH3 domain) binding protein 1 | AK130003.1 | 0.000682 |
| 11723250_a_at | EML2 | echinoderm microtubule associated protein like 2 | NM_012155.1 | 0.00072 |
| 11752369_a_at | IMPDH2 | IMP (inosine 5'-monophosphate) dehydrogenase 2 | AK293397.1 | 0.000726 |
| 11729643_s_at | TPD52 | tumor protein D52 | CB219128 | 0.000732 |
| 11755057_s_at | ATP2C1 | ATPase, Ca++ transporting, type 2C, member 1 | AB037768.1 | 0.000739 |
| 11716946_s_at | TM9SF3 | transmembrane 9 superfamily member 3 | AF269150.1 | 0.000773 |
| 11756300_a_at | ANP32B | acidic (leucine-rich) nuclear phosphoprotein 32 family, member B | BX432546 | 0.000781 |
| 11716134_a_at | MTOR | mechanistic target of rapamycin (serine/threonine kinase) | NM_004958.3 | 0.000785 |
| 11755203_x_at | RPL21 | ribosomal protein L21 | BX647669.1 | 0.000869 |
| 11730938_x_at | PYCR1 | pyrroline-5-carboxylate reductase 1 | NM_153824.1 | 0.000877 |
| 11750545_a_at | CNOT7 | CCR4-NOT transcription complex, subunit 7 | BC007315.2 | 0.000884 |
| 11727826_a_at | C2orf56 | chromosome 2 open reading frame 56 | BC004548.2 | 0.00093 |
| 11718344_a_at | CNOT7 | CCR4-NOT transcription complex, subunit 7 | NM_013354.5 | 0.000947 |
| 11756600_a_at | TPD52 | tumor protein D52 | AK308983.1 | 0.000999 |
| 11734619_x_at | ALOX15B | arachidonate 15-lipoxygenase, type B | NM_001141.2 | 0.001036 |
| 11715621_at | UFC1 | ubiquitin-fold modifier conjugating enzyme 1 | NM_016406.3 | 0.001145 |
| 11715958_s_at | RPL7 | ribosomal protein L7 | NM_000971.3 | 0.001233 |
| 11748713_a_at | ASPM | asp (abnormal spindle) homolog, microcephaly associated (Drosophila) | AY971957.1 | 0.001256 |
| 11758707_s_at | C5orf25 | chromosome 5 open reading frame 25 | DB526316 | 0.001417 |
| 200081_PM_s_at | RPS6 | ribosomal protein S6 | BE741754 | 0.00144 |
| 11726299_x_at | LGALS8 | lectin, galactoside-binding, soluble, 8 | AF342815.1 | 0.001452 |
| 11756210_a_at | RCL1 | RNA terminal phosphate cyclase-like 1 | AL582781 | 0.001477 |
| 11743604_s_at | RRM1 | ribonucleotide reductase M1 | B618815 | 0.001487 |
| 11729641_a_at | TPD52 | tumor protein D52 | BG389015 | 0.001493 |
| 11718461_at | SLC39A11 | solute carrier family 39 (metal ion transporter), member 11 | NM_139177.3 | 0.001532 |
| 11725053_x_at | TOP1MT | topoisomerase (DNA) I, mitochondrial | NM_052963.1 | 0.001534 |
| 11758027_s_at | HOOK1 | hook homolog 1 (Drosophila) | C243255 | 0.001606 |
| 11745205_s_at | TPD52 | tumor protein D52 | BC018117.1 | 0.001623 |
| 11760342_a_at | PPP3CB | protein phosphatase 3, catalytic subunit, beta isozyme | M29550.1 | 0.001681 |
| 11725875_at | WDR66 | WD repeat domain 66 | NM_144668.4 | 0.001791 |
| 11739308_s_at | DLG1 | discs, large homolog 1 (Drosophila) | BM681931 | 0.001815 |
| 11719666_a_at | STMN1 | stathmin 1 | BC082228.1 | 0.001852 |
| 11752283_a_at | ALOX15B | arachidonate 15-lipoxygenase, type B | AK298095.1 | 0.001904 |
| 11723312_a_at | PXMP2 | peroxisomal membrane protein 2, 22kDa | NM_018663.1 | 0.001916 |
| 11719667_s_at | STMN1 | stathmin 1 | BC082228.1 | 0.001961 |
| 11728791_at | THRSP | thyroid hormone responsive | NM_003251.2 | 0.001966 |
| 11734917_a_at | METTL4 | methyltransferase like 4 | BQ009802 | 0.001983 |
| 11717236_x_at | RPS7 | ribosomal protein S7 | NM_001011.3 | 0.002022 |
| 11754132_x_at | COMT | catechol-O-methyltransferase | BT007125.1 | 0.002101 |
| 11743372_s_at | PTGES3 | prostaglandin E synthase 3 (cytosolic) | CR611609.1 | 0.002174 |
| 11730411_a_at | PXMP4 | peroxisomal membrane protein 4, 24kDa | BF057649 | 0.002209 |
| 200063_PM_s_at | NPM1 | nucleophosmin (nucleolar phosphoprotein B23, numatrin) | BC002398.1 | 0.002233 |
| 11722642_a_at | DGAT2 | diacylglycerol O-acyltransferase 2 | BC015234.1 | 0.002305 |
| 11752550_x_at | CCT8 | chaperonin containing TCP1, subunit 8 (theta) | AK293705.1 | 0.002336 |
| 11758217_s_at | FAM108C1 | family with sequence similarity 108, member C1 | C997200 | 0.002358 |
| 11717182_a_at | PDS5A | PDS5, regulator of cohesion maintenance, homolog A (S. cerevisiae) | NM_001100399.1 | 0.002387 |
| 11717153_a_at | C20orf3 | chromosome 20 open reading frame 3 | NM_020531.2 | 0.002394 |
| 11742779_a_at | HIBCH | 3-hydroxyisobutyryl-CoA hydrolase | U66669.1 | 0.002431 |
| 11744264_a_at | SEC11C | SEC11 homolog C (S. cerevisiae) | AI816180 | 0.002433 |
| 11753788_x_at | CDKN3 | cyclin-dependent kinase inhibitor 3 | AF213040.1 | 0.002434 |
| 11758709_s_at | RDH11 | retinol dehydrogenase 11 (all-trans/9-cis/11-cis) | AI972157 | 0.002449 |
| 11727320_at | IGFL2 | IGF-like family member 2 | NM_001002915.2 | 0.002476 |
| 11730803_a_at | PRPF38B | PRP38 pre-mRNA processing factor 38 (yeast) domain containing B | NM_018061.2 | 0.002515 |
| 11753740_x_at | CYB5A | cytochrome b5 type A (microsomal) | CR456990.1 | 0.002572 |
| 11718246_a_at | KIAA0146 | KIAA0146 | NM_001080394.1 | 0.002609 |
| 11720768_at | METTL9 | methyltransferase like 9 | NM_016025.3 | 0.002613 |
| 11755017_a_at | CHCHD7 | coiled-coil-helix-coiled-coil-helix domain containing 7 | AK098285.1 | 0.002843 |
| 11732128_s_at | CCT4 | chaperonin containing TCP1, subunit 4 (delta) | BC106934.1 | 0.002855 |
| 11744900_x_at | FADS2 | fatty acid desaturase 2 | AF108658.1 | 0.002865 |
| 11715881_a_at | DAP3 | death associated protein 3 | NM_004632.2 | 0.002925 |
| 11756875_x_at | COMMD6 | COMM domain containing 6 | CR603325.1 | 0.002962 |
| 11756783_a_at | TF | transferrin | BC045772.1 | 0.002967 |
| 11723197_at | HNRNPA3 | heterogeneous nuclear ribonucleoprotein A3 | BX434302 | 0.003022 |
| 11729941_at | TMEM56 | transmembrane protein 56 | NM_152487.2 | 0.003035 |
| 11716813_a_at | GATM | glycine amidinotransferase (L-arginine:glycine amidinotransferase) | AK298350.1 | 0.003053 |
| 11721242_s_at | FDFT1 | farnesyl-diphosphate farnesyltransferase 1 | NM_004462.3 | 0.003125 |
| 11749786_x_at | HNRNPF | heterogeneous nuclear ribonucleoprotein F | AK296696.1 | 0.003153 |
| 11723313_s_at | PXMP2 | peroxisomal membrane protein 2, 22kDa | NM_018663.1 | 0.003162 |
| 11727286_a_at | ZNF323 | zinc finger protein 323 | NM_001135215.1 | 0.003185 |
| 11720813_at | INTS10 | integrator complex subunit 10 | NM_018142.2 | 0.003216 |
| 11749874_a_at | OXCT1 | 3-oxoacid CoA transferase 1 | AK299668.1 | 0.003244 |
| 11757320_x_at | CYB5A | cytochrome b5 type A (microsomal) | AA706740 | 0.003263 |
| 11733591_a_at | C1orf204 | chromosome 1 open reading frame 204 | NM_001134233.1 | 0.003297 |
| 11718135_at | PRPS2 | phosphoribosyl pyrophosphate synthetase 2 | NM_001039091.1 | 0.003316 |
| 11716302_s_at | ACSL1 | acyl-CoA synthetase long-chain family member 1 | NM_001995.2 | 0.003359 |
| 11744392_a_at | PAPOLA | poly(A) polymerase alpha | BC000927.1 | 0.003388 |
| 11741012_a_at | SC4MOL | sterol-C4-methyl oxidase-like | AK292418.1 | 0.00342 |
| 11722800_a_at | SS18 | synovial sarcoma translocation, chromosome 18 | CB241009 | 0.003458 |
| 11755439_x_at | UBAC2 | UBA domain containing 2 | BC053346.1 | 0.003608 |
| 11756674_s_at | STRBP | spermatid perinuclear RNA binding protein | CR596677.1 | 0.003641 |

### Example 2 - Theme mapping.

This example illustrates mapping a gene expression signature onto a biological process grid or Gene Ontology, to yield a physiological theme pattern. Table 13 below shows Gene Ontology Biological Process terms that are significantly enriched in the epidermis of subjects with Type I periorbital dyschromia. The results in Table 13 were generated from differentially expressed genes in those subjects with top ranked genes shown in Tables 1 and 2. Only the most highly significant themes are shown (p ≤ 1 x 10⁻² to p, 1 x 10⁻⁵, and the theme analysis was done separately for the up- and down-regulated genes. The level of indentation in the terms column (i.e., the number of dots preceding the term) generally indicates the level in the GO hierarchy and parent/child relationships between terms.

**Table 13**

| Gene Ontology Biological Process Terms | Type I Epidermis vs. No Dyschromia | | |
|---|---|---|---|
| | Up | Down | Directional |
| ....GO:0002764 immune response-regulating signaling pathway | | ** | |
| .......GO:0002758 innate immune response-activating signal transduction | | *** | |
| ........GO:0002224 toll-like receptor signaling pathway | | ** | |
| ...GO:0048468 cell development | ** | | |
| .GO:0002376 immune system process | | **** | |
| ..GO:0002252 immune effector process | | **** | |
| ..GO:0002253 activation of immune response | | **** | |
| ...GO:0002218 activation of innate immune response | | *** | |
| ..GO:0006955 immune response | | **** | |
| ...GO:0045087 innate immune response | | **** | |
| ......GO:0071383 cellular response to steroid hormone stimulus | ** | | |
| ....GO:0006954 inflammatory response | | * | |
| ........GO:0045047 protein targeting to ER | | **** | |
| ....GO:0032984 macromolecular complex disassembly | | **** | |
| ......GO:0016568 chromatin modification | * | | |
| ....GO:0050776 regulation of immune response | | **** | |
| ......GO:0006749 glutathione metabolic process | | ** | |
| .....GO:0019752 carboxylic acid metabolic process | | * | |
| ..GO:0007154 cell communication | * | | |
| ....GO:0016042 lipid catabolic process | | * | |
| ......GO:0070588 calcium ion transmembrane transport | * | | |

| | | | |
|---|---|---|---|
| * p-value between 1x10⁻² and 1x10⁻³ ** p-value between 1x10⁻³ and 1x10⁻⁴ *** p-value between 1x10⁻⁴ and 1x10⁻⁵ ****p-value less than 1x10⁻⁵ | | | |

Table 14 below shows Gene Ontology Biological Process terms that are significantly enriched in the epidermis of subjects with Type II periorbital dyschromia. The results in Table 14 were generated from differentially expressed genes in those subjects with top ranked genes shown in Tables 5 and 6. Only the most highly significant themes are shown (p ≤ 1 X 10⁻²), and the theme analysis was done separately for the up- and down-regulated genes.

**Table 14**

| Gene Ontology Biological Process Terms | Type II Epidermis vs. No Dyschromia | | |
|---|---|---|---|
| | Up | Down | Directional |
| .....GO:0042770 signal transduction in response to DNA damage | | **** | |
| .....GO:0072331 signal transduction by p53 class mediator | | **** | |
| ........GO:0007173 epidermal growth factor receptor signaling pathway | | ** | |
| .....GO:0001942 hair follicle development | | **** | |
| ......GO:0008544 epidermis development | | **** | |
| ....GO:0045444 fat cell differentiation | * | | |
| ..GO:0019882 antigen processing and presentation | | **** | |
| ....GO:0071453 cellular response to oxygen levels | | | ** |
| ......GO:0071456 cellular response to hypoxia | | | ** |
| ......GO:0031960 response to corticosteroid stimulus | * | | |
| ...GO:0033554 cellular response to stress | | **** | |
| .....GO:0006281 DNA repair | | ** | |
| .....GO:0000077 DNA damage checkpoint | | **** | |
| ..GO:0006950 response to stress | | ** | |
| ...GO:0009268 response to pH | | ** | |
| .....GO:0006886 intracellular protein transport | | **** | |
| ......GO:0006605 protein targeting | | **** | |
| .....GO:0015031 protein transport | | **** | |
| ....GO:0046907 intracellular transport | | **** | |
| .....GO:0048193 Golgi vesicle transport | | ** | |
| .....GO:0006839 mitochondrial transport | | **** | |
| .......GO:0015986 ATP synthesis coupled proton transport | | *** | |
| ......GO:0016568 chromatin modification | ** | | |
| .....GO:0031326 regulation of cellular biosynthetic process | ** | | |
| ......GO:0006521 regulation of cellular amino acid metabolic process | | **** | |
| .....GO:0010565 regulation of cellular ketone metabolic process | | **** | |
| ....GO:0051171 regulation of nitrogen compound metabolic process | | | |
| ....GO:0051726 regulation of cell cycle | | ** | |
| ....GO:0045454 cell redox homeostasis | | ** | |
| .....GO:0032652 regulation of interleukin-1 production | ** | | |
| ...GO:0035383 thioester metabolic process | | **** | |
| ....GO:0006637 acyl-CoA metabolic process | | **** | |
| .......GO:0006099 tricarboxylic acid cycle | | **** | |
| ......GO:0006635 fatty acid beta-oxidation | | ** | |
| ........GO:0018108 peptidyl-tyrosine phosphorylation | ** | | |
| ...GO:0006082 organic acid metabolic process | | **** | |
| ......GO:0006520 cellular amino acid metabolic process | | ** | |
| .......GO:0043038 amino acid activation | | **** | |
| ...GO:0006091 generation of precursor metabolites and energy | | **** | |
| ....GO:0006096 glycolysis | | ** | |
| ....GO:0006119 oxidative phosphorylation | | **** | |
| .....GO:0042773 ATP synthesis coupled electron transport | | **** | |
| .....GO:0045333 cellular respiration | | **** | |
| ..GO:0007049 cell cycle | | **** | |
| ...GO:0005975 carbohydrate metabolic process | | *** | |
| ........GO:0006007 glucose catabolic process | | *** | |
| ....GO:0016052 carbohydrate catabolic process | | **** | |
| ...GO:0055114 oxidation-reduction process | | **** | |

Table 15 shows Gene Ontology Biological Process terms that are significantly enriched in the epidermis of subjects with Type III periorbital dyschromia. The results in Table 15 were generated from differentially expressed genes in those subjects with top ranked genes shown in Tables 9 and 10. Only the most highly significant themes are shown (p ≤ 1 X 10⁻²), and the theme analysis was done separately for the up- and down-regulated genes.

**Table 15**

| Gene Ontology Biological Process Terms | Type III Epidermis vs. No Dyschromia | | |
|---|---|---|---|
| | Up | Down | Directional |
| ....GO:0048731 system development | ** | | |
| ..GO:0048856 anatomical structure development | *** | | |
| ...GO:0030154 cell differentiation | ** | | |
| ...GO:0070482 response to oxygen levels | * | | |
| .....GO:0033365 protein localization to organelle | | ** | |
| ........GO:0045047 protein targeting to ER | | ** | |
| ......GO:0006605 protein targeting | | ** | |
| .......GO:0006612 protein targeting to membrane | | ** | |
| .....GO:0006839 mitochondrial transport | | ** | |
| ........GO:0001934 positive regulation of protein phosphorylation | ** | | |
| ......GO:0051347 positive regulation of transferase activity | ** | | |
| .....GO:0010627 regulation of intracellular protein kinase cascade | ** | | |
| .....GO:0080135 regulation of cellular response to stress | * | | |
| ....GO:0051320 S phase | | *** | |
| ......GO:0006414 translational elongation | | **** | |
| ...GO:0006091 generation of precursor metabolites and energy | | *** | |
| ....GO:0006119 oxidative phosphorylation | | ** | |
| ....GO:0022900 electron transport chain | | **** | |
| .....GO:0045333 cellular respiration | | **** | |
| ...GO:0055114 oxidation-reduction process | | *** | |

Table 16 shows Gene Ontology Biological Process terms that are significantly enriched in the dermis of subjects with Type I periorbital dyschromia. The results in Table 16 were generated from differentially expressed genes in those subjects with top ranked genes shown in Tables 3 and 4. Only the most highly significant themes are shown (p ≤ 1 X 10⁻²), and the theme analysis was done separately for the up- and down-regulated genes.

**Table 16**

| Gene Ontology Biological Process Terms | Type I Dermis vs No Dyschromia | | |
|---|---|---|---|
| | Up | Down | Directional |
| ......GO:0000165 MAPK cascade | ** | | |
| .......GO:0048011 nerve growth factor receptor signaling pathway | * | | |
| .......GO:0007179 transforming growth factor beta receptor signaling pathway | * | | |
| ....GO:0050877 neurological system process | *** | | |
| ....GO:0007596 blood coagulation | * | | |
| ...GO:0050878 regulation of body fluid levels | * | | |
| .....GO:0001944 vasculature development | **** | | |
| ......GO:0001568 blood vessel development | **** | | |
| .......GO:0048514 blood vessel morphogenesis | **** | | |
| ........GO:0001525 angiogenesis | **** | | |
| ..........GO:0030183 B cell differentiation | | | * |
| ......GO:0007399 nervous system development | **** | | |
| .......GO:0022008 neurogenesis | *** | | |
| .......GO:0048699 generation of neurons | *** | | |
| ........GO:0030182 neuron differentiation | ** | | |
| .........GO:0048666 neuron development | ** | | |
| ..GO:0048870 cell motility | ** | | |
| ......GO:0043627 response to estrogen stimulus | ** | | |
| .....GO:0006281 DNA repair | | **** | |
| ...GO:0001666 response to hypoxia | * | | |
| ...GO:0006979 response to oxidative stress | * | | |
| ...GO:0009611 response to wounding | *** | | |
| ...GO:0070482 response to oxygen levels | ** | | |
| .....GO:0033365 protein localization to organelle | | **** | |
| ........GO:0045047 protein targeting to ER | | **** | |
| ......GO:0006605 protein targeting | | **** | |
| .....GO:0015031 protein transport | | **** | |
| ....GO:0046907 intracellular transport | | **** | |
| ...GO:0034330 cell junction organization | ** | | |
| ....GO:0030198 extracellular matrix organization | * | | |
| ....GO:0007010 cytoskeleton organization | * | | |
| ....GO:0051276 chromosome organization | | *** | |
| .....GO:0032200 telomere organization | | ** | |
| .......GO:0006338 chromatin remodeling | | * | |
| ......GO:0043408 regulation of MAPK cascade | *** | | |
| .......GO:0051924 regulation of calcium ion transport | * | | |
| .....GO:0010827 regulation of glucose transport | | *** | |
| ......GO:0030334 regulation of cell migration | **** | | |
| .....GO:0010564 regulation of cell cycle process | | ** | |
| ....GO:0010646 regulation of cell communication | **** | | |
| ..GO:0022402 cell cycle process | | ** | |
| ...GO:0022403 cell cycle phase | | *** | |
| ..GO:0007049 cell cycle | | ** | |
| ...GO:0000278 mitotic cell cycle | | **** | |
| ..GO:0007154 cell communication | **** | | |
| ....GO:0008202 steroid metabolic process | * | | |
| .....GO:0006694 steroid biosynthetic process | | ** | |
| ......GO:0016126 sterol biosynthetic process | | **** | |
| .......GO:0006695 cholesterol biosynthetic process | | **** | |
| ...GO:0061061 muscle structure development | * | | |

Table 17 shows Gene Ontology Biological Process terms that are significantly enriched in the dermis of subjects with Type II periorbital dyschromia. The results in Table 17 were generated from differentially expressed genes in those subjects with top ranked genes shown in Tables 7 and 8. Only the most highly significant themes are shown (p ≤ 1 X 10⁻²), and the theme analysis was done separately for the up- and down-regulated genes.

**Table 17**

| Gene Ontology Biological Process Terms | Type II Dermis vs. No Dyschromia | | |
|---|---|---|---|
| | Up | Down | Directional |
| ....GO:0019226 transmission of nerve impulse | * | | |
| ....GO:0050877 neurological system process | ** | | |
| .....GO:0007399 nervous system development | ** | | |
| ......GO:0022008 neurogenesis | **** | | |
| .......GO:0048699 generation of neurons | *** | | |
| ........GO:0030182 neuron differentiation | *** | | |
| .........GO:0048666 neuron development | *** | | |
| ..........GO:0031175 neuron projection development | ** | | |
| ...........GO:0007409 axonogenesis | ** | | |
| ..........GO:0048667 cell morphogenesis involved in neuron differentiation | ** | | |
| ...GO:0048468 cell development | *** | | |
| .....GO:0006281 DNA repair | | **** | |
| .....GO:0033365 protein localization to organelle | | **** | |
| ......GO:0070972 protein localization to endoplasmic reticulum | | **** | |
| ......GO:0006605 protein targeting | | **** | |
| .......GO:0006612 protein targeting to membrane | | **** | |
| .....GO:0015031 protein transport | | **** | |
| ...GO:0006810 transport | | **** | |
| ..GO:0051641 cellular localization | | **** | |
| ..GO:0016043 cellular component organization | | **** | |
| ...GO:0022411 cellular component disassembly | | **** | |
| ....GO:0030198 extracellular matrix organization | * | | |
| ....GO:0032984 macromolecular complex disassembly | | **** | |
| ......GO:0043624 cellular protein complex disassembly | | **** | |
| ......GO:0071156 regulation of cell cycle arrest | | ** | |
| ....GO:0010646 regulation of cell communication | * | | |
| ....GO:0051320 S phase | | ** | |
| ...GO:0044248 cellular catabolic process | | **** | |
| .....GO:0006457 protein folding | | ** | |
| ..GO:0007049 cell cycle | | * | |
| ...GO:0000278 mitotic cell cycle | | ** | |
| ..GO:0007154 cell communication | * | | |
| .....GO:0006694 steroid biosynthetic process | | ** | |
| .......GO:0006695 cholesterol biosynthetic process | | *** | |

Table 18 shows Gene Ontology Biological Process terms that are significantly enriched in the epidermis of subjects with Type III periorbital dyschromia. The results in Table 18 were generated from differentially expressed genes in those subjects with top ranked genes shown in Tables 11 and 12. Only the most highly significant themes are shown (p ≤ 1 X 10⁻²), and the theme analysis was done separately for the up- and down-regulated genes.

**Table 18**

| Gene Ontology Biological Process Terms | Type III Dermis vs. No Dyschromia | | |
|---|---|---|---|
| | Up | Down | Directional |
| ..GO:0007155 cell adhesion | ** | | |
| ....GO:0019226 transmission of nerve impulse | * | | |
| .......GO:0051403 stress-activated MAPK cascade | *** | | |
| ........GO:0007254 JNK cascade | ** | | |
| ......GO:0000165 MAPK cascade | **** | | |
| .....GO:0016055 Wnt receptor signaling pathway | * | | |
| ....GO:0050877 neurological system process | ** | | |
| .....GO:0001944 vasculature development | *** | | |
| ......GO:0001568 blood vessel development | ** | | |
| .......GO:0048514 blood vessel morphogenesis | ** | | |
| ......GO:0022008 neurogenesis | ** | | |
| ........GO:0030182 neuron differentiation | ** | | |
| .........GO:0048666 neuron development | ** | | |
| ...GO:0061061 muscle structure development | ** | | |
| ....GO:0042692 muscle cell differentiation | ** | | |
| ....GO:0048646 anatomical structure formation involved in morphogenesis | **** | | |
| ...GO:0033554 cellular response to stress | | * | |
| .....GO:0006281 DNA repair | | **** | |
| ...GO:0001666 response to hypoxia | ** | | |
| ...GO:0070482 response to oxygen levels | *** | | |
| ....GO:0036293 response to decreased oxygen levels | ** | | |
| ....GO:0034613 cellular protein localization | | **** | |
| .....GO:0033365 protein localization to organelle | | **** | |
| ......GO:0070972 protein localization to endoplasmic reticulum | | **** | |
| ........GO:0045047 protein targeting to ER | | **** | |
| .....GO:0006886 intracellular protein transport | | **** | |
| ......GO:0006605 protein targeting | | **** | |
| .......GO:0006612 protein targeting to membrane | | **** | |
| .....GO:0015031 protein transport | | **** | |
| ....GO:0006811 ion transport | *** | | |
| .....GO:0006812 cation transport | ** | | |
| ......GO:0030001 metal ion transport | ** | | |
| ....GO:0050000 chromosome localization | | **** | |
| ...GO:0022411 cellular component disassembly | | **** | |
| ...GO:0034330 cell junction organization | * | | |
| .....GO:0034622 cellular macromolecular complex assembly | | **** | |
| ....GO:0007010 cytoskeleton organization | ** | | |
| .....GO:0000226 microtubule cytoskeleton organization | | ** | |
| ....GO:0070925 organelle assembly | | **** | |
| ....GO:0051276 chromosome organization | | *** | |
| .....GO:0032200 telomere organization | | *** | |
| .....GO:0000819 sister chromatid segregation | | **** | |
| ......GO:0043408 regulation of MAPK cascade | **** | | |
| ....GO:0010646 regulation of cell communication | **** | | |
| ....GO:0019725 cellular homeostasis | * | | |
| ..GO:0022402 cell cycle process | | **** | |
| ...GO:0035383 thioester metabolic process | | ** | |
| .....GO:0071616 acyl-CoA biosynthetic process | | ** | |
| .....GO:0035337 fatty-acyl-CoA metabolic process | | *** | |
| ...GO:0044248 cellular catabolic process | | **** | |
| .....GO:0046394 carboxylic acid biosynthetic process | | *** | |
| ...GO:0044255 cellular lipid metabolic process | | * | |
| ....GO:0006631 fatty acid metabolic process | | ** | |
| .....GO:0019752 carboxylic acid metabolic process | | **** | |
| ..GO:0007049 cell cycle | | **** | |
| ..GO:0007154 cell communication | *** | | |
| ...GO:0005975 carbohydrate metabolic process | | ** | |
| .....GO:0006694 steroid biosynthetic process | | ** | |
| .......GO:0006695 cholesterol biosynthetic process | | **** | |
| ....GO:0008610 lipid biosynthetic process | | ** | |
| ...GO:0055114 oxidation-reduction process | | ** | |

### Example 3 - using C-mapping to identify potential actives for treating periorbital dyschromia

This example illustrates the use of C-mapping to identify identifying connections between potential actives based on the gene expression signatures for Type I, Type II and Type III periorbital dyschromia, i.e., determining whether a perturbagen modulates one or more aspects of skin health with respect to one or more types of periorbital dyschromia. Table 19 illustrates the potential actives for Type I periorbital dyschromia, Table 20 illustrates potential actives for use in treating Type II periorbital dyschromia, and Table 21 illustrates potential actives for use in treating Type III periorbital dyschromia.

**Table 19 - Type I Periorbital Dyschromia**

| **Chip ID** | **Name** | **Cell Line** | **Dermis Score** | **Epidermis score** |
|---|---|---|---|---|
| CMP_559_55 | Permethrin | BJ Fibroblasts | -0.658449476 | -0.343864443 |
| CMP_516_94 | Permethrin | BJ Fibroblasts | -0.552276997 | 0 |
| CMP_520_57 | Permethrin | BJ Fibroblasts | 0 | -0.466751263 |
| CMP_512_22 | D-ALPHA-TOCOPHERYLQUINONE | t-keratinocytes | 0 | -0.399138011 |
| CMP_523_17 | D-ALPHA-TOCOPHERYLQUINONE | t- keratinocytes | -0.615476031 | -0.504972308 |
| CMP_521_32 | clonidine | t- keratinocytes | -0.547668634 | -0.424951413 |
| CMP_539_07 | clonidine | t- keratinocytes | 0 | -0.371356988 |
| CMP_545_94 | Orotic Acid | t- keratinocytes | -0.252665896 | 0 |
| CMP_535_42 | Dermaveil | t- keratinocytes | -0.571687664 | 0 |
| CMP_536_58 | German Chamomile flower fraction | t- keratinocytes | 0 | 0 |
| CMP_529_90 | GABA | BJ Fibroblasts | -0.563701337 | 0 |
| CMP_537_32 | 2-cyano-1-t--pentyl-3-(3-pyridyl)-GuanidineP1075 | BJ Fibroblasts | 0 | 0.311745684 |
| CMP_541_61 | 2-cyano-1-t--pentyl-3-(3-pyridyl)-GuanidineP1075 | BJ Fibroblasts | -0.592612947 | 0.371974114 |
| CMP_533_80 | METHANESULFONIC ACID | BJ Fibroblasts | -0.631155134 | 0.277957722 |
| CMP_539_14 | citalopram | t- keratinocytes | 0.564416854 | -0.380445296 |
| CMP_548_79 | DN-AGE | t- keratinocytes | 0.579732416 | -0.390786521 |
| CMP_511_53 | Volufiline | BJ Fibroblasts | 0 | -0.395847281 |
| CMP_560_87 | DN-AGE | t- keratinocytes | 0.530060455 | -0.40218956 |
| CMP_518_32 | Volufiline | BJ Fibroblasts | 0 | -0.428810784 |
| CMP_521_44 | OTZ (2-OXO-1,3-Thiazolidine) | t- keratinocytes | 0 | -0.430632747 |
| CMP_523_60 | German Chamomile flower fraction | t- keratinocytes | -0.237365143 | -0.394010711 |
| CMP_516_56 | GABA | BJ Fibroblasts | -0.469891668 | -0.168871848 |
| CMP_535_63 | DC Instalift Goji | t- keratinocytes | -0.345598158 | 0 |
| CMP_549_31 | GABA | BJ Fibroblasts | -0.642644595 | 0 |
| CMP_512_42 | OTZ (2-OXO-1,3-Thiazolidine) | t- keratinocytes | -0.689179194 | -0.29710892 |
| CMP_525_65 | Orotic Acid | t- keratinocytes | 0 | -0.387489704 |
| CMP_551_65 | DN-AGE | t- keratinocytes | 0.519529142 | -0.421309314 |
| CMP_512_44 | Orotic Acid | t- keratinocytes | 0 | -0.381400807 |
| CMP_538_11 | Dermaveil | t- keratinocytes | -0.660145862 | -0.318650315 |
| CMP_525_35 | German Chamomile flower fraction | t- keratinocytes | 0 | -0.477525004 |
| CMP_521_33 | citalopram | t- keratinocytes | -0.327948187 | -0.451192259 |
| CMP_553_09 | Sodium Chondroitin Sulfate | t- keratinocytes | -0.581237092 | -0.439141866 |
| CMP_554_11 | METHANESULFONIC ACID | BJ Fibroblasts | -0.602542146 | -0.278325726 |
| CMP_519_21 | DC Instalift Goji | t- keratinocytes | 0 | -0.498178037 |
| CMP_551_72 | Sodium Chondroitin Sulfate | t- keratinocytes | -0.245168482 | 0 |
| CMP_540_05 | 2-cyano-1-t--pentyl-3-(3-pyridyl)-GuanidineP1075 | BJ Fibroblasts | -0.593970746 | 0 |
| CMP_521_05 | DC Instalift Goji | t- keratinocytes | 0 | -0.450386667 |
| CMP_541_12 | HCl (4mM) and BSA (0.1%) | BJ Fibroblasts | 0 | -0.396596068 |
| CMP_520_64 | Actipone Hortensia Root extract | BJ Fibroblasts | 0 | -0.222269288 |
| CMP_535_66 | serotonin | t- keratinocytes | -0.491743047 | -0.326515935 |
| CMP_543_35 | HCl (4mM) and BSA (0.1%) | BJ Fibroblasts | 0 | 0 |
| CMP_550_94 | Actipone Hortensia Root extract | BJ Fibroblasts | -0.534829895 | -0.200762989 |
| CMP_533_19 | HCl (4mM) and BSA (0.1%) | BJ Fibroblasts | 0 | -0.216752277 |
| CMP_547_68 | serotonin | t- keratinocytes | 0 | -0.517834987 |
| CMP_518_06 | Actipone Hortensia Root extract | BJ Fibroblasts | -0.611695778 | -0.353350171 |
| CMP_520_12 | Biocellact Chamomilla BD | BJ Fibroblasts | -0.413741911 | -0.356343294 |
| CMP_518_31 | Biocellact Chamomilla BD | BJ Fibroblasts | -0.640122938 | -0.258669994 |
| CMP_529_45 | German Chamomile serum fraction | BJ Fibroblasts | 0 | 0 |
| CMP_525_76 | Instensyl | t- keratinocytes | 0.493508815 | -0.309000669 |
| CMP_524_11 | Coscap EGCG | t- keratinocytes | 0 | 0.215984014 |
| CMP_523_50 | Lunawhite | t- keratinocytes | -0.441294301 | -0.267300428 |
| CMP_545_50 | Unitone | t- keratinocytes | 0.528006208 | 0 |
| CMP_535_58 | MJB extract | t- keratinocytes | 0.486156858 | 0 |
| CMP_544_03 | Unitone | t- keratinocytes | 0.639182643 | -0.367850608 |
| CMP_556_06 | Nachyline | BJ Fibroblasts | -0.409539691 | 0 |
| CMP_519_16 | Peptamide 6 pure | t- keratinocytes | 0.634824214 | 0 |
| CMP_541_90 | monosodium tartrate | BJ Fibroblasts | 0.434738807 | -0.250054166 |
| CMP_559_84 | Acyclovir | BJ Fibroblasts | 0 | 0 |
| CMP_526_28 | German Chamomile flower fraction | BJ Fibroblasts | 0 | -0.319341489 |
| CMP_517_44 | monosodium tartrate | BJ Fibroblasts | 0 | -0.305614387 |
| CMP_521_55 | Lunawhite | t- keratinocytes | -0.233951474 | -0.324226158 |
| CMP_525_11 | Elestan-YL PW LS 9879 | t- keratinocytes | -0.509347575 | 0.328782586 |
| CMP_513_36 | Oxygeskin | BJ Fibroblasts | 0.483620189 | -0.21672773 |
| CMP_516_68 | Benzenebutanoic Acid | BJ Fibroblasts | 0 | -0.290528878 |
| CMP_537_60 | monosodium tartrate | BJ Fibroblasts | 0.418384761 | -0.248365488 |
| CMP_542_40 | Olixxol | t- keratinocytes | 0 | 0.270092508 |
| CMP_534_86 | Oxygeskin | BJ Fibroblasts | 0 | 0 |
| CMP_523_08 | German Chamomile vehicle | t- keratinocytes | 0 | 0 |
| CMP_523_89 | Himilayan Raspberry | t- keratinocytes | -0.67063579 | 0 |
| CMP_522_06 | German Chamomile flower fraction | BJ Fibroblasts | 0 | -0.352056073 |
| CMP_554_04 | Benzimidazole | BJ Fibroblasts | -0.274668006 | 0.264535735 |
| CMP_533_06 | monosodium tartrate | BJ Fibroblasts | 0 | 0 |
| CMP_523_63 | Glycocholate | t- keratinocytes | -0.317510397 | 0 |
| CMP_530_44 | Hexyldecanol (.05%) | t- keratinocytes | -0.422621468 | 0 |
| CMP_555_86 | TM B-8 | BJ Fibroblasts | -0.55144199 | 0.451887286 |
| CMP_519_20 | Puerarin | t- keratinocytes | 0 | 0 |
| CMP_554_43 | Stearyl Gallate | BJ Fibroblasts | 0.532029294 | -0.224775932 |
| CMP_519_10 | Lunawhite | t- keratinocytes | 0 | -0.349844197 |
| CMP_527_46 | Verityl AB1000 | t- keratinocytes | -0.347091473 | 0.289512913 |
| CMP_529_14 | Pinoxide | BJ Fibroblasts | 0 | 0 |
| CMP_530_68 | THIABENDAZOLE | t- keratinocytes | -0.269333374 | 0.284532489 |
| CMP_541_60 | nipecotic acid | BJ Fibroblasts | -0.342825756 | 0 |
| CMP_560_59 | 19719-NF2-6 | t- keratinocytes | -0.500882275 | 0.306164364 |
| CMP_533_94 | aminooxyacetic acid | BJ Fibroblasts | 0 | 0 |
| CMP_524_20 | 8-Cyclopentyl-1,3-dipropylxanthine | t- keratinocytes | -0.594482381 | 0.377966648 |
| CMP_557_60 | 19719-NF2-6 | t- keratinocytes | -0.692996369 | 0.242907715 |
| CMP_516_36 | nipecotic acid | BJ Fibroblasts | -0.616012213 | 0 |
| CMP_516_09 | aminooxyacetic acid | BJ Fibroblasts | -0.704887509 | 0.202559998 |
| CMP_521_84 | THIABENDAZOLE | t- keratinocytes | -0.684727852 | -0.258105411 |
| CMP_525_89 | Himilayan Raspberry | t- keratinocytes | -0.539449009 | 0 |
| CMP_525_50 | Pyridoxine Triisopalmitate | t- keratinocytes | -0.398732274 | 0 |
| CMP_520_62 | Shiso | BJ Fibroblasts | -0.24329276 | 0 |
| CMP_515_20 | Oxygenated Glycerol Triesters D | BJ Fibroblasts | -0.555548861 | 0 |
| CMP_516_06 | Oxygenated Glycerol Triesters D | BJ Fibroblasts | -0.548088978 | 0.178830868 |
| CMP_526_55 | Shiso | BJ Fibroblasts | -0.568364474 | 0 |
| CMP_524_58 | DMPO | t- keratinocytes | 0.549815592 | -0.318031769 |
| CMP_536_78 | German Chamomile vehicle | t- keratinocytes | 0 | -0.391646481 |
| CMP_539_84 | Net-STG | t- keratinocytes | 0.387856288 | -0.334582395 |
| CMP_539_89 | Unitone | t- keratinocytes | 0.516461364 | -0.303091514 |
| CMP_560_96 | Instensyl | t- keratinocytes | 0.512615179 | -0.283173676 |
| CMP_511_54 | Crodarom purple orchid | BJ Fibroblasts | 0.490638022 | -0.337486864 |
| CMP_518_33 | Crodarom purple orchid | BJ Fibroblasts | 0 | -0.325294869 |
| CMP_535_19 | p-Coumaric acid | t- keratinocytes | 0 | -0.333469052 |
| CMP_520_30 | PRAZIQUANTEL | BJ Fibroblasts | -0.273288094 | 0.192072302 |
| CMP_543_06 | lactobionic acid | BJ Fibroblasts | 0 | -0.33770718 |
| CMP_526_36 | Ecophysallis | BJ Fibroblasts | -0.34401132 | 0.203286471 |
| CMP_534_16 | bu224 | BJ Fibroblasts | 0.520147891 | -0.25691092 |
| CMP_511_26 | Eterniskin | BJ Fibroblasts | 0 | -0.373887976 |
| CMP_511_33 | Reactive Blue 2 | BJ Fibroblasts | 0 | -0.369857992 |
| CMP_526_16 | Unisooth EG-28 | BJ Fibroblasts | 0 | -0.303639462 |
| CMP_531_12 | 6-ACETAMIDOHEXANOIC ACID | BJ Fibroblasts | -0.390316678 | -0.217585864 |
| CMP_518_16 | Corum 8802 | BJ Fibroblasts | 0 | -0.218773862 |
| CMP_533_15 | TM B-8 | BJ Fibroblasts | 0 | 0 |
| CMP_528_21 | Paeonol | t- keratinocytes | -0.203870124 | 0 |
| CMP_535_37 | Pinoxide | t- keratinocytes | -0.333991845 | 0 |
| CMP_545_10 | Olixxol | t- keratinocytes | -0.495525531 | 0.312763273 |
| CMP_525_29 | Pinoxide | t- keratinocytes | 0 | 0 |
| CMP_545_09 | Nachyline | t- keratinocytes | -0.548868196 | 0 |
| CMP_530_70 | Arginine Tartrate | t- keratinocytes | -0.365826588 | 0 |
| CMP_544_35 | Nachyline | t- keratinocytes | -0.422480271 | -0.187984095 |
| CMP_525_88 | Coscap EGCG | t- keratinocytes | -0.48075812 | 0.317229221 |
| CMP_525_78 | German Chamomile vehicle | t- keratinocytes | -0.327706165 | -0.258671414 |
| CMP_546_04 | lactobionic acid | BJ Fibroblasts | -0.6318317 | 0 |
| CMP_523_27 | n-Butyl Alcohol | t- keratinocytes | -0.402604224 | 0 |
| CMP_515_19 | Phytostem Edelweiss | BJ Fibroblasts | -0.574146228 | 0.265363236 |
| CMP_536_24 | Coscap EGCG | t- keratinocytes | -0.503865863 | 0 |
| CMP_522_04 | German Chamomile serum fraction | BJ Fibroblasts | -0.576011198 | 0 |
| CMP_524_80 | Elestan-YL PW LS 9879 | t- keratinocytes | -0.556325239 | 0.31989106 |
| CMP_545_20 | Glycocholate | t- keratinocytes | -0.64099223 | 0.320024547 |
| CMP_559_80 | PRAZIQUANTEL | BJ Fibroblasts | -0.626768507 | 0 |
| CMP_532_24 | Arginine Tartrate | t- keratinocytes | -0.571132818 | 0.286311038 |
| CMP_544_10 | Olixxol | t- keratinocytes | -0.628208671 | 0.301961739 |
| CMP_545_35 | Net-STG | t- keratinocytes | 0.379605624 | -0.363947011 |
| CMP_522_45 | Pinoxide | BJ Fibroblasts | -0.452594486 | 0.194474469 |
| CMP_551_71 | THIABENDAZOLE | t- keratinocytes | 0 | -0.29710182 |
| CMP_549_19 | Benzenebutanoic Acid | BJ Fibroblasts | 0 | -0.413569067 |
| CMP_542_14 | Nachyline | t- keratinocytes | 0 | 0.25914288 |
| CMP_516_10 | TM B-8 | BJ Fibroblasts | -0.383280993 | 0 |
| CMP_523_21 | DMPO | t- keratinocytes | -0.431640192 | -0.592430771 |
| CMP_523_22 | Pinoxide | t- keratinocytes | -0.552294849 | -0.414390076 |
| CMP_534_06 | Eterniskin | BJ Fibroblasts | 0 | 0 |
| CMP_522_23 | thioperamide | BJ Fibroblasts | -0.605351673 | -0.430059846 |
| CMP_521_16 | Puerarin | t- keratinocytes | 0 | -0.320406954 |
| CMP_538_87 | p-Coumaric acid | t- keratinocytes | 0 | 0 |
| CMP_536_49 | Acetylcarnitine | t- keratinocytes | 0 | 0 |
| CMP_521_22 | p-Coumaric acid | t- keratinocytes | 0.311860305 | -0.319119956 |
| CMP_520_29 | Oxygeskin | BJ Fibroblasts | 0 | -0.400918589 |
| CMP_521_42 | n-Butyl Alcohol | t- keratinocytes | 0.331767797 | -0.451919339 |
| CMP_516_05 | Phytostem Edelweiss | BJ Fibroblasts | -0.46505163 | 0 |
| CMP_512_01 | Peptamide 6 pure | t- keratinocytes | 0.597146248 | -0.247924655 |
| CMP_558_94 | lactobionic acid | BJ Fibroblasts | -0.508700018 | 0 |
| CMP_511_36 | Corum 8802 | BJ Fibroblasts | -0.562115716 | -0.302381474 |
| CMP_554_68 | bu224 | BJ Fibroblasts | 0.582096849 | -0.374480352 |
| CMP_523_71 | Pyridoxine Triisopalmitate | t- keratinocytes | -0.549774004 | 0 |
| CMP_536_28 | Arginine Tartrate | t- keratinocytes | 0 | 0 |
| CMP_554_77 | 6-ACETAMIDOHEXANOIC ACID | BJ Fibroblasts | 0 | -0.452315339 |
| CMP_520_55 | Ecophysallis | BJ Fibroblasts | -0.585528574 | 0 |
| CMP_523_67 | Instensyl | t- keratinocytes | -0.656533585 | -0.367195748 |
| CMP_522_47 | Unisooth EG-28 | BJ Fibroblasts | 0.232607267 | -0.379380034 |
| CMP_520_24 | Benzimidazole | BJ Fibroblasts | -0.518026698 | -0.414248473 |
| CMP_534_83 | Pinoxide | BJ Fibroblasts | -0.551397156 | 0 |
| CMP_553_54 | Hexyldecanol (.05%) | t- keratinocytes | 0.484783438 | -0.382892703 |
| CMP_538_07 | Acetylcarnitine | t- keratinocytes | 0 | -0.403721015 |
| CMP_534_46 | Corum 8802 | BJ Fibroblasts | -0.388850952 | 0 |
| CMP_535_40 | Peptamide 6 pure | t- keratinocytes | 0.522119774 | -0.390754468 |
| CMP_525_39 | n-Butyl Alcohol | t- keratinocytes | 0 | -0.238107642 |
| CMP_536_34 | Verityl AB1000 | t- keratinocytes | -0.616266407 | -0.234339156 |
| CMP_546_52 | Reactive Blue 2 | BJ Fibroblasts | 0 | -0.225552513 |
| CMP_535_02 | Hexyldecanol (.05%) | t- keratinocytes | 0.50781896 | -0.350861989 |
| CMP_542_83 | Acetylcarnitine | t- keratinocytes | 0 | -0.32693567 |
| CMP_534_05 | thioperamide | BJ Fibroblasts | 0 | 0 |
| CMP_519_40 | MJB extract | t- keratinocytes | 0.26577019 | -0.251206459 |
| CMP_523_01 | 8-Cyclopentyl-1,3-dipropylxanthine | t- keratinocytes | -0.447210557 | -0.414939241 |
| CMP_516_95 | Benzimidazole | BJ Fibroblasts | -0.620673929 | 0 |
| CMP_549_07 | aminooxyacetic acid | BJ Fibroblasts | -0.342423265 | 0 |
| CMP_531_85 | Stearyl Gallate | BJ Fibroblasts | -0.248281906 | -0.378735723 |
| CMP_518_10 | Eterniskin | BJ Fibroblasts | -0.369642546 | -0.352699572 |
| CMP_552_46 | nipecotic acid | BJ Fibroblasts | 0 | -0.426218124 |
| CMP_521_85 | Paeonol | t- keratinocytes | -0.553534173 | 0 |
| CMP_558_56 | Nachyline | BJ Fibroblasts | -0.584569411 | -0.272479662 |
| CMP_524_56 | Verityl AB1000 | t- keratinocytes | 0 | 0 |
| CMP_523_86 | MJB extract | t- keratinocytes | 0 | -0.462376808 |
| CMP_534_24 | German Chamomile serum fraction | BJ Fibroblasts | -0.318945286 | -0.458518045 |
| CMP_535_55 | Puerarin | t- keratinocytes | -0.488436492 | -0.502061347 |
| CMP_520_41 | Acyclovir | BJ Fibroblasts | -0.62461242 | -0.369630576 |
| CMP_555_10 | Nachyline | BJ Fibroblasts | 0 | -0.355442558 |
| CMP_540_55 | monosodium tartrate | BJ Fibroblasts | -0.408280486 | -0.442344755 |
| CMP_511_23 | Actistem Acanax | BJ Fibroblasts | 0 | 0 |

**Table 20 - Type II Periorbital Dyschromia**

| **Chip ID** | Name | **Cell Line** | **Dermis Score** | **Epidermis Score** |
|---|---|---|---|---|
| CMP_540_46 | Phenacetin | BJ Fibroblasts | -0.335621216 | -0.556650031 |
| CMP_522_03 | CR10010 | BJ Fibroblasts | -0.519975261 | -0.590887144 |
| CMP_546_66 | Phenacetin | BJ Fibroblasts | -0.767589284 | -0.676832613 |
| CMP_537_85 | CR10010 | BJ Fibroblasts | -0.317972292 | -0.701517457 |
| CMP_543_24 | Phenacetin | BJ Fibroblasts | -0.449518881 | -0.758261619 |
| CMP_526_20 | CR10010 | BJ Fibroblasts | -0.614855485 | -0.795060556 |
| CMP_546_05 | L-Leucine | BJ Fibroblasts | -0.626634771 | 0 |
| CMP_540_40 | L-Leucine | BJ Fibroblasts | 0 | -0.422826162 |
| CMP_521_09 | METHANESULFONIC ACID | t- keratinocytes | -0.62415864 | -0.603844765 |
| CMP_528_05 | METHANESULFONIC ACID | t- keratinocytes | -0.39629114 | -0.646498083 |
| CMP_543_10 | L-Leucine | BJ Fibroblasts | -0.372909861 | -0.705912604 |
| CMP_559_91 | Potassium Sorbate | BJ Fibroblasts | -0.602278794 | 0 |
| CMP_558_36 | Potassium Sorbate | BJ Fibroblasts | -0.739060804 | 0 |
| CMP_520_80 | Formononetin | BJ Fibroblasts | -0.593358258 | 0 |
| CMP_518_38 | Ecosamba PRO | BJ Fibroblasts | -0.610153574 | 0 |
| CMP_559_46 | Formononetin | BJ Fibroblasts | -0.626910778 | 0 |
| CMP_516_22 | Formononetin | BJ Fibroblasts | -0.418234349 | -0.435343152 |
| CMP_534_34 | Ecosamba PRO | BJ Fibroblasts | -0.60890689 | -0.531916094 |
| CMP_532_47 | lithium chloride | t- keratinocytes | -0.707525609 | 0 |
| CMP_547_91 | lithium chloride | t- keratinocytes | -0.48373543 | -0.691299982 |
| CMP_546_88 | Tazarotene | BJ Fibroblasts | 0 | 0 |
| CMP_534_49 | HerbEx Resverol | BJ Fibroblasts | 0.484419763 | 0 |
| CMP_519_02 | Hyadisine | t- keratinocytes | 0 | 0 |
| CMP_541_78 | HerbEx Resverol | BJ Fibroblasts | 0 | -0.66400564 |
| CMP_543_77 | Tazarotene | BJ Fibroblasts | -0.393891153 | -0.665250238 |
| CMP_560_07 | Hyadisine | t- keratinocytes | -0.415823365 | -0.731918934 |
| CMP_550_63 | HerbEx Resverol | BJ Fibroblasts | 0.273870346 | -0.794026941 |
| CMP_521_32 | clonidine | t- keratinocytes | -0.539811278 | -0.801410342 |
| CMP_540_56 | Tazarotene | BJ Fibroblasts | -0.55674876 | -0.803060678 |
| CMP_523_31 | Hyadisine | t- keratinocytes | -0.345352456 | -0.812016108 |
| CMP_539_07 | clonidine | t- keratinocytes | -0.429086137 | -0.834424158 |
| CMP_560_60 | Capsuji | t- keratinocytes | -0.456163418 | 0.60433875 |
| CMP_537_47 | Glycyrrhizic Acid | BJ Fibroblasts | 0 | 0.578048404 |
| CMP_555_12 | EIPA | BJ Fibroblasts | -0.375064682 | 0.560736616 |
| CMP_516_38 | Norepinephrine | BJ Fibroblasts | 0 | 0.54756152 |
| CMP_551_20 | BASF1 | t- keratinocytes | 0 | 0.514985089 |
| CMP_535_42 | Dermaveil | t- keratinocytes | -0.486568533 | 0.497876169 |
| CMP_539_42 | DL-Lysine monohydrate | t- keratinocytes | -0.641870848 | 0.495650295 |
| CMP_557_43 | Dermcom | t- keratinocytes | -0.198828468 | 0.477493559 |
| CMP_513_21 | cinnarizine | BJ Fibroblasts | -0.582548463 | 0.47646745 |
| CMP_539_69 | Phenytoin | t- keratinocytes | -0.33630114 | 0.446966101 |
| CMP_523_34 | Phenytoin | t- keratinocytes | -0.62429753 | 0.445567728 |
| CMP_538_27 | L-Cysteine | t- keratinocytes | 0 | 0.43903252 |
| CMP_554_04 | Benzimidazole | BJ Fibroblasts | -0.372225682 | 0.426684925 |
| CMP_514_82 | Pro-Lipiskin w/o preservative | BJ Fibroblasts | -0.637367055 | 0.415208245 |
| CMP_529_90 | GABA | BJ Fibroblasts | -0.333864137 | 0.3818812 |
| CMP_526_43 | D-(+)-Mannose | BJ Fibroblasts | -0.236951748 | 0.37116406 |
| CMP_511_56 | OTZ (2-OXO-1,3-Thiazolidine) | BJ Fibroblasts | -0.347105914 | 0.361653379 |
| CMP_527_46 | Verityl AB1000 | t- keratinocytes | -0.394591633 | 0 |
| CMP_530_68 | THIABENDAZOLE | t- keratinocytes | 0 | 0 |
| CMP_541_60 | nipecotic acid | BJ Fibroblasts | -0.513955225 | 0 |
| CMP_527_40 | I-n6-(1-iminoethyl)lysine | t- keratinocytes | -0.596664764 | 0 |
| CMP_528_83 | Ethanolamine | t- keratinocytes | -0.335668265 | 0 |
| CMP_559_59 | Adenine | BJ Fibroblasts | -0.468184086 | 0 |
| CMP_533_94 | aminooxyacetic acid | BJ Fibroblasts | -0.570383076 | 0 |
| CMP_517_06 | Pro-Lipiskin w/o preservative | BJ Fibroblasts | -0.553438625 | 0 |
| CMP_524_20 | 8-Cyclopentyl-1,3-dipropylxanthine | t- keratinocytes | -0.703182299 | 0 |
| CMP_553_12 | Matrine | t- keratinocytes | -0.329625042 | 0 |
| CMP_523_15 | Matrine | t- keratinocytes | -0.498315497 | 0 |
| CMP_560_03 | Dermcom | t- keratinocytes | -0.614633481 | 0 |
| CMP_547_84 | I-n6-(1-iminoethyl)lysine | t- keratinocytes | -0.564952527 | 0 |
| CMP_516_36 | nipecotic acid | BJ Fibroblasts | -0.641111585 | 0 |
| CMP_516_09 | aminooxyacetic acid | BJ Fibroblasts | -0.711171529 | 0 |
| CMP_521_84 | THIABENDAZOLE | t- keratinocytes | -0.615607276 | 0 |
| CMP_518_36 | OTZ (2-OXO-1,3-Thiazolidine) | BJ Fibroblasts | -0.58754277 | 0 |
| CMP_525_50 | Pyridoxine Triisopalmitate | t- keratinocytes | -0.468424428 | 0 |
| CMP_517_50 | Phyco AC | BJ Fibroblasts | -0.576783368 | 0 |
| CMP_557_26 | Marine Elastine | t- keratinocytes | -0.240314863 | 0 |
| CMP_560_83 | Marine Elastine | t- keratinocytes | -0.753825115 | 0 |
| CMP_515_04 | Phyco AC | BJ Fibroblasts | -0.612121659 | 0 |
| CMP_533_80 | METHANESULFONIC ACID | BJ Fibroblasts | -0.751219454 | 0 |
| CMP_543_06 | lactobionic acid | BJ Fibroblasts | 0 | 0 |
| CMP_538_05 | DL-Lysine monohydrate | t- keratinocytes | 0 | 0 |
| CMP_516_13 | EIPA | BJ Fibroblasts | -0.534623255 | 0 |
| CMP_541_86 | Lipochroman-6 | BJ Fibroblasts | -0.54835891 | 0 |
| CMP_560_89 | Regu Fade | t- keratinocytes | 0 | 0 |
| CMP_516_56 | GABA | BJ Fibroblasts | -0.414139513 | 0 |
| CMP_536_20 | Regu Fade | t- keratinocytes | -0.410258826 | 0 |
| CMP_553_83 | BASF1 | t- keratinocytes | -0.481416034 | 0 |
| CMP_537_11 | Lipochroman-6 | BJ Fibroblasts | -0.562912595 | 0 |
| CMP_548_72 | BASF1 | t- keratinocytes | -0.605197261 | 0 |
| CMP_545_09 | Nachyline | t- keratinocytes | -0.503405229 | 0 |
| CMP_545_78 | beta-Ionone | t- keratinocytes | -0.483920981 | 0 |
| CMP_544_35 | Nachyline | t- keratinocytes | -0.603897085 | 0 |
| CMP_534_31 | Anti-Leukine 6 | BJ Fibroblasts | -0.571391358 | 0 |
| CMP_545_89 | L-Cysteine | t- keratinocytes | -0.616419373 | 0 |
| CMP_526_33 | urocanic acid | BJ Fibroblasts | -0.562298544 | 0 |
| CMP_546_04 | lactobionic acid | BJ Fibroblasts | -0.477456608 | 0 |
| CMP_558_45 | Anti-Leukine 6 | BJ Fibroblasts | -0.744562551 | 0 |
| CMP_549_31 | GABA | BJ Fibroblasts | -0.674358254 | 0 |
| CMP_558_34 | urocanic acid | BJ Fibroblasts | -0.583875767 | 0 |
| CMP_535_34 | Regu Fade | t- keratinocytes | -0.592232297 | 0 |
| CMP_532_18 | beta-Ionone | t- keratinocytes | -0.619682128 | 0 |
| CMP_534_15 | Norepinephrine | BJ Fibroblasts | -0.322964944 | -0.365178017 |
| CMP_537_14 | Yuzu Ceramide B | BJ Fibroblasts | 0 | -0.374076441 |
| CMP_521_95 | Ethanolamine | t- keratinocytes | 0 | -0.381902907 |
| CMP_551_71 | THIABENDAZOLE | t- keratinocytes | -0.248781851 | -0.384125941 |
| CMP_513_04 | OTZ (2-OXO-1,3-Thiazolidine) | BJ Fibroblasts | -0.479433342 | -0.398099324 |
| CMP_541_36 | Yuzu Ceramide B | BJ Fibroblasts | -0.667639961 | -0.398481529 |
| CMP_542_14 | Nachyline | t- keratinocytes | 0 | -0.415187958 |
| CMP_541_09 | EIPA | BJ Fibroblasts | -0.537347399 | -0.433002658 |
| CMP_538_11 | Dermaveil | t- keratinocytes | -0.651117586 | -0.443130465 |
| CMP_522_19 | Anti-Leukine 6 | BJ Fibroblasts | 0 | -0.45438683 |
| CMP_559_35 | cinnarizine | BJ Fibroblasts | -0.498323117 | -0.465786623 |
| CMP_520_11 | Adenine | BJ Fibroblasts | -0.299833739 | -0.466241454 |
| CMP_540_32 | Glycyrrhizic Acid | BJ Fibroblasts | -0.507734538 | -0.481349482 |
| CMP_558_94 | lactobionic acid | BJ Fibroblasts | -0.678537338 | -0.497654434 |
| CMP_539_86 | L-Cysteine | t- keratinocytes | -0.326245189 | -0.502494066 |
| CMP_523_71 | Pyridoxine Triisopalmitate | t- keratinocytes | -0.714446259 | -0.506622644 |
| CMP_538_83 | Ethanolamine | t- keratinocytes | -0.694496893 | -0.520209157 |
| CMP_540_60 | Yuzu Ceramide B | BJ Fibroblasts | -0.467115863 | -0.52154809 |
| CMP_524_31 | Matrine | t- keratinocytes | -0.594420152 | -0.530316272 |
| CMP_520_24 | Benzimidazole | BJ Fibroblasts | 0 | -0.531404662 |
| CMP_554_11 | METHANESULFONIC ACID | BJ Fibroblasts | -0.555312142 | -0.541688678 |
| CMP_536_34 | Verityl AB1000 | t- keratinocytes | -0.83662885 | -0.564711419 |
| CMP_523_01 | 8-Cyclopentyl-1,3-dipropylxanthine | t- keratinocytes | -0.484636987 | -0.608229566 |
| CMP_516_95 | Benzimidazole | BJ Fibroblasts | -0.80982277 | -0.611132007 |
| CMP_549_07 | aminooxyacetic acid | BJ Fibroblasts | -0.46068195 | -0.614956282 |
| CMP_544_92 | DL-Lysine monohydrate | t- keratinocytes | -0.534879503 | -0.619315116 |
| CMP_552_46 | nipecotic acid | BJ Fibroblasts | 0 | -0.630761163 |
| CMP_554_24 | D-(+)-Mannose | BJ Fibroblasts | -0.609012619 | -0.639073094 |
| CMP_516_88 | cinnarizine | BJ Fibroblasts | 0 | -0.64930903 |
| CMP_516_23 | Adenine | BJ Fibroblasts | -0.65418175 | -0.649500538 |
| CMP_524_56 | Verityl AB1000 | t- keratinocytes | 0 | -0.653370661 |
| CMP_540_85 | Lipochroman-6 | BJ Fibroblasts | 0 | -0.661430629 |
| CMP_557_10 | Capsuji | t- keratinocytes | -0.616464475 | -0.687035482 |
| CMP_555_55 | Norepinephrine | BJ Fibroblasts | -0.686789845 | -0.729258515 |
| CMP_541_24 | Glycyrrhizic Acid | BJ Fibroblasts | -0.725928879 | -0.912511513 |
| CMP_533_51 | HerbEx Gynostem Extract | BJ Fibroblasts | 0 | 0 |
| CMP_520_12 | Biocellact Chamomilla BD | BJ Fibroblasts | 0 | 0 |
| CMP_518_31 | Biocellact Chamomilla BD | BJ Fibroblasts | -0.581659062 | -0.66936421 |
| CMP_541_55 | HerbEx Gynostem Extract | BJ Fibroblasts | -0.797662034 | -0.845916864 |
| CMP_541_12 | HCl (4mM) and BSA (0.1%) | BJ Fibroblasts | 0 | 0 |
| CMP_520_64 | Actipone Hortensia Root extract | BJ Fibroblasts | 0 | 0 |
| CMP_551_52 | Retinyl Propionate | t- keratinocytes | 0.431990569 | 0 |
| CMP_513_07 | Pitera 8x | BJ Fibroblasts | -0.295801867 | 0 |
| CMP_541_50 | Arginine Aminobenzoate | BJ Fibroblasts | 0 | 0 |
| CMP_546_75 | Retinyl Propionate | BJ Fibroblasts | 0 | -0.340880044 |
| CMP_543_71 | N-Acetyl-L-Cysteine | BJ Fibroblasts | 0 | -0.355630008 |
| CMP_552_87 | Oxidized Glutathione | BJ Fibroblasts | 0 | -0.43576451 |
| CMP_555_60 | Citral | BJ Fibroblasts | 0.291480506 | -0.439378816 |
| CMP_526_15 | I-OMe-Tyrphostin AG 538 | BJ Fibroblasts | 0 | -0.462644595 |
| CMP_511_23 | Actistem Acanax | BJ Fibroblasts | -0.574953802 | -0.467654434 |
| CMP_554_66 | aminopterin | BJ Fibroblasts | -0.290183316 | -0.481288621 |
| CMP_543_46 | Diacetyl monoxime | BJ Fibroblasts | 0.402842566 | -0.49254032 |
| CMP_534_10 | Pitera 8x | BJ Fibroblasts | -0.479538251 | -0.494783641 |
| CMP_545_60 | Lumikit | t- keratinocytes | 0.486426619 | -0.495948918 |
| CMP_512_22 | D-ALPHA-TOCOPHERYLQUINONE | t- keratinocytes | 0.429810775 | -0.50225955 |
| CMP_550_16 | Retinyl Propionate | BJ Fibroblasts | 0 | -0.53078003 |
| CMP_516_11 | aminopterin | BJ Fibroblasts | -0.490306969 | -0.547860142 |
| CMP_533_78 | FGIN-1-27 | BJ Fibroblasts | -0.298272731 | -0.562378025 |
| CMP_548_71 | Retinyl Propionate | t- keratinocytes | 0 | -0.564246242 |
| CMP_535_66 | serotonin | t- keratinocytes | -0.562264439 | -0.56871361 |
| CMP_547_32 | Dimethylglycine | t- keratinocytes | -0.497429532 | -0.570496014 |
| CMP_556_33 | 3-CQA | BJ Fibroblasts | 0.366848237 | -0.579652486 |
| CMP_542_88 | 5 amino leuvulinic acid | t- keratinocytes | -0.167716431 | -0.589265616 |
| CMP_532_42 | 4-imidazolemethanol | t- keratinocytes | 0 | -0.600439413 |
| CMP_543_35 | HCl (4mM) and BSA (0.1%) | BJ Fibroblasts | 0 | -0.602898383 |
| CMP_522_35 | tamoxifen | BJ Fibroblasts | 0 | -0.603707017 |
| CMP_550_94 | Actipone Hortensia Root extract | BJ Fibroblasts | -0.51740814 | -0.609801595 |
| CMP_533_19 | HCl (4mM) and BSA (0.1%) | BJ Fibroblasts | 0 | -0.618409308 |
| CMP_546_51 | tamoxifen | BJ Fibroblasts | 0.512572248 | -0.622040655 |
| CMP_546_60 | Arginine Aminobenzoate | BJ Fibroblasts | -0.249204378 | -0.636809486 |
| CMP_538_10 | 5 amino leuvulinic acid | t- keratinocytes | -0.522804304 | -0.653153592 |
| CMP_518_09 | Actistem Acanax | BJ Fibroblasts | 0 | -0.664576512 |
| CMP_549_56 | Retinyl Propionate | BJ Fibroblasts | 0 | -0.667662954 |
| CMP_550_33 | I-OMe-Tyrphostin AG 538 | BJ Fibroblasts | 0.257022307 | -0.669019333 |
| CMP_549_63 | Diacetyl monoxime | BJ Fibroblasts | 0 | -0.672373765 |
| CMP_526_85 | Citral | BJ Fibroblasts | 0 | -0.673397034 |
| CMP_537_56 | Arginine Aminobenzoate | BJ Fibroblasts | 0 | -0.697962997 |
| CMP_544_83 | Lumikit | t- keratinocytes | -0.271196524 | -0.699375571 |
| CMP_553_56 | Retinyl Propionate | t- keratinocytes | -0.409050638 | -0.699971396 |
| CMP_547_68 | serotonin | t- keratinocytes | 0 | -0.702047755 |
| CMP_554_71 | FGIN-1-27 | BJ Fibroblasts | -0.305297284 | -0.704308523 |
| CMP_549_89 | N-Acetyl-L-Cysteine | BJ Fibroblasts | 0 | -0.709100481 |
| CMP_531_13 | aminopterin | BJ Fibroblasts | -0.514299202 | -0.73297243 |
| CMP_520_49 | Pitera 8x | BJ Fibroblasts | 0 | -0.742128903 |
| CMP_555_83 | 3-CQA | BJ Fibroblasts | 0 | -0.755455947 |
| CMP_523_17 | D-ALPHA-TOCOPHERYLQUINONE | t- keratinocytes | -0.485357036 | -0.762244538 |
| CMP_535_33 | Dimethylglycine | t- keratinocytes | 0 | -0.767352971 |
| CMP_550_35 | Oxidized Glutathione | BJ Fibroblasts | -0.353367704 | -0.793766458 |
| CMP_547_83 | 4-imidazolemethanol | t- keratinocytes | -0.528486069 | -0.879548212 |
| CMP_518_06 | Actipone Hortensia Root extract | BJ Fibroblasts | -0.394436698 | -0.893214047 |

**Table 21 - Type III Periorbital Dyschromia**

| **Chip ID** | Name | **Cell Line** | **Dermis Score** | **Epidermis Score** |
|---|---|---|---|---|
| CMP_515_69 | Timecode | BJ Fibroblasts | -0.471153714 | -0.646811921 |
| CMP_527_29 | Corum 9515 | t- keratinocytes | 0 | 0 |
| CMP_530_68 | THIABENDAZOLE | t- keratinocytes | -0.327815714 | -0.63143915 |
| CMP_516_31 | Timecode | BJ Fibroblasts | -0.411751973 | -0.638585803 |
| CMP_536_36 | Corum 9515 | t- keratinocytes | -0.456578216 | -0.626653886 |
| CMP_521_84 | THIABENDAZOLE | t- keratinocytes | -0.538600207 | -0.773555677 |
| CMP_551_71 | THIABENDAZOLE | t- keratinocytes | -0.332500964 | -0.435931471 |
| CMP_524_10 | Corum 9515 | t- keratinocytes | -0.378655387 | -0.681314588 |
| CMP_521_09 | METHANESULFONIC ACID | t- keratinocytes | -0.453818189 | -0.772010022 |
| CMP_528_05 | METHANESULFONIC ACID | t- keratinocytes | -0.421307894 | -0.523186659 |
| CMP_523_89 | Himilayan Raspberry | t- keratinocytes | -0.515487392 | -0.607127584 |
| CMP_554_04 | Benzimidazole | BJ Fibroblasts | -0.282186517 | 0 |
| CMP_528_83 | Ethanolamine | t- keratinocytes | -0.400834601 | -0.531958087 |
| CMP_533_94 | aminooxyacetic acid | BJ Fibroblasts | -0.412847869 | -0.517370418 |
| CMP_559_91 | Potassium Sorbate | BJ Fibroblasts | -0.414588278 | -0.379569513 |
| CMP_524_20 | 8-Cyclopentyl-1,3-dipropylxanthine | t- keratinocytes | -0.420473698 | -0.56368673 |
| CMP_558_36 | Potassium Sorbate | BJ Fibroblasts | -0.49188262 | -0.65850425 |
| CMP_516_09 | aminooxyacetic acid | BJ Fibroblasts | -0.535100724 | -0.57752054 |
| CMP_525_89 | Himilayan Raspberry | t- keratinocytes | 0 | -0.631366928 |
| CMP_521_95 | Ethanolamine | t- keratinocytes | 0 | -0.290568235 |
| CMP_531_86 | Melibiose | BJ Fibroblasts | -0.446925527 | -0.543053578 |
| CMP_538_83 | Ethanolamine | t- keratinocytes | -0.512583125 | -0.60850851 |
| CMP_520_24 | Benzimidazole | BJ Fibroblasts | -0.295009636 | -0.534493539 |
| CMP_554_34 | Melibiose | BJ Fibroblasts | -0.522362404 | -0.599868541 |
| CMP_523_01 | 8-Cyclopentyl-1,3-dipropylxanthine | t- keratinocytes | -0.496383868 | -0.702263405 |
| CMP_516_95 | Benzimidazole | BJ Fibroblasts | -0.543470675 | -0.567490313 |
| CMP_549_07 | aminooxyacetic acid | BJ Fibroblasts | -0.287912077 | -0.559411884 |
| CMP_511_23 | Actistem Acanax | BJ Fibroblasts | -0.421265088 | 0 |
| CMP_518_09 | Actistem Acanax | BJ Fibroblasts | -0.47789321 | -0.666661392 |
| CMP_560_60 | Capsuji | t- keratinocytes | -0.400311403 | 0 |
| CMP_543_45 | Telosense CR 11033 | BJ Fibroblasts | -0.527645913 | 0 |
| CMP_537_75 | Telosense CR 11033 | BJ Fibroblasts | -0.279641126 | -0.424369383 |
| CMP_540_34 | Telosense CR 11033 | BJ Fibroblasts | -0.499859615 | -0.531134847 |
| CMP_521_17 | Ethylenediamine | t- keratinocytes | 0 | -0.458735317 |
| CMP_530_10 | Ethylenediamine | t- keratinocytes | -0.488704481 | -0.725276206 |
| CMP_533_80 | METHANESULFONIC ACID | BJ Fibroblasts | -0.525706896 | -0.651153714 |
| CMP_554_11 | METHANESULFONIC ACID | BJ Fibroblasts | 0 | -0.538899032 |
| CMP_557_10 | Capsuji | t- keratinocytes | -0.471606922 | -0.651010285 |
| CMP_540_46 | Phenacetin | BJ Fibroblasts | -0.393168198 | 0 |
| CMP_546_66 | Phenacetin | BJ Fibroblasts | -0.539539285 | -0.609345343 |
| CMP_543_24 | Phenacetin | BJ Fibroblasts | -0.407074635 | 0 |
| CMP_521_32 | clonidine | t- keratinocytes | -0.392923742 | -0.740578175 |
| CMP_539_07 | clonidine | t- keratinocytes | -0.342687806 | -0.559401943 |
| CMP_512_22 | D-ALPHA-TOCOPHERYLQUINONE | t- keratinocytes | 0 | 0 |
| CMP_533_78 | FGIN-1-27 | BJ Fibroblasts | -0.381969854 | -0.579879902 |
| CMP_554_71 | FGIN-1-27 | BJ Fibroblasts | -0.352081229 | -0.553805206 |
| CMP_523_17 | D-ALPHA-TOCOPHERYLQUINONE | t- keratinocytes | -0.55924188 | -0.771951596 |
| CMP_525_74 | Retinol H10 | t- keratinocytes | 0.306783925 | -0.472602601 |
| CMP_520_12 | Biocellact Chamomilla BD | BJ Fibroblasts | 0 | -0.553474936 |
| CMP_518_31 | Biocellact Chamomilla BD | BJ Fibroblasts | 0 | -0.568575457 |
| CMP_523_41 | Retinol H10 | t- keratinocytes | -0.332376402 | -0.733664618 |
| CMP_529_45 | German Chamomile serum fraction | BJ Fibroblasts | -0.370871118 | 0 |
| CMP_523_50 | Lunawhite | t- keratinocytes | -0.316704197 | -0.638371168 |
| CMP_556_06 | Nachyline | BJ Fibroblasts | 0 | -0.293941939 |
| CMP_535_93 | BIOCHANIN A | t- keratinocytes | 0.25743209 | -0.422059522 |
| CMP_540_70 | Peptide Q10 CR 10068 | BJ Fibroblasts | 0 | -0.37215345 |
| CMP_538_42 | D-(-)-Fructose | t- keratinocytes | -0.310155803 | 0 |
| CMP_521_55 | Lunawhite | t- keratinocytes | 0 | -0.529463818 |
| CMP_537_36 | Symmatrix | BJ Fibroblasts | 0 | -0.670767858 |
| CMP_525_59 | Allyl isothiocyanate | t- keratinocytes | 0.240290711 | -0.536010793 |
| CMP_557_43 | Dermcom | t- keratinocytes | -0.26088755 | 0 |
| CMP_521_94 | Tego Pep4-17 | t- keratinocytes | 0.223277544 | -0.571795387 |
| CMP_534_59 | Hyadisine | BJ Fibroblasts | -0.348184732 | -0.544957702 |
| CMP_557_17 | Potassium Sorbate | t- keratinocytes | -0.190957945 | 0 |
| CMP_535_09 | Phvcoboreane | t- keratinocytes | 0 | 0 |
| CMP_543_25 | Symmatrix | BJ Fibroblasts | 0.387799282 | -0.403702757 |
| CMP_544_72 | D-(-)-Fructose | t- keratinocytes | -0.459110421 | -0.405870205 |
| CMP_514_82 | Pro-Lipiskin w/o preservative | BJ Fibroblasts | -0.414750573 | -0.505116751 |
| CMP_521_10 | BIOCHANIN A | t- keratinocytes | 0 | -0.731431643 |
| CMP_524_78 | Dermapure HP (no preserv) | t- keratinocytes | 0.369809912 | 0 |
| CMP_532_30 | Adenine | t- keratinocytes | -0.222196661 | -0.280193131 |
| CMP_547_37 | LAM-C7-IDO | t- keratinocytes | 0 | 0 |
| CMP_511_56 | OTZ (2-OXO-1,3-Thiazolidine) | BJ Fibroblasts | -0.328330595 | 0.466665652 |
| CMP_534_02 | Sculptosane | BJ Fibroblasts | 0 | 0 |
| CMP_519_10 | Lunawhite | t- keratinocytes | 0.378345607 | 0 |
| CMP_539_35 | D-(-)-Fructose | t- keratinocytes | 0 | 0 |
| CMP_527_46 | Verityl AB1000 | t- keratinocytes | -0.262548435 | 0.333909886 |
| CMP_524_61 | L-Glutamic acid | t- keratinocytes | -0.265535877 | 0 |
| CMP_515_93 | Bio1048 | BJ Fibroblasts | -0.271577912 | 0 |
| CMP_529_14 | Pinoxide | BJ Fibroblasts | -0.300944759 | 0 |
| CMP_560_69 | LAM-C7-IDO | t- keratinocytes | -0.302597326 | 0 |
| CMP_537_32 | 2-cyano-1-tert-pentyl-3-(3-pyridyl)-GuanidineP1075 | BJ Fibroblasts | -0.317931349 | -0.318354127 |
| CMP_560_68 | Phytocaspaline | t- keratinocytes | -0.324641024 | -0.50515773 |
| CMP_541_60 | nipecotic acid | BJ Fibroblasts | -0.377467592 | -0.440708823 |
| CMP_527_40 | I-n6-(1-iminoethyl)lysine | t- keratinocytes | -0.388135435 | -0.359865701 |
| CMP_530_40 | L-Histidine | t- keratinocytes | -0.393117684 | -0.506784939 |
| CMP_560_59 | 19719-NF2-6 | t- keratinocytes | -0.402235814 | -0.416404763 |
| CMP_559_59 | Adenine | BJ Fibroblasts | -0.407206703 | -0.454937009 |
| CMP_517_06 | Pro-Lipiskin w/o preservative | BJ Fibroblasts | -0.416023979 | 0 |
| CMP_553_12 | Matrine | t- keratinocytes | -0.425067454 | 0 |
| CMP_541_61 | 2-cyano-1-tert-pentyl-3-(3-pyridyl)-GuanidineP1075 | BJ Fibroblasts | -0.432098675 | 0 |
| CMP_523_15 | Matrine | t- keratinocytes | -0.43994482 | -0.412621873 |
| CMP_539_19 | L-Glutamic acid | t- keratinocytes | -0.456929787 | -0.450696042 |
| CMP_560_03 | Dermcom | t- keratinocytes | -0.457230641 | 0 |
| CMP_539_03 | L-Histidine | t- keratinocytes | -0.465673422 | 0 |
| CMP_547_45 | Bernal Ester DCM | t- keratinocytes | -0.470774755 | -0.344883655 |
| CMP_547_84 | I-n6-(1-iminoethyl)lysine | t- keratinocytes | -0.473180776 | 0 |
| CMP_545_80 | LAM-C7-IDO | t- keratinocytes | -0.488344998 | 0 |
| CMP_560_75 | Potassium Sorbate | t- keratinocytes | -0.49339744 | 0 |
| CMP_557_60 | 19719-NF2-6 | t- keratinocytes | -0.50995192 | -0.510662569 |
| CMP_516_36 | nipecotic acid | BJ Fibroblasts | -0.513178139 | -0.560415881 |
| CMP_516_55 | Bio1048 | BJ Fibroblasts | -0.523501309 | -0.57861177 |
| CMP_547_04 | Adenine | t- keratinocytes | -0.524662731 | 0 |
| CMP_518_36 | OTZ (2-OXO-1,3-Thiazolidine) | BJ Fibroblasts | -0.53887976 | -0.59916195 |
| CMP_559_55 | Permethrin | BJ Fibroblasts | -0.577940884 | -0.722352058 |
| CMP_549_55 | apomorphine | BJ Fibroblasts | -0.651533483 | -0.677986124 |
| CMP_541_54 | Peptide Q10 CR 10068 | BJ Fibroblasts | 0.499213681 | 0 |
| CMP_520_75 | 1-octanol | BJ Fibroblasts | 0.357543059 | 0 |
| CMP_540_77 | CR11036 ATP China New Lot # | BJ Fibroblasts | 0.349603392 | 0 |
| CMP_539_21 | Phenyl n-butyrate sodium salt | t- keratinocytes | 0.274784858 | -0.373573935 |
| CMP_551_78 | BIOCHANIN A | t- keratinocytes | 0.21484795 | 0 |
| CMP_512_41 | Dihydroxymethylchromone | t- keratinocytes | 0 | -0.617160043 |
| CMP_516_69 | 1-octanol | BJ Fibroblasts | 0 | -0.611814659 |
| CMP_543_11 | CR11036 ATP China New Lot # | BJ Fibroblasts | 0 | -0.611692938 |
| CMP_521_64 | Dihydroxymethylchromone | t- keratinocytes | -0.216543323 | -0.542478039 |
| CMP_550_91 | UVB | BJ Fibroblasts | -0.277861562 | -0.376272087 |
| CMP_535_37 | Pinoxide | t- keratinocytes | -0.281645467 | 0 |
| CMP_525_29 | Pinoxide | t- keratinocytes | -0.293418538 | -0.52428073 |
| CMP_538_70 | Phenyl n-butyrate sodium salt | t- keratinocytes | -0.306700749 | 0 |
| CMP_556_80 | Vitamin K2 | BJ Fibroblasts | -0.312947883 | -0.521534904 |
| CMP_521_15 | Dermapure HP (no preserv) | t- keratinocytes | -0.314916114 | -0.483554663 |
| CMP_526_24 | Vitamin K2 | BJ Fibroblasts | -0.316767492 | -0.655141298 |
| CMP_518_21 | Hyadisine | BJ Fibroblasts | -0.322580894 | -0.536078754 |
| CMP_520_81 | Hyadisine | BJ Fibroblasts | -0.337020875 | 0 |
| CMP_537_12 | CR11036 ATP China New Lot # | BJ Fibroblasts | -0.369855355 | -0.47434869 |
| CMP_542_34 | Phenyl n-butvrate sodium salt | t- keratinocytes | -0.412320816 | -0.632450652 |
| CMP_539_60 | monosodium tartrate | t- keratinocytes | -0.419919461 | -0.474231635 |
| CMP_522_04 | German Chamomile serum fraction | BJ Fibroblasts | -0.441392896 | -0.5274116 |
| CMP_521_70 | Allyl isothiocyanate | t- keratinocytes | -0.453294585 | -0.739677439 |
| CMP_557_45 | Phytocaspaline | t- keratinocytes | -0.468888077 | 0 |
| CMP_517_83 | Bio1048 | BJ Fibroblasts | -0.401670825 | -0.455737123 |
| CMP_537_14 | Yuzu Ceramide B | BJ Fibroblasts | 0 | 0 |
| CMP_524_69 | Simuthyal | t- keratinocytes | -0.406912543 | -0.420230864 |
| CMP_522_45 | Pinoxide | BJ Fibroblasts | -0.303211612 | 0 |
| CMP_513_04 | OTZ (2-OXO-1,3-Thiazolidine) | BJ Fibroblasts | -0.373176719 | -0.538339115 |
| CMP_541_36 | Yuzu Ceramide B | BJ Fibroblasts | -0.497428438 | -0.526777027 |
| CMP_535_51 | Dihydroxymethylchromone | t- keratinocytes | 0.278202585 | 0 |
| CMP_551_06 | Simuthyal | t- keratinocytes | -0.278226117 | 0 |
| CMP_523_22 | Pinoxide | t- keratinocytes | -0.414273426 | -0.637723409 |
| CMP_523_09 | Phycoboreane | t- keratinocytes | 0 | -0.664004828 |
| CMP_520_11 | Adenine | BJ Fibroblasts | -0.326962449 | -0.454410971 |
| CMP_543_05 | UVB | BJ Fibroblasts | 0.486978881 | 0 |
| CMP_536_63 | monosodium tartrate | t- keratinocytes | 0.40488122 | 0 |
| CMP_543_83 | Hesperetin | BJ Fibroblasts | -0.267393139 | -0.495873451 |
| CMP_525_52 | Phycoboreane | t- keratinocytes | 0 | -0.543473516 |
| CMP_553_09 | Sodium Chondroitin Sulfate | t- keratinocytes | -0.437167955 | -0.754251516 |
| CMP_520_38 | Sculptosane | BJ Fibroblasts | -0.556763232 | -0.42855375 |
| CMP_549_34 | Sodium Thiocyanate | BJ Fibroblasts | -0.4518741 | -0.668507496 |
| CMP_540_60 | Yuzu Ceramide B | BJ Fibroblasts | -0.33007993 | 0 |
| CMP_552_72 | UVB | BJ Fibroblasts | 0 | -0.6282744 |
| CMP_518_25 | Sculptosane | BJ Fibroblasts | -0.302624713 | 0.642253058 |
| CMP_523_43 | Tego Pep4-17 | t- keratinocytes | -0.454238127 | -0.55504737 |
| CMP_524_31 | Matrine | t- keratinocytes | -0.462106993 | 0 |
| CMP_552_24 | Sodium Thiocyanate | BJ Fibroblasts | -0.326227253 | -0.494377498 |
| CMP_534_83 | Pinoxide | BJ Fibroblasts | -0.541274015 | 0 |
| CMP_537_16 | Hesperetin | BJ Fibroblasts | 0 | -0.61859615 |
| CMP_536_34 | Verityl AB1000 | t- keratinocytes | -0.545771002 | -0.623735825 |
| CMP_540_02 | Hesperetin | BJ Fibroblasts | 0 | 0 |
| CMP_551_72 | Sodium Chondroitin Sulfate | t- keratinocytes | -0.380487493 | -0.560032256 |
| CMP_540_05 | 2-cyano-1-tert-pentyl-3-(3-pyridyl)-GuanidineP1075 | BJ Fibroblasts | -0.428861705 | -0.344160428 |
| CMP_516_94 | Permethrin | BJ Fibroblasts | -0.431404662 | 0 |
| CMP_541_33 | apomorphine | BJ Fibroblasts | 0 | 0 |
| CMP_538_55 | monosodium tartrate | t- keratinocytes | -0.504569817 | -0.650430487 |
| CMP_520_57 | Permethrin | BJ Fibroblasts | 0 | 0 |
| CMP_537_24 | Peptide Q10 CR 10068 | BJ Fibroblasts | -0.542519019 | -0.604802102 |
| CMP_540_24 | Symmatrix | BJ Fibroblasts | -0.50182886 | -0.477420932 |
| CMP_542_89 | Bernal Ester DCM | t- keratinocytes | -0.336006938 | -0.477755868 |
| CMP_552_46 | nipecotic acid | BJ Fibroblasts | 0 | 0 |
| CMP_558_56 | Nachyline | BJ Fibroblasts | -0.560053963 | -0.763141825 |
| CMP_549_88 | 1-octanol | BJ Fibroblasts | -0.267762968 | -0.522908729 |
| CMP_525_07 | Simuthyal | t- keratinocytes | -0.360224575 | -0.677490313 |
| CMP_516_23 | Adenine | BJ Fibroblasts | -0.412043089 | -0.480895665 |
| CMP_524_56 | Verityl AB1000 | t- keratinocytes | 0 | 0 |
| CMP_523_24 | Dermapure HP (no preserv) | t- keratinocytes | -0.367480778 | -0.612461607 |
| CMP_534_24 | German Chamomile serum fraction | BJ Fibroblasts | -0.381841032 | -0.621078246 |
| CMP_555_10 | Nachyline | BJ Fibroblasts | 0 | 0 |

Every document cited herein is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent any meaning or definition of a term in this document conflicts with any meaning or definition of the same term in a document incorporated by reference, the meaning or definition assigned to that term in this document shall govern.

The values disclosed herein are not to be understood as being strictly limited to the exact numerical values recited. Instead, unless otherwise specified, each such value is intended to mean both the recited value and a functionally equivalent range surrounding that value.

The present invention should not be considered limited to the specific examples described herein, but rather should be understood to cover all aspects of the invention. Various modifications, equivalent processes, as well as numerous structures and devices to which the present invention may be applicable will be readily apparent to those of skill in the art. Those skilled in the art will understand that various changes may be made without departing from the scope of the invention, which is not to be considered limited to what is described in the specification.

## Claims

1. A system for identifying connections between perturbagens and genes associated with periorbital dyschromia, comprising:
(a) at least one computer readable medium having stored thereon a plurality of instances and a periorbital dychromia-relevant gene expression signature, wherein the instances and the gene expression signature are derived from one of a human epidermal skin cell or a human dermal skin cell, each instance comprises an instance list of rank-ordered identifiers of differentially expressed genes, and the periorbital dychromia-relevant gene expression signature comprises one or more gene expression signature lists of identifiers representing differentially expressed genes associated with at least one type of periorbital dyschromia;
(b) a programmable computer comprising computer-readable instructions that cause the programmable computer to execute one or more of the following:
(i) accessing the plurality of instances and a periorbital dychromia-relevant gene expression signature stored on the computer readable medium;
(ii) comparing the periorbital dychromia-relevant gene expression signature to the plurality of the instances, wherein the comparison comprises comparing each identifier in the gene expression signature list with the position of the same identifier in the instance list for each of the plurality of instances; and
(iii) assigning a connectivity score to each of the plurality of instances.

2. The system of claim 1, further comprising: a microarray scanner for receiving a biological sample obtained from human epidermal or dermal cells; and a second programmable computer for transmitting gene expression data from the scanner to the first programmable computer.

3. The system of claim 1 or 2, further comprising an array of perturbagens for application to the human keratinocyte, fibroblast, melanocyte or melanoma cells.

4. The system of any preceding claim, wherein the plurality of instances comprises between about 50 and about 50,000 instances.

5. A system according to claim 4, wherein the plurality of instances comprises between about 1,000 and about 20,000 instances.

6. The system of any preceding claim, wherein the programmable computer assigns a connectivity score to each of the plurality of instances and the connectivity score has a value between +2 and -2.

7. The system of claim 6, further comprising identifying a skin instance having a negative connectivity score or a positive connectivity score.

8. A method of formulating a cosmetic composition, the method comprising:
(a) accessing with a computer a plurality of skin instances stored on at least one computer readable medium, wherein each instance is associated with a perturbagen and wherein each instance comprises an ordered list comprising a plurality of identifiers representing up-regulated genes and down-regulated genes;
(b) accessing with a computer at least one gene expression signature stored on the at least one computer readable medium, wherein the gene expression signature corresponds to a type of periorbital dyschromia and comprises one or more lists comprising a plurality of identifiers representing a plurality of up-regulated genes and a plurality of down-regulated genes associated with periorbital dyschromia;
(c) assigning with a computer a connectivity score to each of the plurality of instances, wherein each instance is associated with the at least one perturbagen; and
(d) formulating a cosmetic composition comprising a dermatologically acceptable carrier and at least one of the perturbagen(s) associated with the instance, wherein the connectivity score of the instance associated with the at least one perturbagen is negative.

9. The method according to claim 8, wherein each instance comprises identifiers corresponding to between about 5 and about 800 genes.

10. The method according to claim 8 or 9, wherein step (b) comprises accessing a plurality of gene expression signatures corresponding to one or more types of periorbital dyschromia, and step (c) comprises assigning to each of the plurality of instances a connectivity score for each of the plurality of gene expression signatures.

11. The method according to claim 10, wherein step (c) comprises assigning a connectivity score to each of the plurality of skin instances based on a combination of the connectivity scores assigned to each instance for each of the plurality of gene expression signatures.

12. The method according to any of claims 8 to 11, wherein each of the plurality of gene expression signatures comprises one or more gene expression signature lists comprising a plurality of identifiers representing a plurality of up-regulated genes and a plurality of down-regulated genes, wherein the plurality of up-regulated genes comprises between about 80% and about 100% of the up-regulated genes are set forth in at least one of Tables 1, 3, 5, 7, 9 and 11, and wherein the plurality of down-regulated genes comprises between about 80% and about 100% of the down-regulated genes set forth in at least one of Tables 2, 4, 6, 8, 10 and 12.

## Patentansprüche

1. System zur Identifizierung von Zusammenhängen zwischen Perturbagenen und Genen, die mit periorbitaler Dyschromie zusammenhängen, folgendes umfassend:
(a) mindestens ein Computer-lesbares Medium mit einer Vielzahl von darauf gespeicherten Instanzen und einer periorbitalen Dyschromie-relevanten Genexpressionssignatur, wobei sich die Instanzen und die Genexpressionssignatur von einer aus einer menschlichen epidermalen Hautzellen oder einer menschlichen dermalen Hautzellen ableiten, jede Instanz eine Instanzliste von ranggeordneten Kennungen unterschiedlich exprimierter Gene umfasst, und die periorbitale Dyschromie-relevante Genexpressionssignatur eine oder mehrere Genexpressionssignaturlisten von Kennungen umfasst, die unterschiedlich exprimierte Gene darstellen, die mit mindestens einem Typ von periorbitaler Dyschromie zusammenhängen;
(b) einen programmierbaren Computer, umfassend Computer-lesbare Anweisungen, die veranlassen, dass der programmierbare Computer eines oder mehrere des Folgenden ausführt:
(i) Zugreifen auf die Vielzahl von Instanzen und eine periorbitale Dyschromie-relevante Genexpressionssignatur, die auf dem Computer-lesbaren Medium gespeichert sind;
(ii) Vergleichen der periorbitalen Dyschromie-relevanten Genexpressionssignatur mit der Vielzahl der Instanzen, wobei der Vergleich das Vergleichen einer jeden Kennung in der Genexpressionssignaturliste mit der Position der gleichen Kennung in der Instanzliste für jede aus der Vielzahl von Instanzen umfasst; und
(iii) Zuordnen eines Konnektivitätswerts einer jeden der Vielzahl von Instanzen.

2. System nach Anspruch 1, weiterhin umfassend: einen Mikroarrayscanner zur Aufnahme einer biologischen Probe, die aus menschlichen epidermalen oder dermalen Zellen erhalten wird; und einen zweiten programmierbaren Computer zum Übertragen von Genexpressionsdaten von dem Scanner auf den ersten programmierbaren Computer.

3. System nach Anspruch 1 oder 2, weiterhin umfassend eine Reihe von Perturbagenen zur Anwendung auf die menschlichen Keratinozyten-, Fibroblasten-, Melanozyten- oder Melanom-Zellen.

4. System nach einem vorangehenden Anspruch, wobei die Vielzahl von Instanzen zwischen etwa 50 und etwa 50.000 Instanzen umfasst.

5. System nach Anspruch 4, wobei die Vielzahl von Instanzen zwischen etwa 1.000 und etwa 20.000 Instanzen umfasst.

6. System nach einem vorangehenden Anspruch, wobei der programmierbare Computer einer jeden der Vielzahl von Instanzen einen Konnektivitätswert zuordnet und der Konnektivitätswert einen Wert zwischen +2 und -2 aufweist.

7. System nach Anspruch 6, weiterhin umfassend das Erkennen einer Hautinstanz mit einem negativen Konnektivitätswert oder einem positiven Konnektivitätswert.

8. Verfahren zur Formulierung einer kosmetischen Zusammensetzung, wobei das Verfahren folgendes umfasst:
(a) Zugreifen mit einem Computer auf eine Vielzahl von auf mindestens einem Computer-lesbaren Medium gespeicherten Hautinstanzen, wobei jede Instanz mit einem Perturbagen zusammenhängt und wobei jede Instanz eine geordnete Liste umfasst, umfassend eine Vielzahl von Kennungen, die aufregulierte Gene und abregulierte Gene darstellen;
(b) Zugreifen mit einem Computer auf mindestens eine auf mindestens einem Computer-lesbaren Medium gespeicherte Genexpressionssignatur, wobei die Genexpressionssignatur einem Typ von periorbitaler Dyschromie entspricht und eine oder mehrere Listen umfasst, umfassend eine Vielzahl von Kennungen, die eine Vielzahl von aufregulierten Genen und eine Vielzahl von abregulierten Genen darstellen, die mit periorbitaler Dyschromie zusammenhängen;
(c) Zuordnen mit einem Computer eines Konnektivitätswerts einer jeden aus der Vielzahl von Instanzen, wobei jede Instanz mit dem mindestens einen Perturbagen zusammenhängt; und
(d) Formulieren einer kosmetischen Zusammensetzung, umfassend einen dermatologisch verträglichen Träger und mindestens eines der Perturbagen(e), die mit der Instanz zusammenhängen, wobei der Konnektivitätswert der Instanz, die mit dem mindestens einen Perturbagen zusammenhängt, negativ ist.

9. Verfahren nach Anspruch 8, wobei jede Instanz Kennungen, die zwischen etwa 5 und etwa 800 Genen entsprechen, umfasst.

10. Verfahren nach Anspruch 8 oder 9, wobei Schritt (b) das Zugreifen auf eine Vielzahl von einem oder mehreren Typen von periorbitaler Dyschromie entsprechenden Genexpressionssignaturen umfasst, und Schritt (c) das Zuordnen zu jeder der Vielzahl von Instanzen eines Konnektivitätswerts für jede der Vielzahl von Genexpressionssignaturen umfasst.

11. Verfahren nach Anspruch 10, wobei Schritt (c) das Zuordnen eines Konnektivitätswerts zu jeder der Vielzahl von Hautinstanzen basierend auf einer Kombination der Konnektivitätswerte, die mit jeder Instanz zusammenhängen, für jede der Vielzahl von Genexpressionssignaturen umfasst.

12. Verfahren nach einem der Ansprüche 8 bis 11, wobei jede der Vielzahl von Genexpressionssignaturen eine oder mehrere Genexpressionssignaturlisten umfasst, umfassend eine Vielzahl von Kennungen, die eine Vielzahl von aufregulierten Genen und eine Vielzahl von abregulierten Genen darstellen, wobei die Vielzahl von aufregulierten Genen zwischen etwa 80 % und etwa 100 % der in mindestens einer der Tabellen 1, 3, 5, 7, 9 und 11 dargelegten aufregulierten Gene umfasst, und wobei die Vielzahl von abregulierten Genen zwischen etwa 80 % und etwa 100 % der in mindestens einer der Tabellen 2, 4, 6, 8, 10 und 12 dargelegten abregulierten Gene umfasst.

## Revendications

1. Système pour identifier des connexions entre des perturbagènes et des gènes associés à la dyschromie périorbitaire, comprenant :
(a) au moins un support lisible par ordinateur sur lequel sont stockées une pluralité d'instances et une signature d'expression génique se rapportant à la dyschromie périorbitaire, les instances et la signature d'expression génique étant dérivées de l'une parmi une cellule cutanée épidermique humaine ou une cellule cutanée dermique humaine, chaque instance comprenant une liste d'identificateurs d'instances classés hiérarchiquement de gènes exprimés différentiellement et la signature d'expression génique se rapportant à la dyschromie périorbitaire comprenant une ou plusieurs listes d'identificateurs de signature d'expression génique représentant des gènes exprimés différentiellement associés à au moins un type de dyschromie périorbitaire ;
(b) un ordinateur programmable comprenant des instructions lisibles par ordinateur qui amènent l'ordinateur programmable à exécuter l'une ou plusieurs parmi les instructions suivantes :
(i) accéder à la pluralité d'instances et à une signature d'expression génique se rapportant à la dyschromie périorbitaire, stockées sur le support lisible par un ordinateur ;
(ii) comparer la signature d'expression génique, se rapportant à la dyschromie périorbitaire, à la pluralité d'instances, la comparaison comprenant la comparaison de chaque identificateur dans la liste de signatures d'expression génique à la position du même identificateur dans la liste d'instances pour chacune parmi la pluralité d'instances ; et
(iii) affecter un score de connectivité à chacune parmi la pluralité d'instances.

2. Système selon la revendication 1, comprenant en outre : un scanneur à micromatrice pour recevoir un échantillon biologique obtenu à partir de cellules épidermiques ou dermiques humaines ; et un deuxième ordinateur programmable pour transmettre des données d'expression génique du scanneur au premier ordinateur programmable.

3. Système selon la revendication 1 ou 2, comprenant en outre une matrice de perturbagènes pour une application à des cellules humaines de kératinocyte, de fibroblaste, de mélanocyte ou de mélanome.

4. Système selon l'une quelconque revendication précédente, dans lequel la pluralité d'instances comprend entre environ 50 et environ 50 000 instances.

5. Système selon la revendication 4, dans lequel la pluralité d'instances comprend entre environ 1000 et environ 20 000 instances.

6. Système selon l'une quelconque revendication précédente, dans lequel l'ordinateur programmable affecte un score de connectivité à chacune des instances et le score de connectivité présente une valeur entre +2 et -2.

7. Système selon la revendication 6, comprenant en outre l'identification d'une instance cutanée présentant un score de connectivité négatif ou un score de connectivité positif.

8. Procédé de formulation d'une composition cosmétique, le procédé comprenant :
(a) l'accès, à l'aide d'un ordinateur, à une pluralité d'instances cutanées stockées sur au moins un support lisible par un ordinateur, chaque instance étant associée à un perturbagène et chaque instance comprenant une liste classée comprenant une pluralité d'identificateurs représentant des gènes régulés à la hausse et des gènes régulés à la baisse ;
(b) l'accès, à l'aide d'un ordinateur, à au moins une signature d'expression génique stockée sur ledit au moins un support lisible par un ordinateur, la signature d'expression génique correspondant à un type de dyschromie périorbitaire et comprenant une ou plusieurs listes, comprenant une pluralité d'identificateurs représentant une pluralité de gènes régulés à la hausse et une pluralité de gènes régulés à la baisse associés à la dyschromie périorbitaire ;
(c) l'affectation, à l'aide d'un ordinateur, d'un score de connectivité à chacune parmi la pluralité d'instances, chaque instance étant associée à au moins un perturbagène ; et
(d) la formulation d'une composition cosmétique comprenant un support acceptable du point de vue dermatologique et au moins un des perturbagènes associés à l'instance, le score de connectivité associé audit au moins un perturbagène étant négatif.

9. Procédé selon la revendication 8, dans lequel chaque instance comprend des identificateurs correspondant à entre environ 5 et environ 800 gènes.

10. Procédé selon la revendication 8 ou 9, dans lequel l'étape (b) comprend l'accès à une pluralité de signatures d'expression génique correspondant à un ou plusieurs types de dyschromie périorbitaire et l'étape (c) comprend l'affectation, à chacune parmi la pluralité d'instances, d'un score de connectivité pour chacune parmi la pluralité de signatures d'expression génique.

11. Procédé selon la revendication 10, dans lequel l'étape (c) comprend l'affectation d'un score de connectivité à chacune parmi la pluralité d'instances cutanées, sur la base d'une combinaison des scores de connectivité affectés à chaque instance pour chacune parmi la pluralité de signatures d'expression génique.

12. Procédé selon l'une quelconque des revendications 8 à 11, dans lequel chacune parmi la pluralité de signatures d'expression génique comprend une ou plusieurs listes de signatures d'expression génique, comprenant une pluralité d'identificateurs représentant une pluralité de gènes régulés à la hausse et une pluralité de gènes régulés à la baisse, la pluralité de gènes régulés à la hausse comprenant entre environ 80 % et environ 100 % des gènes régulés à la hausse représentés dans au moins l'un des Tableaux 1, 3, 5, 7, 9 et 11 et la pluralité de gènes régulés à la baisse comprenant entre environ 80 % et environ 100 % des gènes régulés à la baisse représentés dans au moins l'un des Tableaux 2, 4, 6, 8, 10 et 12.
